# EUROPEAN PATENT SPECIFICATION

(11) **EP 3 784 174 B1**
(45) Date of publication and mention of the grant of the patent: **06.11.2024**
(21) Application number: 19793496.1
(22) Date of filing: 24.04.2019
(51) Int. Cl.: A61F 2/24, A61B 17/00

(54) **SYSTEMS FOR HEART VALVE THERAPY**
SYSTEME FÜR HERZKLAPPENTHERAPIE
SYSTÈMES POUR VALVULOTHÉRAPIE CARDIAQUE

(30) Priority: 24.04.2018 US 201862661922 P
(43) Date of publication of application: 03.03.2021
(73) Proprietor: Caisson Interventional, LLC, Maple Grove, Minnesota 55369 (US)
(72) Inventor: SCHNEIDER, Lucas T., Champlin, Minnesota 55316 (US); ASKEGAARD, Gunnar, Brainerd, Minnesota 56401 (US); GANESAN, Kavitha, Maple Grove, Minnesota 55369 (US); IYER, Ramji, Maple Grove, Minnesota 55311 (US); SCHWEICH, Jr., Cyril J., Maple Grove, Minnesota 55311 (US); MORTIER, Todd J., Mound, Minnesota 55364 (US); GARVEY, Zachary, Stillwater, Minnesota 55082 (US)
(74) Representative: Grünecker Patent- und Rechtsanwälte PartG mbB
(86) International application number: PCT/US2019/028884
(87) International publication number: WO 2019/209927

(56) References cited:
- WO-A1-2017/139380
- US-A1- 2007 239 254
- US-A1- 2009 171 456
- US-A1- 2010 049 313
- US-A1- 2011 071 613
- US-A1- 2013 296 999
- US-A1- 2014 324 164
- US-A1- 2016 113 765
- US-A1- 2017 042 678
- US-A1- 2017 189 177
- US-B2- 7 524 332
- US-B2- 9 326 853

## Description

### TECHNICAL FIELD

This document relates to prosthetic heart valves, such as prosthetic mitral valves that can be implanted and/or removed using transcatheter techniques. Some embodiments of prosthetic mitral valves described herein include an anchor portion that couples the prosthetic mitral valve to the anatomy near the native mitral valve, and a valve portion that is mateable with the anchor portion. Devices and methods for the removal of the anchor portion and/or the prosthetic mitral valve are also described herein.

### BACKGROUND

The long-term clinical effect of valve regurgitation is recognized as a significant contributor to cardiovascular related morbidity and mortality. Thus, for many therapies intended to treat the mitral valve, one primary goal is to significantly reduce or eliminate regurgitation. By eliminating the regurgitation at the mitral valve, the destructive volume overload effects on the left ventricle can be attenuated. The volume overload of mitral regurgitation (MR) relates to the excessive kinetic energy required during isotonic contraction to generate overall stroke volume in an attempt to maintain forward stroke volume and cardiac output. It also relates to the pressure potential energy dissipation of the leaking valve during the most energy-consuming portion of the cardiac cycle, isovolumetric contraction. Additionally, therapies for MR reduction can have the effect of reducing the elevated pressures in the left atrium and pulmonary vasculature reducing pulmonary edema (congestion) and shortness of breath symptomatology. Such therapies for MR reduction may also have a positive effect on the filling profile of the left ventricle (LV) and the restrictive LV physiology that can result with MR. These pathophysiologic issues indicate the potential benefits of MR therapy, but also indicate the complexity of the system and the need for a therapy to focus beyond the MR level or grade.

In some percutaneous access procedures in which a medical device is introduced through a patient's skin and into a patient's blood vessel, such an access can be used to introduce devices into the patient without the use of large cut downs, which can be painful and in some cases can hemorrhage or become infected. A percutaneous access generally employs only a small hole through the skin, which subsequently seals relatively easily, and heals quickly in comparison to a surgical cut down.

Document US 2017/042678 A1 is directed to systems and methods for heart valve therapy. A valve delivery sheath includes a flared distal end portion. The flared distal end portion can be advantageous, for example, for recapturing the valve assembly within the lumen of the valve delivery sheath after the valve assembly has been expressed from the flared distal end portion. The valve delivery sheath can be withdrawn into a guide catheter while a valve delivery catheter is held substantially stationary to thereby express a valve assembly from a lumen of the valve delivery sheath. Document US 2011/071613 A1 is directed to an integrated stent retrieval loop adapted for snare removal and/or optimized purse stringing. Document US 2013/296999 A1 describes a prosthesis delivery system. Document US 2007/239254 A1 is directed to a system for percutaneous delivery and removal of a prosthetic valve.

### SUMMARY

This document describes prosthetic heart valves, such as prosthetic mitral valves, that can interface and anchor in cooperation with the anatomical structures of a native mitral valve. The invention is described in the claims. The invention provides a two-part prosthetic heart valve, being comprised of an anchor assembly and a valve assembly, and deployment system, according to claim 1. Preferred embodiments are described in the dependent claims. The embodiments which do not fall within the scope of the claims are to be interpreted as examples useful for a better understanding of the invention. Some embodiments of prosthetic mitral valves described herein include an anchor portion that couples the prosthetic mitral valve to the anatomy near the native mitral valve, and a valve portion that is mateable with the anchor portion. In some implementations, a prosthetic mitral valve and deployment system includes a prosthetic mitral valve system, a system of multiple catheters configured to deliver the prosthetic mitral valve system, and a deployment frame system. At least some catheters of the multiple catheters are slidably engageable with each other. At least a first catheter of the multiple catheters is releasably coupleable to the prosthetic anchor assembly. At least a second catheter of the multiple catheters is releasably coupleable to the prosthetic valve assembly. The prosthetic mitral valve system can include a prosthetic anchor assembly comprising an anchor frame that defines an interior space, and a prosthetic valve assembly comprising a valve frame and multiple valve leaflets attached to the valve frame. The valve frame is configured to releasably couple with the prosthetic anchor assembly within the interior space of the anchor frame.

The claimed invention is directed to a two-part prosthetic heart valve and deployment system. The two-part prosthetic heart valve and deployment system includes: (i) a guide catheter sized for insertion within a vasculature of a human patient and defining a lumen; (ii) an inner catheter releasably coupleable with an anchor assembly of the prosthetic heart valve (wherein the inner catheter and the anchor assembly are disposable within the lumen of the guide catheter while the anchor assembly is in a low-profile delivery configuration); (iii) a valve delivery catheter releasably coupleable with a valve assembly of the prosthetic heart valve (wherein the valve delivery catheter and the valve assembly are disposable within the lumen of the guide catheter while the valve assembly is in a low-profile delivery configuration); and (iv) a valve retrieval catheter defining a lumen. The valve delivery catheter is disposable within the lumen of the valve retrieval catheter. A distal end portion of the valve retrieval catheter includes a valve engagement portion that is configured to selectively mechanically engage with the valve assembly.

The valve engagement portion includes a framework structure defining one or more interstitial open spaces. The one or more interstitial open spaces are sized and positioned to selectively mechanically engage with one or more hooks on a proximal end of the valve assembly. The one or more hooks may each be biased to project radially outward away from a center of the valve assembly at an acute angle. The two-part prosthetic heart valve and deployment system may also include an anchor retrieval sheath defining a lumen. The inner catheter and the anchor assembly may be disposable within the lumen of the anchor retrieval sheath. The anchor retrieval sheath may be disposable within the lumen of the guide catheter.

An example forming not part of the claimed invention is directed to a method for deploying and retrieving a prosthetic mitral valve system within a heart of a patient. The method includes one or more of: (a) navigating a delivery sheath of a prosthetic mitral valve delivery system through a vasculature of the patient such that a distal end of the delivery sheath is positioned within a left atrium of a heart of the patient; (b) expressing an anchor assembly of the prosthetic mitral valve system from the distal end of the delivery sheath such that the anchor assembly at least partially expands within the left atrium, the anchor assembly comprising an expandable anchor frame configured to selectively mate with a valve assembly of the prosthetic mitral valve system; (c) positioning the anchor assembly within an annulus of a native mitral valve of the heart; (d) after positioning the anchor assembly within the annulus of the native mitral valve, navigating a retrieval sheath through the vasculature of the patient such that a distal end of the retrieval sheath is positioned within the left atrium; (e) while the distal end of the retrieval sheath is positioned within the left atrium, retracting the anchor assembly into the retrieval sheath; and (f) removing the retrieval sheath containing the anchor assembly from the heart.

Such a method for deploying and retrieving a prosthetic mitral valve system within a heart of a patient may optionally include one or more of the following features. The method may also include navigating a guidewire through the vasculature of the patient such that a distal end of the guidewire is positioned in the heart. Navigating the delivery sheath through the vasculature of the patient may include passing the delivery sheath over the guidewire. The anchor assembly may be releasably coupled with an inner catheter during the expressing, the positioning, the retracting, and the removing steps of the method. Retracting the anchor assembly into the retrieval sheath may be accomplished by pulling the inner catheter proximally relative to the retrieval sheath. Retracting the anchor assembly into the retrieval sheath may cause the anchor assembly to invert and then enter into the retrieval sheath.

An example not forming part of the claimed invention is directed to a method for deploying and retrieving a prosthetic mitral valve system within a heart of a patient. The method includes one or more of: (1) navigating a delivery sheath of a prosthetic mitral valve delivery system through a vasculature of the patient such that a distal end of the delivery sheath is positioned within a left atrium of a heart of the patient; (2) expressing an anchor assembly of the prosthetic mitral valve system from the distal end of the delivery sheath such that the anchor assembly at least partially expands within the left atrium (the anchor assembly comprising an expandable anchor frame configured to selectively mate with a valve assembly of the prosthetic mitral valve system); (3) positioning the anchor assembly within an annulus of a native mitral valve of the heart; (4) after positioning the anchor assembly within the annulus, navigating a valve delivery catheter and a valve delivery sheath of the prosthetic mitral valve delivery system through the vasculature of the patient; (5) expressing, into the left atrium, the valve assembly out of the valve delivery sheath; (6) after expressing the valve assembly out of the valve delivery sheath, coupling a valve retrieval catheter to the valve assembly and then retracting the valve assembly into the valve delivery sheath; and (7) while the valve assembly is coupled to the valve retrieval catheter and within the valve delivery sheath, removing the retrieval catheter, the valve assembly, and the valve delivery sheath from the heart.

Such a method for deploying and retrieving a prosthetic mitral valve system within a heart of a patient may optionally include one or more of the following features. Coupling the valve retrieval catheter to the valve assembly may include mechanically engaging a framework structure defining one or more interstitial open spaces at a distal end portion of the valve retrieval catheter with one or more hooks on a proximal end of the valve assembly. The one or more hooks may each be biased to project radially outward away from a center of the valve assembly at an acute angle so as to mechanically engage the framework structure. The method may also include: after removing the retrieval catheter, the valve assembly, and the valve delivery sheath from the heart, implanting a second valve assembly into engagement with the anchor assembly while the anchor assembly is positioned within the annulus of the native mitral valve. The method may also include: after removing the retrieval catheter, the valve assembly, and the valve delivery sheath from the heart, removing the anchor assembly from the heart. Removing the anchor assembly from the heart may include: navigating a retrieval sheath through the vasculature of the patient such that a distal end of the retrieval sheath is positioned within the left atrium; while the distal end of the retrieval sheath is positioned within the left atrium, retracting the anchor assembly into the retrieval sheath; and removing the retrieval sheath containing the anchor assembly from the heart. Retracting the anchor assembly into the retrieval sheath may cause the anchor assembly to invert and then enter into the retrieval sheath.

An example not forming part of the claimed invention is directed to a method for deploying and retrieving a prosthetic mitral valve system within a heart of a patient. The method including one or more of: (i) navigating a delivery sheath of a prosthetic mitral valve delivery system through a vasculature of the patient such that a distal end of the delivery sheath is positioned within a left atrium of a heart of the patient; (ii) expressing an anchor assembly of the prosthetic mitral valve system from the distal end of the delivery sheath such that the anchor assembly at least partially expands within the left atrium (the anchor assembly comprising an expandable anchor frame configured to selectively mate with a valve assembly of the prosthetic mitral valve system); (iii) positioning the anchor assembly within an annulus of a native mitral valve of the heart; (iv) after positioning the anchor assembly within the annulus, navigating a valve delivery catheter and a valve delivery sheath of the prosthetic mitral valve delivery system through the vasculature of the patient; (v) expressing, into the left atrium, the valve assembly out of the valve delivery sheath; (vi) retracting the valve assembly into the valve delivery sheath using a retrieval catheter disposed within the valve delivery sheath; (vii) after retracting the valve assembly into the valve delivery sheath, retracting the anchor assembly into the valve delivery sheath; and (viii) after retracting the anchor assembly into the valve delivery sheath, removing the retrieval catheter, the valve assembly, the anchor assembly, and the valve delivery sheath from the vasculature of the patient.

An example not forming part of the claimed invention is directed to a prosthetic mitral valve system that includes a valve assembly comprising an expandable valve frame that defines a central longitudinal axis and an occluder attached to the expandable valve frame, and an anchor assembly comprising an expandable anchor frame, the expandable anchor frame configured to selectively couple with the valve assembly. A proximal end of the expandable valve frame includes one or more hooks that are each biased to project radially outward away from the central longitudinal axis at an acute angle.

An example not forming part of the claimed invention is directed to a two-part prosthetic heart valve and deployment system that includes: a guide catheter defining a lumen, the guide catheter sized for insertion within a vasculature of a human patient; an inner catheter releasably coupleable with an anchor assembly of the prosthetic heart valve; a valve delivery catheter releasably coupleable with a valve assembly of the prosthetic heart valve; and a valve retrieval catheter configured to selectively mechanically engage with the valve assembly.

An example not forming part of the claimed invention is directed to a prosthetic heart valve and retrieval system that includes a prosthetic heart valve component and a retrieval catheter including a distal end portion configured to selectively mechanically interlock with one or more corresponding features of the prosthetic heart valve component while the prosthetic heart valve component is in a heart.

Some or all of the embodiments described herein may provide one or more of the following advantages. First, some embodiments of the prosthetic mitral valve systems provided herein can be used in a percutaneous transcatheter mitral replacement procedure (e.g., complete delivery and anchoring of the prosthetic valve components via one or more catheters advanced percutaneously into the venous system or arterial system and to the heart) that is safe, reliable, and repeatable by surgeons and/or interventional cardiologists of a variety of different skill levels. For example, in some implementations the prosthetic mitral valve system can establish a reliable and consistent anchor/substrate to which the valve/occluder structure subsequently engages. Thus, the prosthetic mitral valve system can be specifically designed to make use of the geometry/mechanics of the native mitral valve to create sufficient holding capability. In one particular aspect, the anatomical gutter found below a native mitral valve annulus can be utilized as a site for anchoring the prosthetic mitral valve system, yet the anchoring structure can be deployed in a matter that maintains native leaflet function of the mitral valve, thereby providing the ability to completely separate and stage the implantation of the components of the prosthetic mitral valve system. Accordingly, some embodiments of the prosthetic mitral valve systems described herein are configured to be implanted in a reliable, repeatable, and simplified procedure that is broadly applicable to a variety of patients and physicians, while also employing a significantly less invasive method.

Second, some embodiments of the prosthetic mitral valve systems provided herein include features to facilitate convenient engagement of prosthetic mitral valve components to the deployment catheter system. For example, in preparation for deployment of the prosthetic valve assembly, a clinician may need to engage one or more control wires of the deployment catheter system with the valve assembly by threading the wire through multiple control wire engagement features located on the valve assembly. To assist the clinician with that task, in some embodiments the valve assembly is provided with a removable guide tube extending through each of the control wire engagement features. To engage a control wire with the valve assembly, the clinician inserts the control wire through the tube, and then removes the tube while leaving the control wire in place relative to the valve assembly. In that fashion, the control wire can be installed through each of the control wire engagement features in a convenient manner. The same feature can be included in the prosthetic anchor assembly.

Third, some embodiments of the prosthetic mitral valve systems and deployment systems include multiple control wires to provide highly user-controllable diametric expansion of the prosthetic mitral valve components during deployment. For example, some embodiments of the anchor assembly and anchor assembly deployment system include a first, proximal control wire and a second, mid-body control wire. As described further below, independent control of the proximal and mid-body portions of the anchor assembly during deployment can advantageously facilitate a user-friendly and clinically effective transcatheter deployment technique.

Fourth, some embodiments of the prosthetic mitral valve systems are configured to perform with reduced *in situ* stress levels. For example, in some embodiments, the structure of the anchor and/or valve assembly framework is specifically designed to function within the dynamic environment of the heart while incurring low levels of stress and strain within the framework members. Such features can allow for greater durability and longevity of the prosthetic mitral valve systems.

Fifth, some embodiments of the prosthetic mitral valve systems include features to reduce the potential of interference or entanglement with the native valve's chordae tendineae. For example, in some embodiments the anchor assembly framework is specifically designed such that particular sub-annular framework members extend essentially parallel with the chordae tendineae. In result, an anchor assembly can be implanted in a native mitral valve with minimal or no impact on the natural functioning of the native valve leaflets.

Sixth, in particular embodiments, the prosthetic mitral valve system can include two different expandable components (e.g., an anchor assembly and a valve assembly) that are separately delivered to the implantation site, and both components can abut and engage with native heart tissue at the mitral valve. For example, the first component (e.g., the anchor assembly) can be configured to engage with the heart tissue that is at or proximate to the annulus of the native mitral valve, and the second component (e.g., the valve assembly) can be configured to provide a seal interface with native valve leaflets of the mitral valve.

Seventh, in some embodiments the prosthetic mitral valve system includes features for enhanced coupling alignment and strength between the anchor assembly and the valve assembly. Such features may provide strong decoupling resistance and, in turn, enhanced migration resistance of the prosthetic mitral valve system.

Eighth, some embodiments of the prosthetic mitral valve systems described herein are configured with a systolic anterior motion (SAM) containment member feature. SAM containment members can reduce or prevent the potential for a natural mitral valve anterior leaflet to "flop" outward and/or from being drawn by a Venturi effect into the left ventricular outflow tract (LVOT). Accordingly, the SAM containment members can reduce the risk of full or partial blockages of the LVOT. In some patient scenarios, the potential for suffering future adverse health events, such as heart failure, is thereby reduced.

Ninth, some embodiments of the prosthetic mitral valve systems herein include methods and devices for removal of the anchor portion and/or the prosthetic mitral valve as necessary. For example, during or after the implantation process, if the anchor size is found to be suboptimal, the anchor is not engaging with the native anatomy as desired, or for other reasons, the anchor may need to be removed and/or repositioned. In addition, if the prosthetic mitral valve is not performing as desired, or is not coupling to the anchor properly, the prosthetic mitral valve may need to be removed and/or repositioned. The systems and methods described herein can advantageously facilitate such removal and/or repositioning procedures.

Tenth, using the devices, systems, and methods described herein, various medical conditions, such as heart valve conditions, can be treated in a minimally invasive fashion. Such minimally invasive techniques can tend to reduce recovery times, patient discomfort, and treatment costs.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 shows a perspective view of a portion of a prosthetic mitral valve deployment system in a cross-sectional view of a native human heart (from a rear side of the heart), in accordance with some embodiments.
FIG. 2 shows a perspective view of a prosthetic mitral valve anchor assembly in the left atrium of the heart after the anchor assembly has emerged from an anchor delivery sheath of the deployment system of FIG. 1.
FIG. 3 shows a distal end portion of some components of the deployment system of FIG. 1, including two wires for controlling the diametric expansion of the anchor assembly of FIG. 2.
FIG. 4 shows a perspective view of the distal end portion of the deployment system as shown in FIG. 3 in engagement with the anchor assembly of FIG. 2.
FIG. 5 shows a perspective view of the anchor assembly of FIG. 2 after being rotated/panned in the left atrium so as to orient the anchor assembly axis generally perpendicular to the native mitral valve.
FIG. 6 shows a side view of a delivery catheter of prosthetic mitral valve deployment system.
FIG. 7 shows a perspective view in a commissural cross-sectional view of the heart (from the left side of the heart) of the anchor assembly of FIG. 2 after being partially advanced through the native mitral valve so as to position projections of the anchor assembly below an annulus of the native mitral valve.
FIG. 8 shows a perspective view of the anchor assembly of FIG. 7 after being diametrically expanded to align the projections of the anchor assembly with a sub-annular gutter of the native mitral valve.
FIG. 9 shows a perspective view of the anchor assembly of FIG. 8 after being retracted so as to position the projections of the anchor assembly in the sub-annular gutter of the native mitral valve.
FIG. 10 shows a perspective view of the anchor assembly of FIG. 7 after the release and retraction of the control wires of the deployment system.
FIG. 11 shows a perspective view of the anchor assembly of FIG. 7 after the retraction of some of the catheters of the deployment system.
FIG. 12 is a top view of a native mitral valve and depicts a gutter perimeter of the sub-annular gutter of FIG. 7 (without the anchor assembly).
FIG. 13 shows the native mitral valve of FIG. 12 and a schematic representation of the sub-annular frame members of the anchor assembly of FIG. 7.
FIG. 14 shows a top view of the anchor assembly of FIG. 7 deployed in a sheet material that represents the annular plane of a mitral valve.
FIG. 15 shows a perspective view (slightly from the top) of the anchor assembly of FIG. 7 deployed in the material that represents the annular plane of a mitral valve (as in FIG. 14).
FIG. 16 shows a perspective view (slightly from the bottom) of the anchor assembly of FIG. 7 deployed in the material that represents the annular plane of a mitral valve (as in FIG. 14).
FIG. 17 shows a bottom view of the anchor assembly of FIG. 7 deployed in the material that represents the annular plane of a mitral valve (as in FIG. 14).
FIG. 18 shows a perspective top view of an example frame of the anchor assembly of FIG. 7, in accordance with some embodiments.
FIG. 19 shows a perspective side view of the example frame of the anchor assembly of FIG. 7, in accordance with some embodiments.
FIG. 20 shows a posterior side view of the example frame of the anchor assembly of FIG. 7, in accordance with some embodiments.
FIG. 21 shows a posterior side view (slightly from the top) of the anchor assembly of FIG. 7 including a covering material disposed on portions of the anchor frame.
FIG. 22 is a photographic image showing a perspective top view of the anchor assembly of FIG. 7 implanted within a native mitral valve (with the native mitral valve leaflets in a closed state), and FIG. 23 shows a corresponding anatomical top view of the anchor assembly of FIG. 22.
FIG. 24 is a photographic image showing a perspective top view of the anchor assembly of FIG. 7 implanted within a native mitral valve (with the native mitral valve leaflets in an open state).
FIG. 25 shows a perspective view of the anchor assembly of FIG. 7 implanted within the native mitral valve and an anchor retrieval sheath extending into the left atrium (in a commissural cross-sectional view of the heart).
FIG. 26 shows a perspective view of the anchor assembly of FIG. 7 implanted within the native mitral valve and a valve assembly delivery sheath extending into the left atrium (in a commissural cross-sectional view of the heart).
FIG. 27 shows a perspective view of a valve assembly in the left atrium after partial emergence from the valve assembly delivery sheath of FIG. 26. The valve assembly is configured in a first (partially expanded) arrangement.
FIG. 28 shows a perspective view of the valve assembly of FIG. 27 with the valve deployment system being manipulated in preparation for the installation of the valve assembly into the anchor assembly.
FIG. 29 shows a perspective view of the valve assembly of FIG. 27 (while still in the first, partially expanded arrangement) being positioned within the anchor assembly.
FIG. 30 shows a perspective view of the valve assembly of FIG. 27, with the valve assembly expanded within the anchor assembly, prior to deployment of the SAM containment member.
FIG. 31 shows a perspective view of the valve assembly of FIG. 27, with a valve retrieval catheter being positioned in the left atrium (in a commissural cross-sectional view of the heart).
FIG. 32 shows a perspective view of the valve assembly of FIG. 27, with a retrieval member of the valve retrieval catheter of FIG. 31 engaged to the valve assembly in the left atrium (in a commissural cross-sectional view of the heart).
FIG. 33 shows a perspective view of the valve assembly of FIG. 27 and the valve delivery catheter with radial protrusions for engaging the valve assembly.
FIG. 34 shows a perspective view of the valve assembly of FIG. 27 and the valve delivery catheter with a slotted collar member for engaging the valve assembly.
FIG. 35 shows a perspective view of the valve delivery sheath after removal of the valve assembly of FIG. 27 from the left atrium (in a commissural cross-sectional view of the heart).
FIG. 36 shows a perspective view of the valve assembly of FIG. 27, with the valve assembly expanded within the anchor assembly after the release and retraction of the control wires of the deployment system, after deployment of the SAM containment member, and after the retraction of some of the catheters of the deployment system.
FIG. 37 shows an anterior side view of a valve frame of a valve assembly of FIGS. 27-36, in accordance with some embodiments.
FIG. 38 shows a bottom view of the valve frame of FIG. 37.
FIG. 39 shows an anterior side view of a second valve frame of a second valve assembly of FIGS. 27-36, in accordance with some embodiments.
FIG. 40 shows a bottom view of the second valve frame of FIG. 39.
FIG. 41 shows a top view of the valve assembly of FIGS. 27-36, including a threading tube coupled to the proximal end of the valve assembly.
FIG. 42A is an anterior side perspective view of the valve assembly of FIG. 41.
FIG. 42B shows an enlarged view of a proximal portion of the valve assembly of FIG. 42A.
FIG. 43 is bottom view of the valve assembly of FIG. 41.
FIG. 44 is an exploded posterior side view of the anchor assembly and valve assembly of FIGS. 27-36, in accordance with some embodiments.
FIG. 45 is a top view of an example prosthetic mitral valve system that includes a valve assembly engaged with an anchor assembly, in accordance with some embodiments.
FIG. 46 is an anterior view of the prosthetic mitral valve system of FIG. 44.
FIG. 47 is a posterior view of the prosthetic mitral valve system of FIG. 44.
FIG. 48 is a bottom view of the prosthetic mitral valve system of FIG. 44.
FIG. 49 shows a perspective view of an example prosthetic mitral valve system deployment frame system configuration in accordance with some embodiments.
FIG. 50 shows a perspective view of the example prosthetic mitral valve system deployment frame system configuration of FIG. 49 with an anchor retrieval accessory prior to attachment.
FIG. 51 shows a perspective view of the example prosthetic mitral valve system deployment frame system configuration of FIG. 49 with the anchor retrieval accessory of FIG. 50 attached.
FIG. 52 shows a perspective view of the example prosthetic mitral valve system deployment frame system configuration of FIG. 49 and the anchor retrieval accessory of FIG. 50 attached, with the rail being wrapped around the anchor retrieval accessory.
FIG. 53 shows a perspective view of a valve retrieval accessory.
FIG. 54 shows a perspective view of the valve retrieval accessory of FIG. 53 coupled to the example prosthetic mitral valve system deployment frame system configuration of FIG. 49.

Like reference symbols in the various drawings indicate like elements.

### DETAILED DESCRIPTION OF ILLUSTRATIVE EMBODIMENTS

This disclosure describes embodiments of a prosthetic heart valve system, such as prosthetic mitral valve systems, and transcatheter systems and methods for implanting prosthetic heart valve systems. In some embodiments, the prosthetic mitral valve system can be deployed to interface and anchor in cooperation with the native anatomical structures of a mitral valve (and, optionally, in a manner that permits the continued natural function and movement of the chordae tendineae and the native mitral valve leaflets even after the anchor component is deployed).

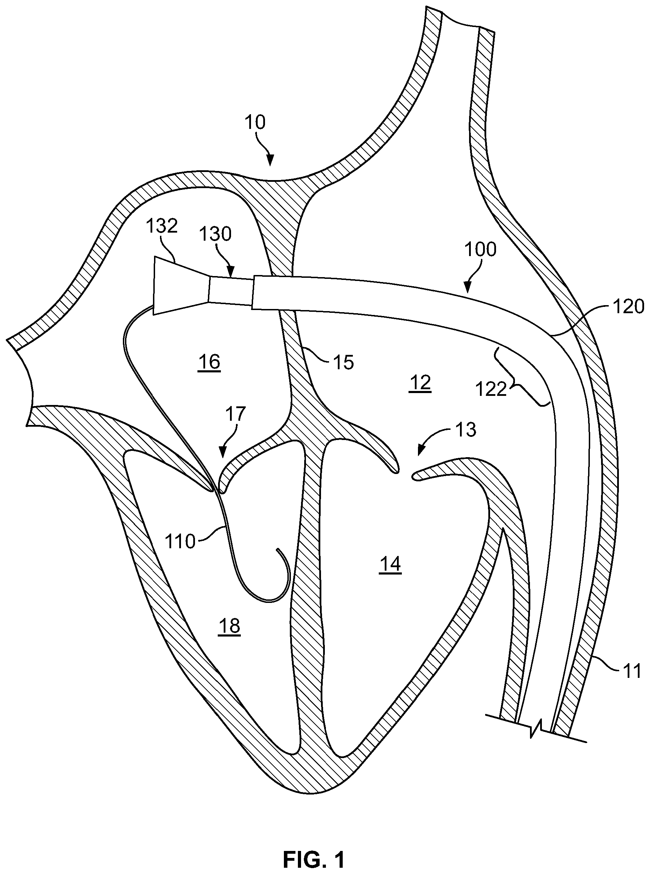

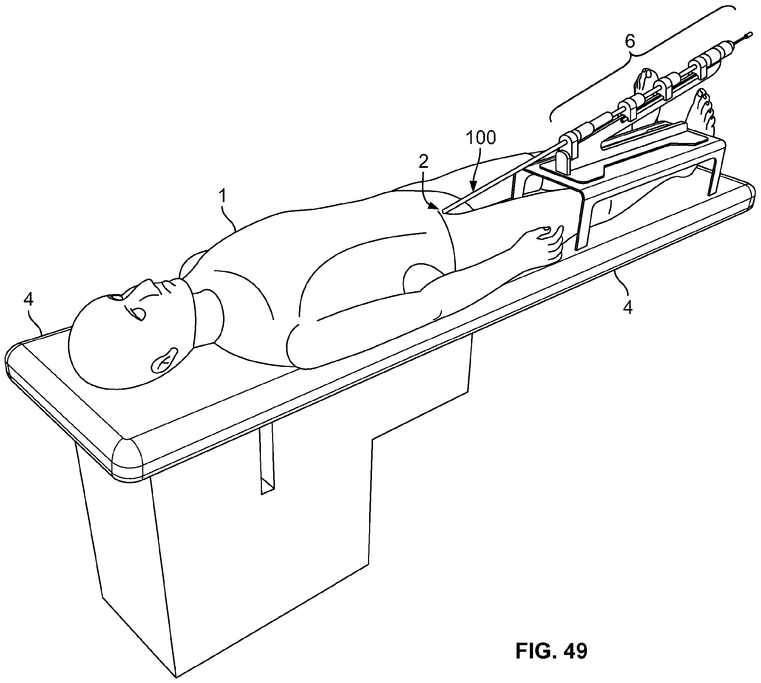

Referring to **FIG. 1****,** an example transcatheter mitral valve delivery system 100 can be navigated through a patient's vasculature to obtain access to the patient's heart 10. The transcatheter delivery system 100 facilitates implantation of a prosthetic mitral valve in a beating heart 10 using a percutaneous, or minimally invasive technique (without open-chest surgery or open-heart surgery). For example, in some implementations the transcatheter delivery system 100 is percutaneously inserted into a femoral or iliac vein via a groin opening/incision 2 in a patient 1 (FIG. 49) using a deployment frame system 6 configured to activate and/or control the movements of various components of the transcatheter delivery system 100. In some implementations, the transcatheter delivery system 100 is used in conjunction with one or more imaging modalities such as x-ray fluoroscopy, echocardiography, magnetic resonance imaging, computed tomography (CT), and the like.

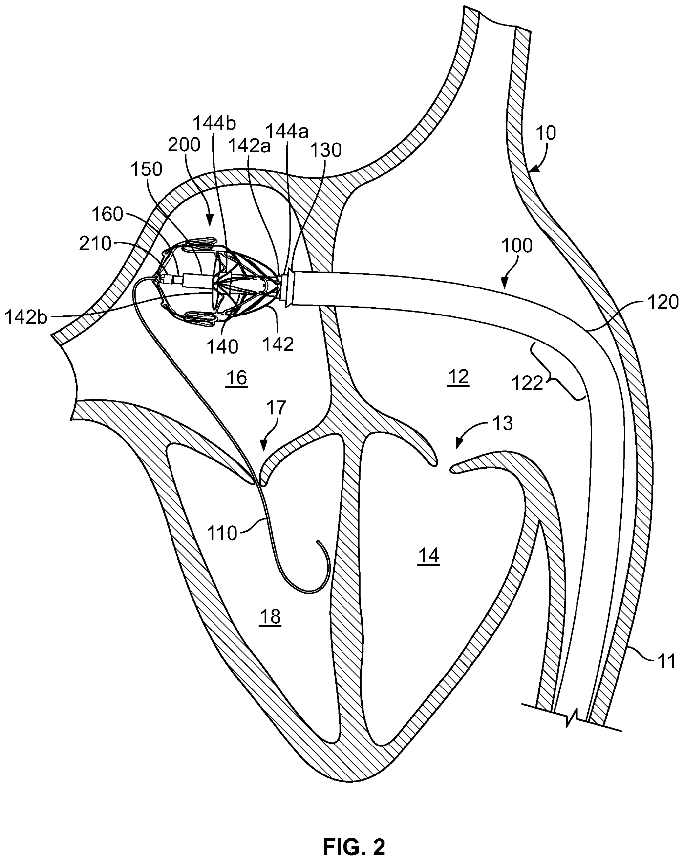

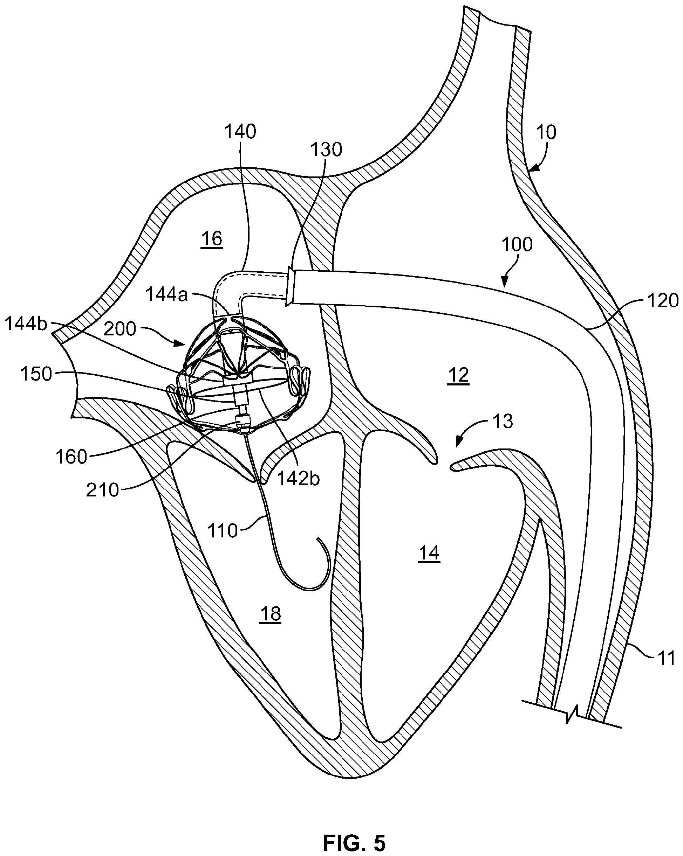

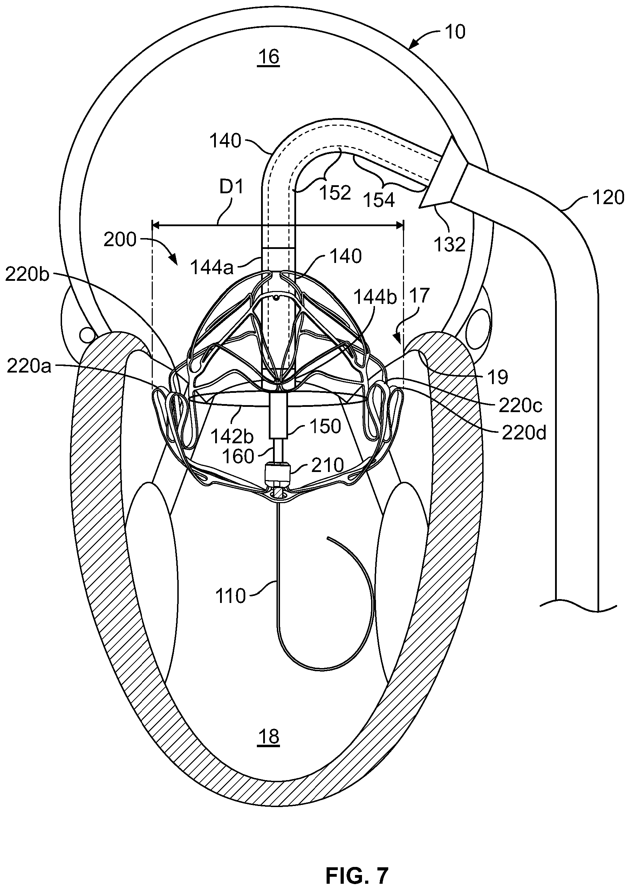

The heart 10 (depicted in cross-section from a posterior perspective in FIG. 1) includes a right atrium 12, a right ventricle 14, a left atrium 16, and a left ventricle 18. A tricuspid valve 13 separates the right atrium 12 from the right ventricle 14. A mitral valve 17 separates the left atrium 16 from the left ventricle 18. An atrial septum 15 separates the right atrium 12 from the left atrium 16. An inferior vena cava 11 is confluent with the right atrium 12. It should be understood that this depiction of the heart 10 is somewhat stylized. The same is true for FIGS. 2 and 5. FIGS. 1, 2 and 5 provide general depictions of the approach to the mitral valve 17 that is used in some implementations. But, the commissural cross-sectional views of FIG. 7 and thereafter more accurately depict the orientation of the prosthetic mitral valves in relation to the heart 10.

Still referring to FIG. 1, in the depicted embodiment, the delivery system 100 includes a guidewire 110, a guide catheter 120, and an anchor delivery sheath 130. Additional components of the delivery system 100 will be described further below. The anchor delivery sheath 130 is slidably (and rotationally) disposed within a lumen of the guide catheter 120. The guidewire 110 is slidably disposed with respect to a lumen of the anchor delivery sheath 130. In this depiction, the anchor delivery sheath 130 has been partially extended relative to the guide catheter 120, allowing an optional flared portion 132 to expand outward, as described further below.

In the depicted implementation, the guidewire 110 is installed into the heart 10 prior to the other components of the delivery system 100. In some embodiments, the guidewire 110 has a diameter of about 0.035 inches (about 0.89 mm). In some embodiments, the guidewire 110 has a diameter in a range of about 0.032 inches to about 0.038 inches (about 0.8 mm to about 0.97 mm). In some embodiments, the guidewire 110 has a diameter smaller than 0.032 inches (about 0.80 mm) or larger than 0.038 inches (about 0.97 mm). In some embodiments, the guidewire 110 is made of materials such as, but not limited to, nitinol, stainless steel, high-tensile-strength stainless steel, and the like, and combinations thereof. The guidewire 110 may include various tip designs (e.g., J-tip, straight tip, etc.), tapers, coatings, covers, radiopaque (RO) markers, and other features. In some embodiments, the guidewire 110 has one or more portions with differing lateral stiffnesses, column strengths, lubricity, and/or other physical properties in comparison to other portions of the guidewire 110.

In some implementations, the guidewire 110 is percutaneously inserted into a femoral vein of the patient. The guidewire 110 is routed to the inferior vena cava 11 and into the right atrium 12. After creating an opening in the atrial septum 15 (e.g., a trans-septal puncture of the fossa ovalis or other portion of the atrial septum), the guidewire 110 is routed into the left atrium 16, and then into the left ventricle 18.

In the depicted implementation, the guide catheter 120 is installed (e.g., via the groin incision 2, refer to FIG. 49) by pushing it (and other components of delivery system 100) over the guidewire 110. In some implementations, a dilator tip is used in conjunction with the guide catheter 120 as the guide catheter 120 is advanced over the guidewire 110. Alternatively, a balloon catheter could be used as the initial dilation means. After the distal end of the guide catheter 120 reaches the left atrium 16, the dilator tip can be withdrawn.

In some embodiments, in order to navigate the guidewire 110 from the left atrium 16 to the left ventricle 18, a catheter with a curved distal tip portion (not shown) is installed over the guidewire 110 within the guide catheter 120. Also, a balloon-tipped catheter (not shown) can be installed over the guidewire 110 within the catheter with the curved distal tip portion. The curved distal tip portion of the catheter can be used to direct the balloon-tipped catheter into the left ventricle 18 (through the mitral valve 17). Such a balloon-tipped catheter can be used advantageously to avoid chordal entanglement as it is advanced through the mitral valve 17. Thereafter, the guidewire 110 can be advanced through the balloon-tipped catheter and into the left ventricle 18. In some implementations, the guidewire 110 can be installed into the heart 10 along other anatomical pathways. The guidewire 110 thereafter serves as a rail over which other components of the delivery system 100 are passed.

By making various adjustments at the proximal end of the guide catheter 120 (as described further below), a clinician can attain a desirable orientation of the guide catheter 120 in relation to the heart 10. For example, the guide catheter 120 can be rotated about its longitudinal axis so that the longitudinal axis of the distal-most tip portion of the guide catheter 120 is pointing toward the perpendicular axis of the mitral valve 17. Such rotational movement of the guide catheter 120 can be performed by the clinician using the deployment system. In addition, in some embodiments a distal end portion of the guide catheter 120 is steerable (also referred to herein as "deflectable"). Using such steering, the distal end portion of the guide catheter 120 can be deflected to navigate the patient's anatomy and/or to be positioned in relation to the patient's anatomy as desired. For example, the guide catheter 120 can be angled within the right atrium 12 to navigate the guide catheter 120 from the inferior vena cava 11 to the atrial septum 15. Accordingly, in some embodiments the guide catheter 120 may include at least one deflection zone 122. As described further below, a clinician can controllably deflect the deflection zone of the guide catheter 120 as desired.

After the guide catheter 120 is oriented within the heart 10 as desired by the clinician, in some embodiments the clinician can releasably lock the guide catheter 120 in the desired orientation. For example, in some embodiments the clinician can releasably lock the guide catheter 120 to a deployment system that is stationary in relation to the patient.

Still referring to FIG. 1, in some embodiments the guide catheter 120 has an outer diameter of about 28 Fr (about 9.3 mm), or about 30 Fr (about 10.0 mm). In some embodiments, the guide catheter 120 has an outer diameter in the range of about 26 Fr to about 34 Fr (about 8.7 mm to about 11.3 mm). In some embodiments, the guide catheter 120 has an outer diameter in the range of about 20 Fr to about 28 Fr (about 6.7 mm to about 9.3 mm).

The guide catheter 120 can comprise a tubular polymeric or metallic material. For example, in some embodiments the guide catheter 120 can be made from polymeric materials such as, but not limited to, polytetrafluoroethylene (PTFE), fluorinated ethylene propylene (FEP), HYTREL^{®}, nylon, PICOFLEX^{®}, PEBAX^{®}, TECOFLEX^{®}, and the like, and combinations thereof. In alternative embodiments, the guide catheter 120 can be made from metallic materials such as, but not limited to, nitinol, stainless steel, stainless steel alloys, titanium, titanium alloys, and the like, and combinations thereof. In some embodiments, the guide catheter 120 can be made from combinations of such polymeric and metallic materials (e.g., polymer layers with metal braid, coil reinforcement, stiffening members, and the like, and combinations thereof). In some embodiments, the guide catheter 120 can comprise a slotted tube.

The example delivery system 100 also includes the anchor delivery sheath 130. In some implementations, after the guide catheter 120 is positioned with its distal end in the left atrium 16, the anchor delivery sheath 130 is installed into a lumen of the guide catheter 120 (over the guidewire 110) and advanced through the guide catheter 120. As described further below, in some embodiments the anchor delivery sheath 130 is preloaded with a prosthetic valve anchor assembly and other components of the delivery system 100.

In some embodiments, the anchor delivery sheath 130 can be made from the materials described above in reference to the guide catheter 120. In some embodiments, the anchor delivery sheath 130 has an outer diameter in the range of about 20 Fr to about 28 Fr (about 6.7 mm to about 9.3 mm). In some embodiments, the anchor delivery sheath 130 has an outer diameter in the range of about 14 Fr to about 24 Fr (about 4.7 mm to about 8.0 mm).

In the depicted embodiment, the anchor delivery sheath 130 includes a flared distal end portion 132. In some embodiments, an inverted-flare distal end portion is included. In some embodiments, no such flared distal end portion 132 is included. The flared distal end portion 132 can collapse to a lower profile when constrained within the guide catheter 120. When the flared distal end portion 132 is expressed from the guide catheter 120, the flared distal end portion 132 can self-expand to the flared shape. In some embodiments, the material of the flared distal end portion 132 includes pleats or folds, may be a continuous flared end or may be separated into sections resembling flower petals, and may include one or more resilient elements that bias the flared distal end portion 132 to assume the flared configuration in the absence of restraining forces (such as from containment within the guide catheter 120). The flared distal end portion 132 can be advantageous, for example, for recapturing (if desired) the anchor assembly within the lumen of the anchor delivery sheath 130 after the anchor assembly has been expressed from the flared distal end portion 132. In some embodiments, a distal-most portion of the flared distal end portion 132 is everted (which can serve to help facilitate recapture of the anchor delivery sheath 130). In some cases, the recapture of the anchor assembly will cause a portion of the flared distal end portion 132 to become everted.

In some embodiments, the maximum outer diameter of the flared distal end portion 132 is in a range of about 30 Fr to about 34 Fr (about 10.0 mm to about 11.3 mm). In some embodiments, the maximum outer diameter of the flared distal end portion 132 is in a range of about 32 Fr to about 44 Fr (about 10.7 mm to about 14.7 mm). In some embodiments, the maximum outer diameter of the flared distal end portion 132 is in a range of about 24 Fr to about 30 Fr (about 8.0 mm to about 10.0 mm). In some embodiments, the maximum outer diameter of the flared distal end portion 132 is less than about 24 Fr (about 8.0 mm) or greater than about 44 Fr (about 14.7 mm).

Referring to **FIG. 2**, additional components of the example delivery system 100 can include an anchor delivery catheter 140, a secondary steerable catheter 150, and an inner catheter 160. The anchor delivery catheter 140 is slidably disposed within a lumen of the anchor delivery sheath 130. The secondary steerable catheter 150 is slidably disposed within a lumen of the anchor delivery catheter 140. The inner catheter 160 is slidably disposed within a lumen of the secondary steerable catheter 150. The guidewire 110 is slidably disposed within a lumen of the inner catheter 160.

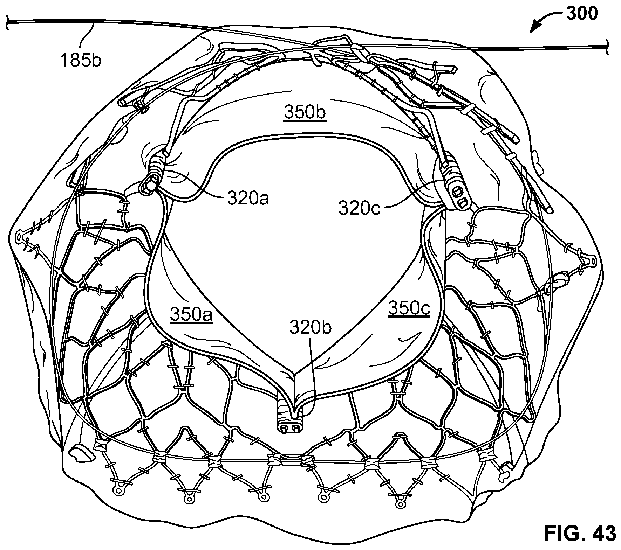

An anchor assembly 200 (shown without covering materials for enhanced visibility) is releasably attached to the inner catheter 160 and is, in effect, slidably disposed on the guidewire 110. As described further below, the components of the delivery system 100 can be individually or jointly manipulated by a clinician operator to control the position and orientation of the anchor assembly 200 during the deployment of the anchor assembly 200. In some embodiments, the inner catheter 160 has a filar construct to advantageously configure the inner catheter 160 to transmit torsion forces. In some implementations, a deployment frame system (such as the example deployment frame system in FIG. 43 described below) is used to initiate and/or control the movements of various components of the transcatheter delivery system 100.

In a preferred implementation of delivery system 100, the anchor delivery catheter 140, the secondary steerable catheter 150, the inner catheter 160, and the anchor assembly 200 are loaded into the anchor delivery sheath 130 prior to the advancement of the anchor delivery sheath 130 into the guide catheter 120 as shown in FIG. 1. That is, in a preferred implementation the anchor delivery catheter 140, the secondary steerable catheter 150, the inner catheter 160, and/or the anchor assembly 200 are already installed in the anchor delivery sheath 130 as the anchor delivery sheath 130 is distally advanced into the guide catheter 120 to attain the arrangement shown in FIG. 1. Then the anchor delivery sheath 130 is individually pulled back (proximally) to reveal the anchor delivery catheter 140, the secondary steerable catheter 150, the inner catheter 160, and/or the anchor assembly 200 as shown in FIG. 2. The anchor assembly 200 may also be at least partially expanded. In some such implementations, the anchor delivery catheter 140, the secondary steerable catheter 150, the inner catheter 160, and/or the anchor assembly 200 are loaded into the anchor delivery sheath 130 in desired relative rotational orientations (i.e., rotational orientations about the longitudinal axis of the delivery system 100). In other implementations, one or more of the anchor delivery catheter 140, the secondary steerable catheter 150, the inner catheter 160, and the anchor assembly 200 are distally advanced into the anchor delivery sheath 130 after the anchor delivery sheath 130 has been advanced into the guide catheter 120 to attain the arrangement shown in FIG. 1.

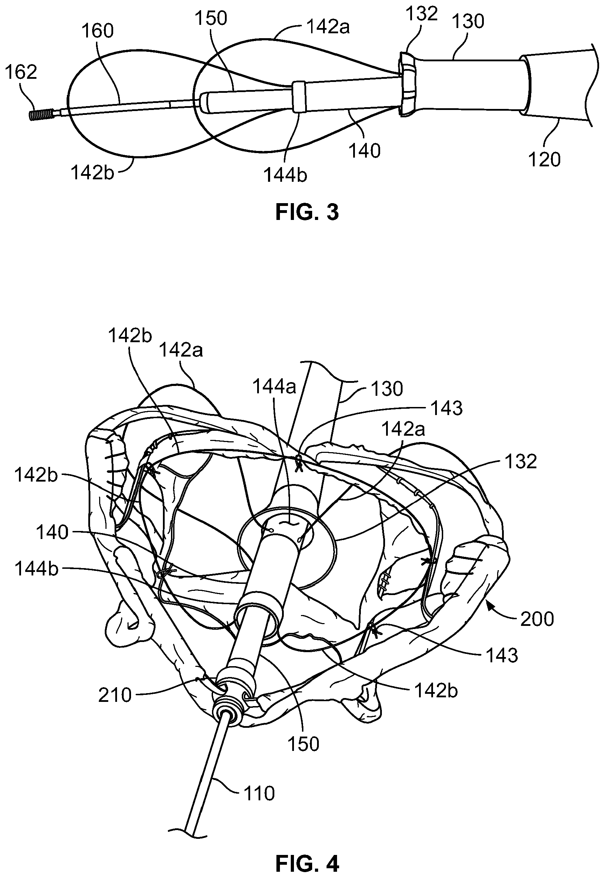

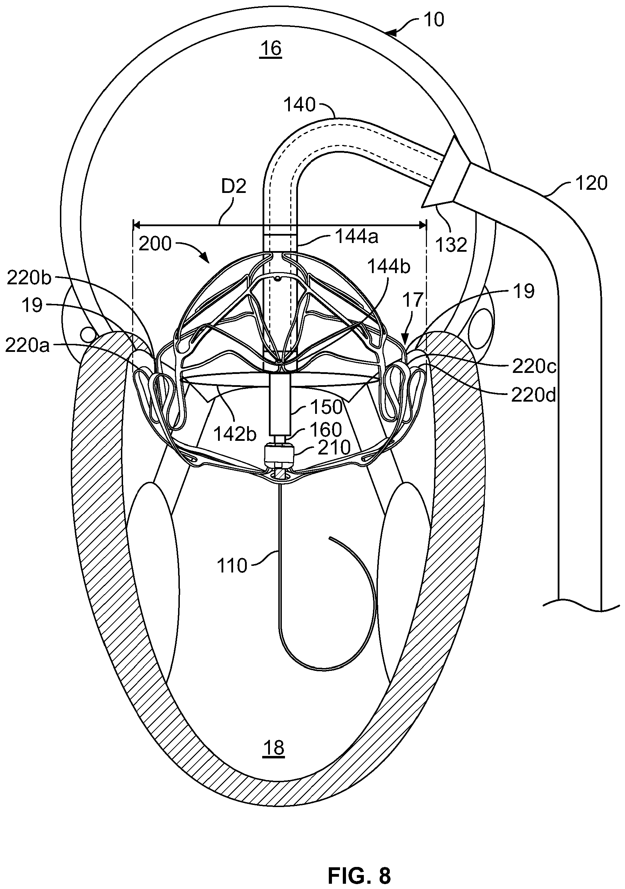

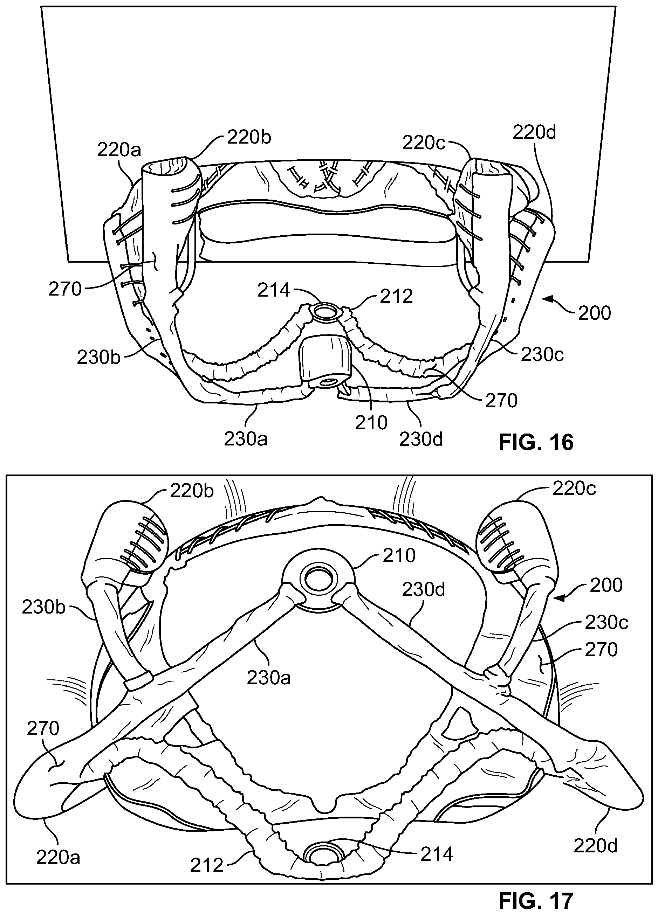

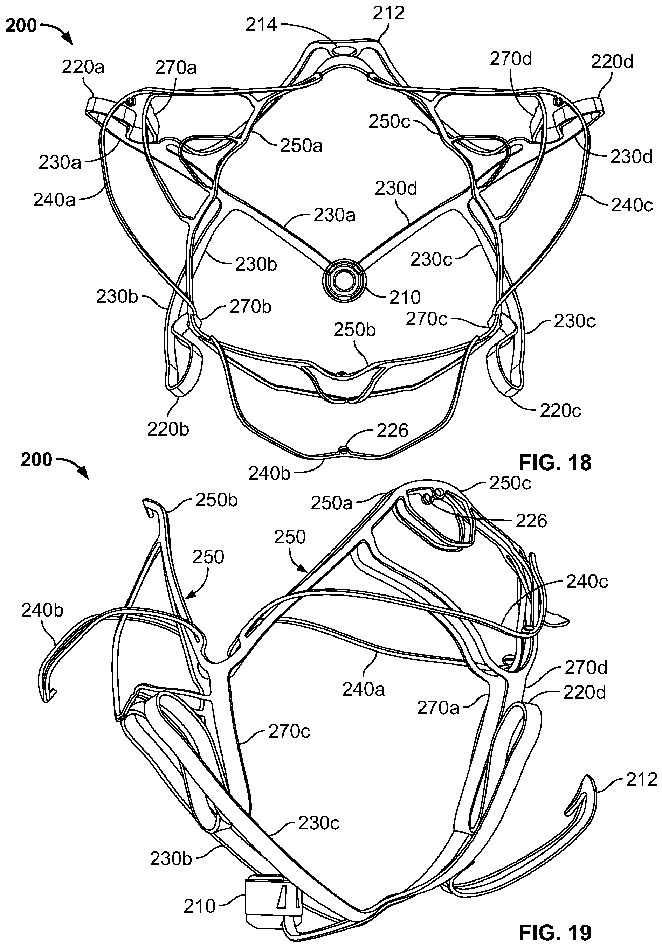

The inner catheter 160 is releasably coupled with a hub 210 of the anchor assembly 200. In some such embodiments, the inner catheter 160 has a threaded distal tip portion 162 (FIG. 3) that threadably engages with a complementary threaded portion of the hub 210. In some embodiments, as described further below, the inner catheter 160 is also releasably coupled with a SAM containment member 212 (refer, for example, to FIGS. 8 and 19) of the anchor assembly 200. For example, in some embodiments the threaded distal tip portion 162 of the inner catheter 160 is threadably engaged with a complementary threaded eyelet 214 (e.g., FIGS. 16 and 17) of the SAM containment member 212. When a clinician operator desires to uncouple the inner catheter 160 from the SAM containment member 212 and/or the hub 210, the clinician can apply a torque to the inner catheter 160 to unscrew the threaded distal tip portion 162 from the eyelet 214 and/or the hub 210. In some embodiments, the inner catheter 160 is a filar construct so as to configure the inner catheter 160 to transmit a torque to facilitate uncoupling the inner catheter 160 from the SAM containment member 212 and/or the hub 210. In some embodiments, other types of mechanisms are used to releasably couple the delivery system 100 to one or more portions of the anchor assembly 200.

One or more portions of the anchor assembly 200 can also be releasably coupled to one or more catheters of the delivery system 100 by one or more control wires. The one or more control wires can be used to control the anchor assembly 200 (e.g., to control the configuration of the anchor assembly 200). For example, the one or more control wires can be used for controlling the diametrical expansion of a self-expanding anchor assembly 200 and/or for controlling the deployment of particular features of the anchor assembly 200. In the depicted embodiment, a proximal portion of the anchor assembly 200 is releasably coupled to the anchor delivery catheter 140 by a proximal control wire 142a, and a mid-body portion of the anchor assembly 200 is releasably coupled to the anchor delivery catheter 140 by a mid-body control wire 142b.

Referring also to **FIGS. 3 and 4****,** in the depicted embodiment the proximal control wire 142a emerges from and reenters into the anchor delivery catheter 140 at a proximal collar 144a that is integral with the anchor delivery catheter 140, and the distal control wire 142b emerges from and reenters into the anchor delivery catheter 140 at a distal collar 144b that is integral with the anchor delivery catheter 140. In some embodiments, the control wires 142a and 142b pass through lumens in the wall of the anchor delivery catheter 140, and travel proximally to the deployment control system (e.g., the example deployment frame system shown in FIG. 43). The two ends of each of the control wires 142a and 142b can be terminated at the deployment control system. At such a deployment control system, the tension on the control wires 142a and 142b can be manipulated by a clinician to control the configuration of the anchor assembly 200. In this example, by tightening the control wires 142a and/or 142b, the anchor assembly 200 will be diametrically contracted, and by loosening the control wires 142a and/or 142b, the anchor assembly 200 will be permitted to diametrically self-expand (for example, so that each control wire 142a and 142b can be operated somewhat similar to an adjustable lasso to control expansion of different portions of the anchor assembly at different stages). When the clinician is satisfied with the deployment orientation of the anchor assembly 200, the control wires 142a and 142b can be decoupled from the anchor assembly 200 by the clinician. To do so, the clinician can release one end of the control wire 142a and/or 142b and pull on the other end so that the control wire 142a and/or 142b becomes disengaged with the anchor assembly 200.

FIG. 4 shows how the control wires 142a and 142b can be releasably coupled with the anchor assembly 200 in some embodiments. It should be understood that this is merely one exemplary control wire coupling arrangement and various other arrangements for coupling one or more control wires to the anchor assembly 200 are also envisioned within the scope of this disclosure. Various types of attachment elements can be used to releasably couple the control wires 142a and 142b to the anchor assembly 200. In the depicted embodiment, suture loops 143 are used as the attachment elements. The suture loops 143 can be constructed of materials such as, but not limited to, ultra-high molecular weight polyethylene, nylon, polypropylene, polybutester, and the like. In some embodiments, two suture loops 143 are used in each location to provide redundancy. The suture loops 143 may be coupled with eyelets on the anchor assembly 200 in some cases. In some embodiments, other types of attachment elements such as, but not limited to, eyelets, grommets, rings, clips, pins, fabric portions, and/or the like, are used as attachment elements.

In the depicted embodiment, the proximal control wire 142a is releasably coupled with attachment elements associated with structural features located at the proximal end of the anchor assembly 200. For example, the proximal control wire 142a is releasably coupled with attachment elements of three arched atrial holding features 240a, 240b, and 240c (e.g., refer to FIGS. 18-21) and three frame lobes 250a, 250b, and 250c (e.g., refer to FIGS. 18-21) of the anchor assembly 200. That is, the proximal control wire 142a emerges from the anchor delivery catheter 140 at the proximal collar 144a, passes through the attachment elements of the three arched atrial holding features 240a, 240b, and 240c, and the three frame lobes 250a, 250b, and 250c, and reenters the anchor delivery catheter 140 at the proximal collar 144a. By applying tension to the proximal control wire 142a, the three arched atrial holding features 240a, 240b, and 240c, and the three frame lobes 250a, 250b, and 250c can be diametrically drawn inward towards the anchor delivery catheter 140. In the arrangement depicted in FIG. 2, for example, the three arched atrial holding features 240a, 240b, and 240c, and the three frame lobes 250a, 250b, and 250c are drawn in very closely to the anchor delivery catheter 140.

In the depicted embodiment, the mid-body control wire 142b is releasably coupled with attachment elements associated with structural features of the anchor assembly 200 located at the longitudinal middle region of the anchor assembly 200. For example, the mid-body control wire 142b is releasably coupled with attachment elements of four inter-annular connections 270a, 270b, 270c, and 270d (e.g., refer to FIGS. 18-21) and a mid-body portion of the supra-annular ring 250 of the anchor assembly 200. That is, the mid-body control wire 142b emerges from the anchor delivery catheter 140 at the distal collar 144b, passes through the attachment elements of the four inter-annular connections 270a, 270b, 270c, and 270d, and the mid-body portion of the supra-annular ring 250, and reenters the anchor delivery catheter 140 at the distal collar 144b. By applying tension to the mid-body control wire 142b, the four inter-annular connections 270a, 270b, 270c, and 270d, and the mid-body portion of the supra-annular ring 250 can be diametrically drawn inward towards the anchor delivery catheter 140. In the arrangement depicted in FIG. 2, the four inter-annular connections 270a, 270b, 270c, and 270d, and the mid-body portion of the supra-annular ring 250 are drawn in toward the anchor delivery catheter 140 such that the diameter of the anchor assembly 200 is less than the fully expanded diameter.

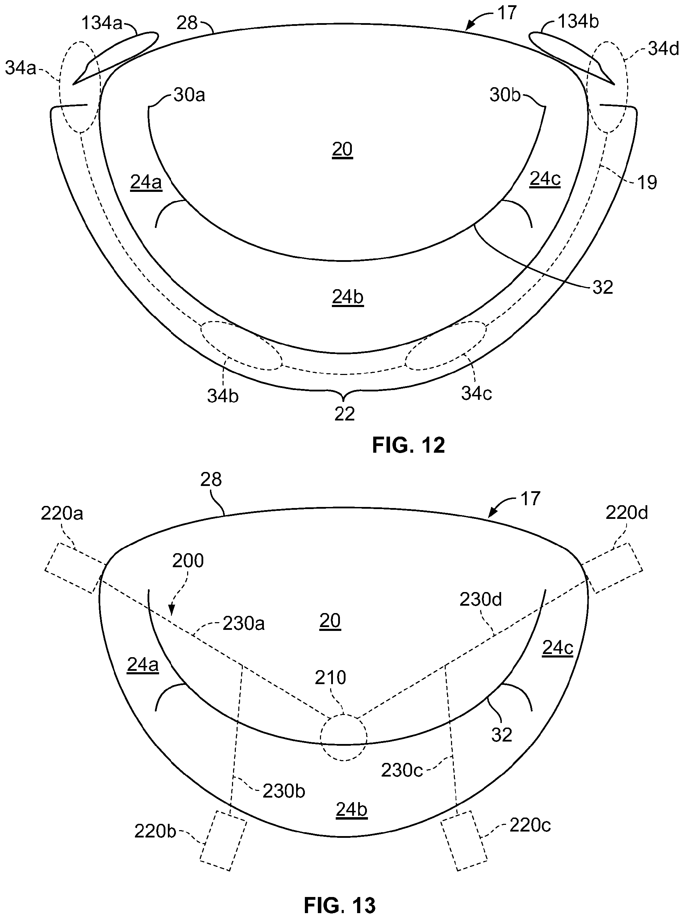

Diametric control of the anchor assembly 200 by manipulation of the tension of the mid-body control wire 142b can be advantageously utilized by a clinician during the deployment of the anchor assembly 200. For example, as described further below, the steps of advancing the anchor assembly 200 through the annulus of the native mitral valve and seating anchor feet 220a, 220b, 220c, and 220d (e.g., refer to FIGS. 18-21) in the sub-annular gutter 19 (FIG. 12) can be facilitated using the diametric control afforded by the mid-body control wire 142b.

While the depicted embodiment includes two control wires 142a and 142b, in some embodiments one, three, four, five, or more than five control wires are included. A clinician can separately control the two control wires 142a and 142b. For example, in some embodiments the mid-body control wire 142b may be partially or fully loosened while the proximal control wire 142a is maintained in a state of full tension. In some implementations, a deployment frame system (such as the example deployment frame system of FIG. 49 described below) is used to control the tension and movements of the two control wires 142a and 142b.

Still referring to FIG. 2, while the components of the delivery system 100 and the anchor assembly 200 are depicted in particular relative orientations and arrangements, it should be understood that the depictions are non-limiting. For example, in some implementations of the deployment process the distal tip of the secondary deflectable catheter 150 may always be, or may sometimes be, abutted to the hub 210 of the anchor assembly 200. Further, in some implementations of the deployment process the distal tip of the anchor delivery catheter 140 may always be, or may sometimes be, positioned within the interior of the anchor assembly 200. In some implementations, a deployment frame system (such as the example deployment frame system of FIG. 49 described below) is used to control such relative arrangements and movements of the anchor delivery catheter 140 and secondary deflectable catheter 150 in relation to the anchor assembly 200, for example.

In some embodiments, the position of the anchor assembly 200 can be controlled by manipulating the relative positions of the inner catheter 160 and/or the anchor delivery catheter 140. For example, in the depicted embodiment the anchor assembly 200 can be expressed out from the anchor delivery sheath 130 (as shown in FIG. 2) by moving the inner catheter 160 and/or the anchor delivery catheter 140 distally in relation to the anchor delivery sheath 130. In some implementations, the expression of the anchor assembly 200 is caused by proximally pulling back the anchor delivery sheath 130 while generally maintaining the positions of the inner catheter 160 and/or the anchor delivery catheter 140. In some implementations, the expression of the anchor assembly 200 is caused by a combination of proximally pulling back the anchor delivery sheath 130 while distally extending the positions of the inner catheter 160 and/or the anchor delivery catheter 140.

As the anchor assembly 200 emerges from the confines of the anchor delivery sheath 130, the anchor assembly 200 may expand from a low-profile delivery configuration to an at least partially expanded configuration (for example, a partially expanded condition, as shown in FIG. 2, that is less that its fully expanded condition as described in more detail below). In addition to control by manipulation of the mid-body control wire 142b, the extent of expansion of the anchor assembly 200 can also be at least partially controlled by the relative positioning of the anchor delivery catheter 140 in relation to the inner catheter 160. For instance, as the anchor delivery catheter 140 is moved proximally in relation to the inner catheter 160, the anchor assembly 200 is axially elongated and radially contracted. Conversely, as the anchor delivery catheter 140 is moved distally in relation to the inner catheter 160, the anchor assembly 200 is axially shortened and radially expanded. In some implementations, this control of the radial size of the anchor assembly 200 is used by a clinician during the process of deploying the anchor assembly 200 within the native mitral valve 17, as described further below. As described above, the one or more control wires 142a and 142b can also be used to control diametrical expansion of the anchor assembly 200 (without changing the relative distance of the anchor delivery catheter 140 in relation to the inner catheter 160).

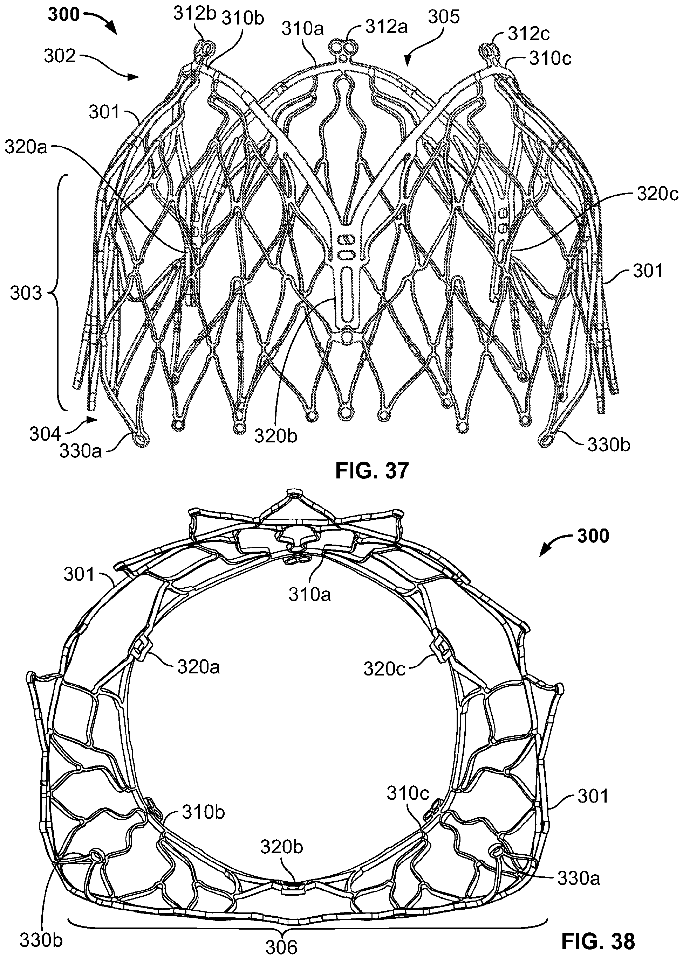

It should be understood that the prosthetic mitral valves provided herein are comprised of an anchor assembly 200 and a separate valve assembly (e.g., refer to FIG. 37). The anchor assembly 200 is deployed to an arrangement interfacing within the native mitral valve 17 prior to deployment of the valve assembly. Said differently, after implanting the anchor assembly 200 within the native mitral valve 17, the valve assembly can then be deployed within the anchor assembly 200 and within the native mitral valve 17 (as described further below). Therefore, it can be said that the prosthetic mitral valves provided herein are deployed using a staged implantation method. That is, the anchor assembly 200 is deployed in one stage, and the valve assembly is deployed in a subsequent stage. In some embodiments, as described further below, the SAM containment member 212 is also deployed as part of the deployment method. In some implementations, the deployment of the valve assembly takes place right after the deployment of the anchor assembly 200 (e.g., during the same medical procedure). In some implementations, the deployment of the valve assembly takes place hours, days, weeks, or even months after the deployment of the anchor assembly 200 (e.g., during a subsequent medical procedure).

The staged implantation method of the prosthetic mitral valves provided herein is facilitated by the fact that when the anchor assembly 200 itself is implanted within the native mitral valve 17, the native mitral valve 17 continues to function essentially as before the implantation of the anchor assembly 200 without a significant impact on cardiovascular physiology. That is the case because, as described further below, the anchor assembly 200 interfaces and anchors within structural aspects of the native mitral valve 17 without substantially interfering with the leaflets or chordae tendineae of the native mitral valve 17.

Still referring to FIG. 2, in the depicted arrangement the distal end portion of the secondary steerable catheter 150 is located at least partially internally within the anchor assembly 200. The secondary steerable catheter 150 can be manipulated by a clinician operator to reversibly bend (deflect) the distal end portion of the secondary steerable catheter 150. As the secondary steerable catheter 150 is bent by the clinician, other components of the delivery system 100 may deflect along with the secondary steerable catheter 150. For example, portions of one or more of the inner catheter 160 and the anchor delivery catheter 140 may bend in response to the bending of the deflectable catheter 150. Because the anchor assembly 200 is coupled to the inner catheter 160 and the anchor delivery catheter 140, the anchor assembly 200 can, in turn, be pivoted or "panned" by bending the secondary steerable catheter 150.

Referring to **FIG. 5****,** as described above, in some embodiments the secondary steerable catheter 150 can be articulated (also referred to as "steered," "deflected," "bent," "curved," and the like) to orient the anchor assembly 200 in relation to the mitral valve 17 as desired. That is, in some embodiments the secondary steerable catheter 150 has one or more deflection zones at a distal end portion of the secondary steerable catheter 150. For example, in the depicted embodiment the secondary steerable catheter 150 has two deflection zones 152 and 154 (refer to FIG. 7) at the distal end portion of the secondary steerable catheter 150. In some embodiments, the two deflection zones 152 and 154 allow for deflection of the distal end portion of the secondary steerable catheter 150 within two separate and distinct planes. For example, in the depicted embodiment deflection zone 152 allows for deflection of the distal end portion of the secondary steerable catheter 150 generally within the plane of FIGS. 1, 2, and 5, while deflection zone 154 allows for deflection of the distal end portion of the secondary steerable catheter 150 generally orthogonal to the plane of FIGS. 1, 2, and 5. In some implementations, a deployment frame system (such as the example deployment frame system of FIG. 43 described below) is used to initiate and control such deflection of the secondary steerable catheter 150, including deflection of the distal end portion of the secondary steerable catheter 150 within two separate and distinct planes, individually.

In some implementations, it is desirable to orient (e.g., laterally pivot, pan, etc.) the anchor assembly 200 within the atrium 16 so that the longitudinal axis of the anchor assembly 200 is generally perpendicular to the native mitral valve 17, and coaxial with the native mitral valve 17 (e.g., to center the anchor assembly 200 with the line or coaptation of the mitral valve 17). The orienting of the partially or fully expanded anchor assembly 200 within the atrium 16 may be advantageous versus having to orient the anchor assembly 200 while it is still constrained within a delivery sheath, as the latter assembly is a relatively large and stiff catheter assembly.

In some implementations, the anchor assembly 200 within the atrium 16 can be additionally, or alternatively, oriented in relation to the native mitral valve 17 by rotating the guide catheter 120 about its longitudinal axis. Such a rotation of the guide catheter 120 about its longitudinal axis can result in a directional adjustment of the longitudinal axis of the distal tip portion of the guide catheter 120. That is, rotation of the guide catheter 120 about its longitudinal axis can result in pointing the distal tip portion of the guide catheter 120 (and the components of the delivery system 100) in a desired direction within the atrium 16. In some implementations, a deployment frame system is used to initiate and control such rotation of the guide catheter 120 about its longitudinal axis.

In some implementations, the relative rotational alignment of the anchor assembly 200 in relation to the mitral valve 17 can be adjusted as desired in preparation for engaging the anchor assembly 200 with the native mitral valve 17. For example, in some implementations the anchor assembly 200 can be rotated about its longitudinal axis by rotating the inner catheter 160 and the anchor delivery catheter 140 generally in unison, while keeping the secondary steerable catheter 150 essentially stationary. In some implementations, a deployment frame system (such as the example deployment frame systems described below) is used to initiate and control such rotation of the anchor assembly 200 about its longitudinal axis.

In preparation for engaging the anchor assembly 200 with the native mitral valve 17, the clinician operator may manipulate the radial size of the anchor frame 200 so that the anchor frame 200 can be passed through the native mitral valve 17 without damaging the native mitral valve 17. For example, the clinician can diametrically expand or retract one or more portions of the anchor assembly 200 by manipulation of the mid-body control wire 142b. Alternatively, or additionally, the clinician can move the anchor delivery catheter 140 proximally in relation to the inner catheter 160 to radially contract the anchor assembly 200. With the anchor assembly 200 configured in a desired diametrical size, and appropriately aligned with the mitral valve 17, the anchor frame 200 can be safely passed through the native mitral valve 17 without damaging the native mitral valve 17 and/or entangling chordae tendineae of the mitral valve 17. Moreover, by controlling the diametrical size of the anchor assembly 200 to just slightly less than the size of the annulus of the mitral valve 17, an advantageous natural centering of the anchor assembly 200 can occur as the sub-annular portions of the anchor assembly 200 are advanced through the mitral valve 17.

Referring to **FIG. 7**, a commissural cross-sectional view of the heart 10 provides another perspective of the anchor assembly 200 in relation to the native mitral valve 17. This commissural cross-sectional view of the heart 10 is a cross-sectional view taken through the mitral valve 17 along a plane through the left atrium 16 and left ventricle 18 that is parallel to the line that intersects the two commissures of the mitral valve. In the following FIGS. 8-11 and 25-36, the commissural cross-sectional view of the heart 10 will be used to describe the delivery system 100 and methods for deploying the prosthetic mitral valves provided herein. The view in FIGS. 7-11 and 25-36 is slightly tilted so that better visualization of the anchor assembly 200 is provided.

While the secondary steerable catheter 150 is retained in its bent (deflected) configuration as described in reference to FIG. 5, the inner catheter 160 and the anchor delivery catheter 140 can be simultaneously advanced. Because the inner catheter 160 is releasably coupled to the hub 210 of the anchor assembly 200, and because the anchor delivery catheter 140 is releasably coupled to the proximal end and the mid-body region of the anchor assembly 200 via the control wires 142a and 142b, generally simultaneous advancement of the inner catheter 160 and the anchor delivery catheter 140 results in advancement of the anchor assembly 200.

In preparation for the advancement of the distal portions of the anchor assembly 200 through the annulus of the mitral valve 17, the mid-body control wire 142b can be manipulated to adjust a mid-body diameter D1 of the anchor assembly 200 to a desired size. For example, in some implementations it is desirable to adjust the mid-body diameter D1 to size that is slightly smaller than the size of the annulus of the mitral valve 17. In such a case, while advancing the distal portions of the anchor assembly 200 through the annulus of the mitral valve 17, a self-centering of the anchor assembly 200 in relation to the mitral valve 17 may naturally occur.

As depicted, the anchor assembly 200 is advanced such that the distal end portions of anchor assembly 200 are positioned within the left ventricle 18 while the proximal end portions of the anchor assembly 200 remain positioned within the left atrium 16. Hence, some portions of the anchor assembly 200 are on each side of the native mitral valve 17. Said differently, the deployed anchor assembly 200 includes supra-annular portions and sub-annular portions.

In the depicted embodiment, the anchor assembly 200 includes four anchor feet: a lateral anterior foot 220a, a lateral posterior foot 220b, a medial posterior foot 220c, and a medial anterior foot 220d (refer also to FIGS. 18-21). In some embodiments, fewer or more anchor feet may be included (e.g., two, three, five, six, or more than six). In some embodiments, the anchor feet 220a, 220b, 220c, and 220d are portions of the anchor assembly 200 that are configured for contact with a sub-annular gutter 19 (also refer to FIG. 12) of the native mitral valve 17, without penetrating tissue of the native mitral valve 17. Accordingly, the anchor feet 220a, 220b, 220c, and 220d have atraumatic surfaces that are generally comparable to feet. However, in some embodiments one or more of the anchor feet 220a, 220b, 220c, and 220d are configured to penetrate tissue and may have anchor features such as barbs, coils, hooks, and the like.

In the arrangement of FIG. 7, the anchor feet 220a, 220b, 220c, and 220d are positioned below the sub-annular gutter 19. In this arrangement then, the mid-body diameter D1 of the anchor assembly 200 can thereafter be increased to align the anchor feet 220a, 220b, 220c, and 220d with the sub-annular gutter 19. For example, in some embodiments the mid-body control wire 142b positioned on or around the mid-body portion of the anchor assembly 200 can be manipulated (e.g., slackened) to allow radial self-expansion of the anchor assembly 200, to align the anchor feet 220a, 220b, 220c, and 220d with the sub-annular gutter 19. Alternatively, or additionally, in some embodiments the clinician can move the anchor delivery catheter 140 distally in relation to the inner catheter 160 to radially expand the anchor assembly 200 to align the anchor feet 220a, 220b, 220c, and 220d with the sub-annular gutter 19. Such alignment can be performed in preparation for seating the anchor feet 220a, 220b, 220c, and 220d within the sub-annular gutter 19.

Referring to **FIG. 8****,** the anchor feet 220a, 220b, 220c, and 220d are positioned below the sub-annular gutter 19. In this position, the anchor feet 220a, 220b, 220c, and 220d are positioned under the systolic and diastolic excursions of the leaflets of the native mitral valve 17.

With the anchor feet 220a, 220b, 220c, and 220d positioned below the sub-annular gutter 19, the anchor feet 220a, 220b, 220c, and 220d can be aligned with the sub-annular gutter 19 in preparation for seating the anchor feet 220a, 220b, 220c, and 220d within the sub-annular gutter 19. For example, to align the anchor feet 220a, 220b, 220c, and 220d with the sub-annular gutter 19, in some implementations tension from the mid-body control wire 142b can be relieved by the clinician to allow the mid-body diameter to expand from D1 (FIG. 7) to D2. When the anchor assembly 200 has a mid-body diameter D2, the anchor feet 220a, 220b, 220c, and 220d are posed in diametrical positions for seating within the sub-annular gutter 19.

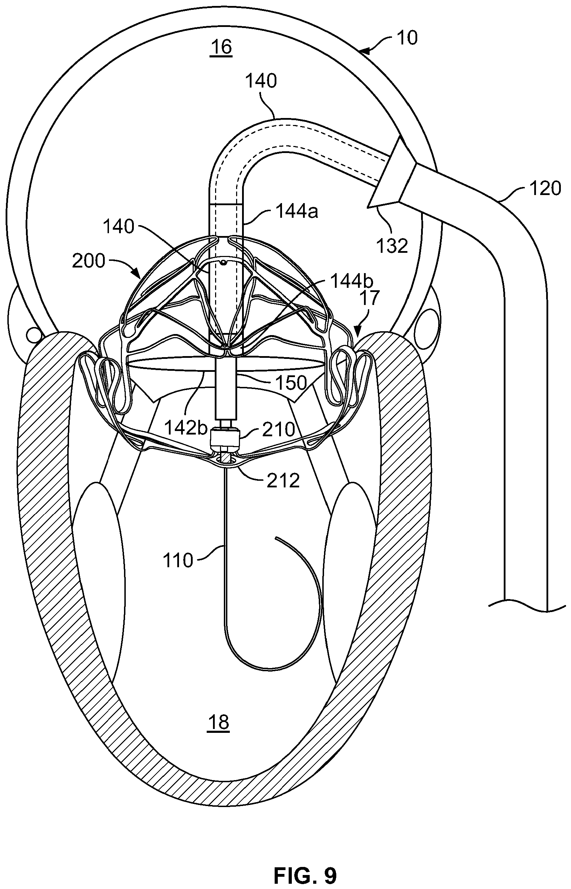

Referring to **FIG. 9****,** the inner catheter 160 and the anchor delivery catheter 140 can be simultaneously retracted while maintaining the secondary steerable catheter 150 and the guide catheter 120 in fixed positions. As a result, the anchor feet 220a, 220b, 220c, and 220d become seated in the sub-annular gutter 19. As described further below, simultaneous movement of two or more components of the delivery system 100 (e.g., the inner catheter 160 in conjunction with the anchor delivery catheter 140, while maintaining the secondary steerable catheter 150 and the guide catheter 120 in fixed positions) can be initiated and controlled using a deployment frame system (such as the example deployment frame system of FIG. 49 described below).

With the anchor feet 220a, 220b, 220c, and 220d seated in the sub-annular gutter 19, the anchor feet 220a, 220b, 220c, and 220d are positioned under the systolic and diastolic excursions of the leaflets of the native mitral valve 17, and the other structures of the anchor assembly 200 do not inhibit the movements of the leaflets. Therefore, with the anchor assembly 200 coupled to the structures of the mitral valve 17 as described, the mitral valve 17 can continue to function as it did before the placement of the anchor assembly 200. In addition, the manner in which the anchor assembly 200 interfaces with the native mitral valve 17 does not result in deformation of the native mitral valve 17. With the SAM containment member 212 in its pre-deployed configuration, the SAM containment member 212 does not affect the natural function of the native mitral valve 17. Therefore, the native mitral valve 17 can continue to function as it did before the placement of the anchor assembly 200.

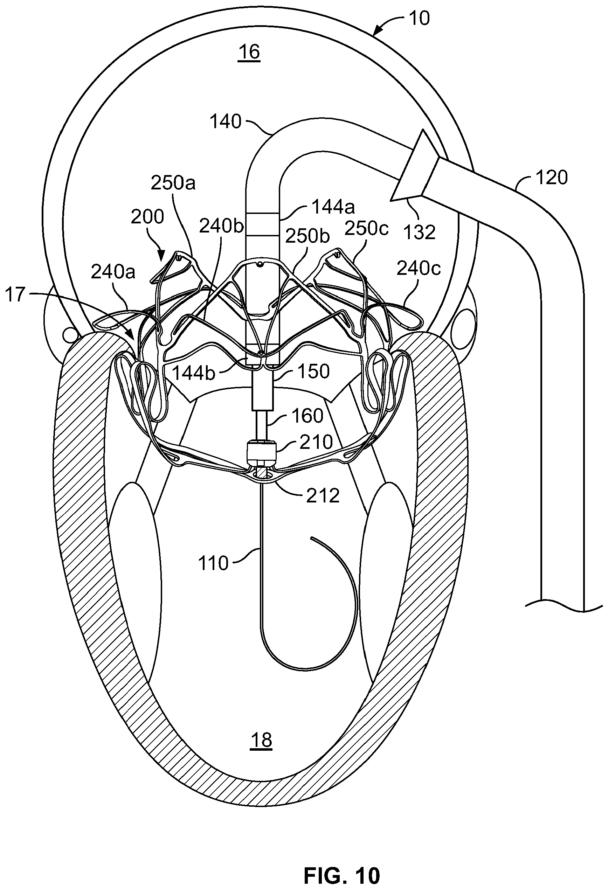

Referring to **FIG. 10****,** with the anchor assembly 200 engaged within the native mitral valve 17, components of the delivery system 100 can be uncoupled from the anchor assembly 200. For example, the one or more control wires 142a and 142b (FIGS. 2-5 and 7-9) can be uncoupled from the anchor assembly 200 (e.g., from the mid-body and proximal end portions of the anchor assembly 200 in some embodiments). As described further below, in some embodiments the frame members of the anchor assembly 200 can be made of an elastic or a super-elastic material with shape memory such that portions of the anchor assembly 200 self-expand/deploy to intended orientations in the absence of constraining forces, such as constraining forces from the control wires 142a and/or 142b.

In the depicted embodiment, when the mid-body control wire 142b is uncoupled from the anchor assembly 200, the mid-body regions of the anchor assembly 200 are no longer diametrically constrained by the mid-body control wire 142b. Hence, mid-body regions of the anchor assembly 200 are allowed to diametrically expand when the mid-body control wire 142b is uncoupled from the anchor assembly 200.

When the proximal control wire 142a is loosened and/or detached from one or more proximal end portions of the anchor assembly 200, the one or more portions that were coupled to the proximal control wire 142a become free to expand and deploy to intended orientations in relation to the mitral valve 17. For example, in the depicted embodiment, the proximal control wire 142a was coupled to three arched atrial holding features 240a, 240b, and 240c. When the proximal control wire 142a is uncoupled (e.g., slid out from or "un-lassoed") from the three arched atrial holding features 240a, 240b, and 240c, the three arched atrial holding features 240a, 240b, and 240c are free to deploy to their intended orientations in relation to the mitral valve 17. The three arched atrial holding features 240a, 240b, and 240c deploy generally radially outward (transversely) in relation to the longitudinal axis (the axis extending between the proximal and distal ends of the anchor assembly 200) of the anchor assembly 200. Hence, in the depicted embodiment the three arched atrial holding features 240a, 240b, and 240c self-deploy to respective positions directly adjacent to, or spaced apart just above, the annulus of the mitral valve 17. In those positions, the three arched atrial holding features 240a, 240b, and 240c resist migration of the anchor assembly 200 towards the left ventricle 18.

In addition, in the depicted embodiment when the proximal control wire 142a is loosened and subsequently detached from the three frame lobes 250a, 250b, and 250c, the three frame lobes 250a, 250b, and 250c become free to expand and deploy to intended orientations. In the depicted embodiment the three frame lobes 250a, 250b, and 250c diametrically expand into positions that are designed to interface with a valve assembly that will be deployed into a mating arrangement with the anchor assembly 200 as described further below.

In the depicted arrangement, the anchor assembly 200 is deployed in engagement with the native mitral valve 17. Nevertheless, the native mitral valve 17 is free to function normally. Moreover, in the depicted arrangement, while the inner catheter 160 is still coupled with the anchor assembly 200 at the hub 210, the anchor delivery catheter 140 (and other components of the transcatheter delivery system 100) are no longer attached to the anchor assembly 200. Hence, some components of the transcatheter delivery system 100 that were used to deploy the anchor assembly 200 can now be retracted and removed from the patient.

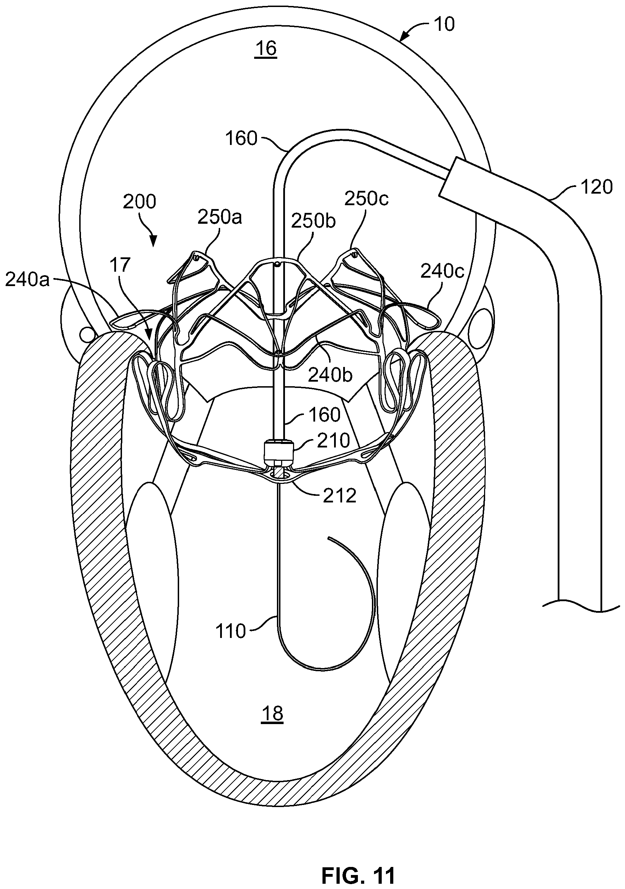

Referring also to **FIG. 11****,** with the anchor assembly 200 deployed within the mitral valve 17 (as described above), the anchor delivery catheter 140 can be withdrawn, the secondary steerable catheter 150 can be withdrawn, and the anchor delivery sheath 130 can also be withdrawn. In fact, if so desired, the anchor delivery catheter 140, the secondary steerable catheter 150, and the anchor delivery sheath 130 can be completely withdrawn from the guide catheter 120. In contrast, in some implementations the inner catheter 160 is advantageously left attached to the hub 210 of the anchor assembly 200 (and left attached to the SAM containment member 212 in some implementations). As will be described further below, in some implementations the inner catheter 160 can be used as a "rail" on which a valve assembly is later deployed into the interior of the anchor assembly 200. However, in some implementations the anchor assembly 200 is completely detached from the delivery system 100, and the delivery system 100 is removed from the patient. After a period of minutes, hours, days, weeks, or months, subsequent to the deployment of the anchor assembly 200, a valve assembly can be installed into the anchor assembly 200 to complete the installation of the prosthetic mitral valve.

In some implementations, withdrawal of the anchor delivery catheter 140, the secondary steerable catheter 150, and the anchor delivery sheath 130 can be performed as follows. First, the anchor delivery catheter 140 can be withdrawn into the anchor delivery sheath 130. Then, the secondary steerable catheter 150 can be withdrawn into the anchor delivery sheath 130 while generally simultaneously undeflecting (relaxing) the bend(s) in the secondary steerable catheter 150. Thereafter, in some embodiments the anchor delivery catheter 140, the secondary steerable catheter 150, and the anchor delivery sheath 130 can be simultaneously withdrawn further, including up to completely from the guide catheter 120. As described further below, such individual and/or simultaneous movements of components of the delivery system 100 can be initiated and controlled using a deployment frame system (such as the example deployment frame system of FIG. 49 described below) in some implementations.

In the depicted implementation, the SAM containment member 212 is still restrained in its pre-deployed configuration. As described further below, in some embodiments the depicted embodiment of the SAM containment member 212 is deployed after the installation of a valve assembly into the anchor assembly 200. Alternatively, as described further below, in some embodiments of the SAM containment member 212, the SAM containment member 212 is deployed prior to the installation of a valve assembly into the anchor assembly 200.

Referring to **FIG. 12****,** the anatomy of the native mitral valve 17 includes some consistent and predictable structural features across patients that can be utilized for engaging the anchor assembly 200 therewith. For example, the native mitral valve 17 includes the aforementioned sub-annular gutter 19. In addition, the native mitral valve 17 includes a D-shaped annulus 28, an anterolateral commissure 30a, a posteromedial commissure 30b, a left fibrous trigone 134a, and a right fibrous trigone 134b. Further, the native mitral valve 17 includes an anterior leaflet 20 and a three-part posterior leaflet 22. The posterior leaflet 22 includes a lateral scallop 24a, a middle scallop 24b, and a medial scallop 24c. The free edges of the posterior leaflet 22 and the anterior leaflet 20 meet along a coaptation line 32.

The D-shaped annulus 28 defines the structure from which the anterior leaflet 20 and posterior leaflet 22 extend and articulate. The left and right fibrous trigones 134a and 134b are located near the left and right ends of the anterior leaflet 20 and generally adjacent the lateral and medial scallops 24a and 24c of the posterior leaflet 22. The sub-annular gutter 19 runs along the annulus 28 between the left and right fibrous trigones 134a and 134b along the posterior leaflet 22.

The regions at or near the high collagen annular trigones 134a and 134b can generally be relied upon to provide strong, stable anchoring locations. The muscle tissue in the regions at or near the trigones 134a and 134b also provides a good tissue ingrowth substrate for added stability and migration resistance of the anchor assembly 200. Therefore, the regions at or near the trigones 134a and 134b define a left anterior anchor zone 34a and a right anterior anchor zone 34d respectively. The left anterior anchor zone 34a and the right anterior anchor zone 34d provide advantageous target locations for placement of the lateral anterior foot 220a and the medial anterior foot 220d respectively.

Referring also to **FIG. 13****,** a schematic representation of the anchor assembly 200 is shown in combination with the native mitral valve 17 of FIG. 12. The depicted portions of the anchor assembly 200 include the hub 210, the lateral anterior anchor foot 220a, the lateral posterior anchor foot 220b, the medial posterior anchor foot 220c, the medial anterior anchor foot 220d, the lateral anterior sub-annular support arm 230a, the lateral posterior sub-annular support arm 230b, the medial posterior sub-annular support arm 230c, and the medial anterior sub-annular support arm 230d. Each of those portions of the anchor assembly 200 reside below the mitral valve 17 when deployed, hence those portions of the anchor assembly 200 are drawn with dashed lines.

In the depicted embodiment, the lateral anterior sub-annular support arm 230a extends from the hub 210. The lateral anterior anchor foot 220a is disposed on an outer end of the lateral anterior sub-annular support arm 230a. Similarly, the medial anterior sub-annular support arm 230d extends from the hub 210, and the medial anterior anchor foot 220d is disposed on an outer end of the medial anterior sub-annular support arm 230d. The lateral posterior sub-annular support arm 230b extends from a middle portion of the lateral anterior sub-annular support arm 230a. The lateral posterior anchor foot 220b is disposed on an outer end of the lateral posterior sub-annular support arm 230b. The medial posterior sub-annular support arm 230c extends from a middle portion of the medial anterior sub-annular support arm 230d. The medial posterior anchor foot 220c is disposed on an outer end of the medial posterior sub-annular support arm 230c.

The depicted arrangement of the sub-annular support arms 230a, 230b, 230c, and 230d is advantageous because the arrangement is designed to reduce or minimize the potential for interference (by the anchor assembly 200) with the natural functioning of the chordae tendineae of the mitral valve 17. For example, the lateral posterior sub-annular support arm 230b and the medial posterior sub-annular support arm 230c are aligned generally parallel with the chordae tendineae in the areas where the posterior sub-annular support arms 230b and 230c are disposed.

Moreover, other sub-annular portions of the anchor assembly are also positioned in advantageous locations for interfacing with the native mitral valve 17. For example, the hub 210 is advantageously positioned generally directly below the coaptation line 32. In addition, the lateral anterior anchor foot 220a can be positioned in the left anterior anchor zone 34a and the medial anterior anchor foot 220d can be positioned in the right anterior anchor zone 34d. Further, the lateral posterior anchor foot 220b and the medial posterior anchor foot 220c can be positioned in posterior areas of the sub-annular gutter 19, namely a lateral posterior anchor zone 34b and a medial posterior anchor zone 34c, respectively, in order to provide balanced and atraumatic coupling of the anchor assembly 200 to the native mitral valve 17. In some implementations, the locations of the lateral posterior anchor zone 34b and the medial posterior anchor zone 34c may vary from the depicted locations while still remaining within the sub-annular gutter 19. It should be understood that the depicted anchor assembly 200 is merely one non-limiting example of the anchor assemblies provided within the scope of this disclosure.

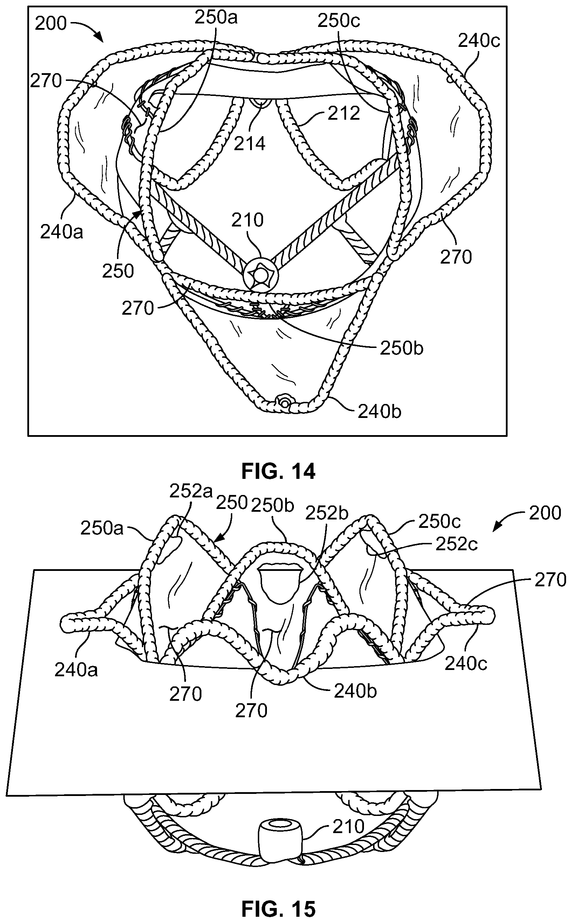

With reference to **FIGS. 14 and 15****,** the example anchor assembly 200 is shown in a sheet material that represents the annular plane of a native mitral valve, to more clearly show which structures are supra-annular vs. sub-annular. A covering-material 270 is included on the framework of the anchor assembly 200. The supra-annular structures of the example anchor assembly 200 are shown.

In the depicted embodiment, the supra-annular structures of the anchor assembly 200 include: the lateral anterior atrial holding feature 240a, the posterior atrial holding feature 240b, and the medial anterior atrial holding feature 240c; the lateral anterior anchor arch 250a, the posterior anchor arch 250b, and the medial anterior anchor arch 250c. The lateral anterior anchor arch 250a, the posterior anchor arch 250b, and the medial anterior anchor arch 250c are joined with each other to form an undulating supra-annular ring 250 that acts as a supra-annular structural element for the anchor assembly 200. As will be described further below, the supra-annular ring 250 also defines an opening to a space within the interior of the anchor assembly 200 that is configured to receive and engage with a valve assembly. The atrial holding features 240a, 240b, and 240c are configured to contact the shelf-like supra-annular tissue surface above the mitral valve annulus, and to thereby stabilize the anchor assembly 200 in supra-annular areas and to provide migration resistance in the direction towards the left ventricle.

In some embodiments, the anchor assembly 200 includes a covering material 270 disposed on one or more portions of the anchor assembly 200. The covering material 270 can provide various benefits. For example, in some implementations the covering material 270 can facilitate tissue ingrowth and/or endothelialization, thereby enhancing the migration resistance of the anchor assembly 200 and preventing thrombus formation on blood contact elements. In another example, as described further below, the covering material 270 can be used to facilitate coupling between the anchor assembly 200 and a valve assembly that is received therein. The cover material 270 also prevents or minimizes abrasion and/or fretting between the anchor assembly 200 and valve assembly 300. The cover material 270 also prevents valve outer tissue abrasion related wear, and supports to the cuff material to enhance durability. The covering material 270 may also provide redundant sealing in addition to the cuff material of the valve assembly.

In the depicted embodiment, the covering material 270 is disposed essentially on the entire anchor assembly 200, including the SAM containment member 212 (except for the eyelet 214, although in some embodiments the eyelet 214 may be essentially covered by the covering material 270). In some embodiments, the covering material 270 is disposed on one or more portions of the anchor assembly 200, while one or more other portions of the anchor assembly 200 do not have the covering material 270 disposed thereon. While the depicted embodiment includes the covering material 270, the covering material 270 is not required in all embodiments. In some embodiments, two or more portions of covering material 270, which can be separated and/or distinct from each other, can be disposed on the anchor assembly 200. That is, in some embodiments a particular type of covering material 270 is disposed on some areas of the anchor assembly 200 and a different type of covering material 270 is disposed on other areas of the anchor assembly 200.

In some embodiments, the covering material 270, or portions thereof, comprises a fluoropolymer, such as an expanded polytetrafluoroethylene (ePTFE) polymer. In some embodiments, the covering material 270, or portions thereof, comprises a polyester, a silicone, a urethane, ELAST-EON^{™} (a silicone and urethane polymer), another biocompatible polymer, DACRON^{®}, polyethylene terephthalate (PET), copolymers, or combinations and subcombinations thereof. In some embodiments, the covering material 270 is manufactured using techniques such as, but not limited to, extrusion, expansion, heat-treating, sintering, knitting, braiding, weaving, chemically treating, and the like. In some embodiments, the covering material 270, or portions thereof, comprises a biological tissue. For example, in some embodiments the covering material 270 can include natural tissues such as, but not limited to, bovine, porcine, ovine, or equine pericardium. In some such embodiments, the tissues are chemically treated using glutaraldehyde, formaldehyde, or triglycidylamine (TGA) solutions, or other suitable tissue crosslinking agents.

In the depicted embodiment, the covering material 270 is disposed on the interior and the exterior of the anchor assembly 200. In some embodiments, the covering material 270 is disposed on the just the exterior of the anchor assembly 200. In some embodiments, the covering material 270 is disposed on the just the interior of the anchor assembly 200. In some embodiments, some portions of the anchor assembly 200 are covered by the covering material 270 in a different manner than other portions of the anchor assembly 200.

In some embodiments, the covering material 270 is attached to at least some portions of the anchor assembly 200 using an adhesive. In some embodiments, epoxy is used as an adhesive to attach the covering material 270 to the anchor assembly 200, or portions thereof. In some embodiments, wrapping, stitching, lashing, banding, and/or clips, and the like can be used to attach the covering material 270 to the anchor assembly 200. In some embodiments, a combination of techniques are used to attach the covering material 270 to the anchor assembly 200.

In some embodiments, the covering material 270, or portions thereof, has a microporous structure that provides a tissue ingrowth scaffold for durable sealing and/or supplemental anchoring strength of the anchor assembly 200. In some embodiments, the covering material 270 is made of a membranous material that inhibits or reduces the passage of blood through the covering material 270. In some embodiments, the covering material 270, or portions thereof, has a material composition and/or configuration that inhibits or prevents tissue ingrowth and/or endothelialization to the covering material 270.

In some embodiments, the covering material 270 can be modified by one or more chemical or physical processes that enhance certain physical properties of the covering material 270. For example, a hydrophilic coating may be applied to the covering material 270 to improve the wettability and echo translucency of the covering material 270. In some embodiments, the covering material 270 may be modified with chemical moieties that promote or inhibit one or more of endothelial cell attachment, endothelial cell migration, endothelial cell proliferation, and resistance to thrombosis. In some embodiments, the covering material 270 may be modified with covalently attached heparin or impregnated with one or more drug substances that are released *in situ.*

In some embodiments, covering material 270 is pre-perforated to modulate fluid flow through the covering material 270 and/or to affect the propensity for tissue ingrowth to the covering material 270. In some embodiments, the covering material 270 is treated to make the covering material 270 stiffer or to add surface texture. In some embodiments, selected portions of the covering material 270 are so treated, while other portions of the covering material 270 are not so treated. Other covering material 270 material treatment techniques can also be employed to provide beneficial mechanical properties and tissue response interactions. In some embodiments, portions of the covering material 270 have one or more radiopaque markers attached thereto to enhance *in vivo* radiographic visualization.

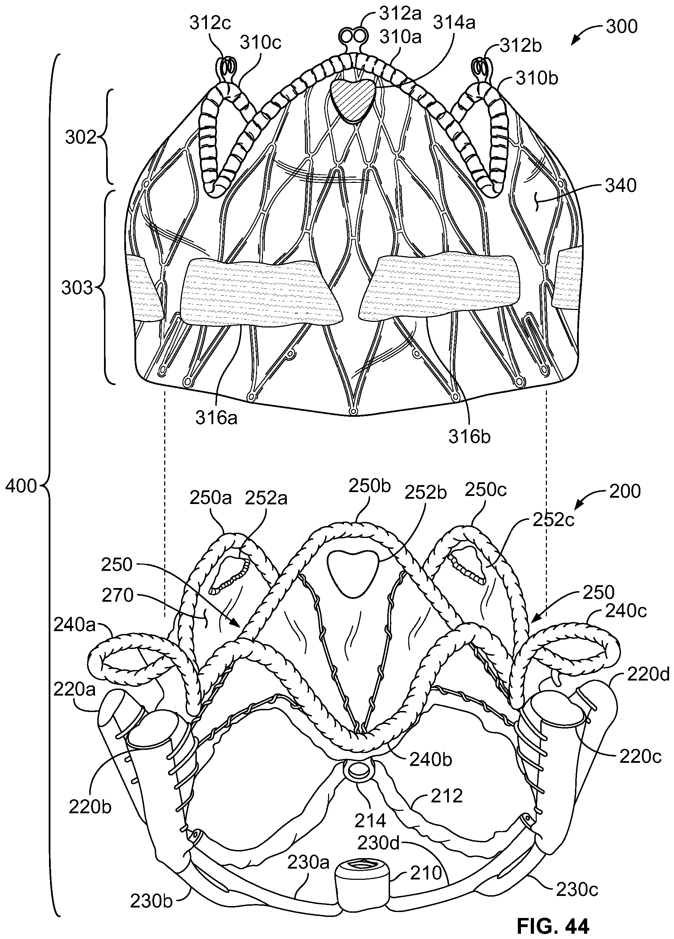

In some embodiments, the anchor assembly 200 can include features that are designed for coupling with a valve assembly that is received by the anchor assembly 200. For example, the lateral anterior anchor arch 250a, the posterior anchor arch 250b, and the medial anterior anchor arch 250c can be shaped and arranged for coupling with a valve assembly (as described further below). In addition, in some embodiments the anchor arches 250a, 250b, and 250c can include one or more covering-material cut-outs 252a, 252b, and 252c respectively. In some embodiments, the valve assembly (as described further below in reference to FIG. 44) can include features that become physically disposed within the covering-material cut-outs 252a, 252b, and 252c when the valve assembly is coupled with the anchor assembly 200. Such an arrangement can serve to provide a robust coupling arrangement between the valve assembly and the anchor assembly 200.

With reference to **FIGS. 16 and 17****,** the example anchor assembly 200 is shown in a sheet material that represents the annular plane of a native mitral valve. The sub-annular portions of the example anchor assembly 200 are shown.

In the depicted embodiment, the sub-annular portions of the anchor assembly 200 include the hub 210, the SAM containment member 212, the lateral anterior anchor foot 220a, the lateral posterior anchor foot 220b, the medial posterior anchor foot 220c, the medial anterior anchor foot 220d, the lateral anterior sub-annular support arm 230a, the lateral posterior sub-annular support arm 230b, the medial posterior sub-annular support arm 230c, and the medial anterior sub-annular support arm 230d. Each of those portions of the anchor assembly 200 reside below the native mitral valve annulus when deployed the anchor assembly 200 is deployed in a native mitral valve.

In the depicted embodiment, the lateral anterior sub-annular support arm 230a extends from the hub 210. The lateral anterior anchor foot 220a is disposed on an outer end of the lateral anterior sub-annular support arm 230a. Similarly, the medial anterior sub-annular support arm 230d extends from the hub 210, and the medial anterior anchor foot 220d is disposed on an outer end of the medial anterior sub-annular support arm 230d. The lateral posterior sub-annular support arm 230b extends from a middle portion of the lateral anterior sub-annular support arm 230a. The lateral posterior anchor foot 220b is disposed on an outer end of the lateral posterior sub-annular support arm 230b. The medial posterior sub-annular support arm 230c extends from a middle portion of the medial anterior sub-annular support arm 230d. The medial posterior anchor foot 220c is disposed on an outer end of the medial posterior sub-annular support arm 230c. A first end of the SAM containment member 212 extends from the lateral anterior sub-annular support arm 230a, and a second end of the SAM containment member 212 extends from the medial anterior sub-annular support arm 230d.

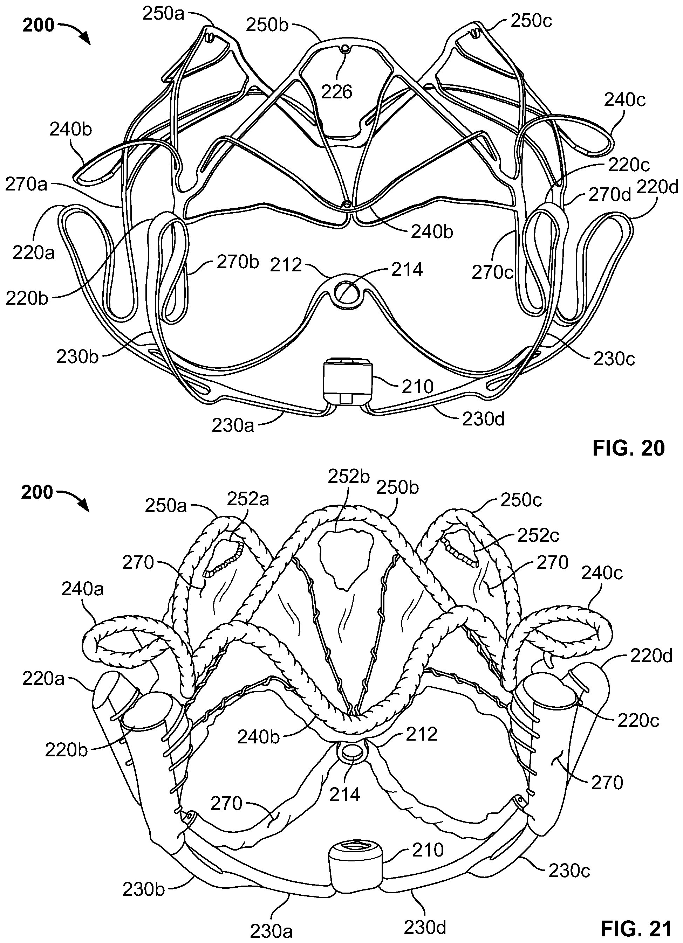

Referring to **FIGS. 18-21****,** the frame of an example anchor assembly 200 is shown in its fully expanded configuration. The anchor assembly 200 is shown without a covering-material so that the elongate member framework of the example anchor assembly 200 is clearly visible in FIGS. 18-20, and with covering-material in FIG. 21.

In some embodiments, the elongate members of the anchor assembly 200 are formed from a single piece of precursor material (e.g., sheet or tube) that is cut, expanded, and connected to the hub 210. For example, some embodiments are fabricated from a tube that is laser-cut (or machined, chemically etched, water-jet cut, etc.) and then expanded and shape-set into its final expanded size and shape. In some embodiments, the anchor assembly 200 is created compositely from multiple elongate members (e.g., wires or cut members) that are joined together with the hub 210 and each other to form the anchor assembly 200.

The elongate members of the anchor assembly 200 can be comprised of various materials and combinations of materials. In some embodiments, nitinol (NiTi) is used as the material of the elongate members of the anchor assembly 200, but other materials such as stainless steel, L605 steel, polymers, MP35N steel, stainless steels, titanium, cobalt/chromium alloy, polymeric materials, Pyhnox, Elgiloy, or any other appropriate biocompatible material, and combinations thereof can be used. The super-elastic properties of NiTi make it a particularly good candidate material for the elongate members of the anchor assembly 200 because, for example, NiTi can be heat-set into a desired shape. That is, NiTi can be heat-set so that the anchor assembly 200 tends to self-expand into a desired shape when the anchor assembly 200 is unconstrained, such as when the anchor assembly 200 is deployed out from the anchor delivery sheath 130. A anchor assembly 200 made of NiTi, for example, may have a spring nature that allows the anchor assembly 200 to be elastically collapsed or "crushed" to a low-profile delivery configuration and then to reconfigure to the expanded configuration as shown in FIGS. 18-20. The anchor assembly 200 may be generally conformable, fatigue resistant, and elastic such that the anchor assembly 200 can conform to the topography of the surrounding tissue when the anchor assembly 200 is deployed in a native mitral valve of a patient.

In some embodiments, the diameter or width/thickness of one or more of the elongate members forming the anchor assembly 200 may be within a range of about 0.008" to about 0.015" (about 0.20 mm to about 0.40 mm), or about 0.009" to about 0.030" (about 0.23 mm to about 0.76 mm), or about 0.01" to about 0.06" (about 0.25 mm to about 1.52 mm), or about 0.02" to about 0.10" (about 0.51 mm to about 2.54 mm), or about 0.06" to about 0.20" (about 1.52 mm to about 5.08 mm). In some embodiments, the elongate members forming the anchor assembly 200 may have smaller or larger diameters or widths/thicknesses. In some embodiments, each of the elongate members forming the anchor assembly 200 has essentially the same diameter or width/thickness. In some embodiments, one or more of the elongate members forming the anchor assembly 200 has a different diameter or width/thickness than one or more of the other elongate members of the anchor assembly 200. In some embodiments, one or more portions of one or more of the elongate members forming the anchor assembly 200 may be tapered, widened, narrowed, curved, radiused, wavy, spiraled, angled, and/or otherwise non-linear and/or not consistent along the entire length of the elongate members of the anchor assembly 200. Such features and techniques can also be incorporated with the valve assemblies of the prosthetic mitral valves provided herein.

In some embodiments, the elongate members forming the anchor assembly 200 may vary in diameter, thickness and/or width so as to facilitate variations in the forces that are exerted by the anchor assembly 200 in specific regions thereof, to increase or decrease the flexibility of the anchor assembly 200 in certain regions, to enhance migration resistance, and/or to control the process of compression (crushability) in preparation for deployment and the process of expansion during deployment of the anchor assembly 200.

In some embodiments, one or more of the elongate members of the elongate members forming the anchor assembly 200 may have a circular cross-section. In some embodiments, one or more of the elongate members forming the anchor assembly 200 may have a rectangular cross-sectional shape, or another cross-sectional shape that is not rectangular. Examples of cross-sectional shapes that the elongate members forming the anchor assembly 200 may have include circular, C-shaped, square, ovular, rectangular, elliptical, triangular, D-shaped, trapezoidal, including irregular cross-sectional shapes formed by a braided or stranded construct, and the like. In some embodiments, one or more of the elongate members forming the anchor assembly 200 may be essentially flat (i.e., such that the width to thickness ratio is about 2:1, about 3:1, about 4:1, about 5:1, or greater than about 5:1). In some examples, one or more of the elongate members forming the anchor assembly 200 may be formed using a center-less grind technique, such that the diameter of the elongate members varies along the length of the elongate members.

The anchor assembly 200 may include features that are directed to enhancing one or more desirable functional performance characteristics of the prosthetic mitral valve devices. For example, some features of the anchor assembly 200 may be directed to enhancing the conformability of the prosthetic mitral valve devices. Such features may facilitate improved performance of the prosthetic mitral valve devices by allowing the devices to conform to irregular tissue topographies and/or dynamically variable tissue topographies, for example. Such conformability characteristics can be advantageous for providing effective and durable performance of the prosthetic mitral valve devices. In some embodiments of the anchor assembly 200, some portions of the anchor assembly 200 are designed to be more conformable than other portions of the same anchor assembly 200. That is, the conformability of a single anchor assembly 200 can be designed to be different at various areas of the anchor assembly 200.

In some embodiments, the anchor assembly 200 includes features for enhanced *in vivo* radiographic visibility. In some embodiments, portions of the anchor assembly 200, such as one or more of the anchor feet 220a, 220b, 220c, and 220d, and/or SAM containment member 212, may have one or more radiopaque markers attached thereto. In some embodiments, some or all portions of the anchor assembly 200 are coated (e.g., sputter coated) with a radiopaque coating.

The anchor assembly 200 can also include one or more eyelets 226 in frame portions adjacent the arches. The eyelets 226 can be used for various purposes such as, but not limited to, holding radiopaque marker material, attachment points for suture loops or other elements which are additional control points for delivery and retrieval of the assembly, locations to secure a positional delivery frame, and the like.

In some embodiments, such as the depicted embodiment, the supra-annular structures and sub-annular structures of the anchor assembly 200 are interconnected by a lateral anterior inter-annular connection 270a, a lateral posterior inter-annular connection 270b, a medial posterior inter-annular connection 270c, and a medial anterior inter-annular connection 270d. For example, the lateral anterior inter-annular connection 270a connects the lateral anterior anchor foot 220a with the lateral anterior anchor arch 250a. Similarly, the medial anterior inter-annular connection 270d connects the medial anterior anchor foot 220d with the medial anterior anchor arch 250c. In addition, the lateral posterior inter-annular connection 270b connects the lateral posterior anchor foot 220b with the lateral anterior anchor arch 250a and the posterior anchor arch 250b, and the medial posterior inter-annular connection 270c connects the medial posterior anchor foot 220c with the posterior anchor arch 250b and the medial anterior anchor arch 250c.

In the depicted embodiment, the SAM containment member 212 extends anteriorly from the sub-annular support arms of the anchor assembly 200. For example, the SAM containment member 212, as depicted, comprises an elongate member with a first end that extends from the lateral anterior sub-annular support arm 230a and a second end that extends from the medial anterior sub-annular support arm 230d. In some embodiments, portions of the SAM containment member 212 may extend from other areas on the anchor assembly 200. While one particular embodiment of the SAM containment member 212 is depicted, it should be understood that multiple SAM containment member embodiments are envisioned and within the scope of this disclosure.

In the depicted embodiment, the SAM containment member 212 is integrally formed as part of the anchor assembly 200. In specific embodiments, the SAM containment member 212, or portions thereof, may be formed separately from the anchor assembly 200 and thereafter attached to the anchor assembly 200.

The SAM containment member 212, as shown, is in a deployed configuration. In some embodiments, the SAM containment member 212 is biased to self-reconfigure to the deployed configuration when the SAM containment member 212 is unconstrained. When the anchor assembly 200 is implanted in a native mitral valve and the SAM containment member 212 is in the deployed configuration, the SAM containment member 212 is disposed behind the anterior leaflet of a native mitral valve to physically block the anterior leaflet from obstructing the LVOT. As used herein, "behind" an anterior leaflet refers to the aortic side of the native mitral valve leaflet when the leaflet is open. In some implementations, while the SAM containment member 212 is deployed, the elongate members of the SAM containment member 212 may engage with the anterior leaflet and/or chordae to reduce the likelihood of SAM. The engagement can be anywhere along the lengths of the elongate members of the SAM containment member 212. For example, in some implementations portions of the elongate members of the SAM containment member 212 can actually engage the lateral edge of the anterior leaflet and/or chordae to spread or widen the anterior leaflet at the lateral edges thereby restricting its movement and also reducing likelihood of SAM.

In some embodiments, a shape-setting process is used to instill a bias so that the SAM containment member 212 tends seek its deployed configuration. Alternatively or additionally, as described further below, in some embodiments the SAM containment member 212 may be deflected into the deployed configuration by the application of one or more forces during the deployment of the SAM containment member 212.

In some embodiments, the SAM containment member 212 includes an attachment element 214 (a threaded eyelet 214 in this embodiment). The eyelet 214 provides an attachment feature that can be used to control the configuration and deployment of the SAM containment member 212. In some embodiments, other types of attachment elements 214 (as alternatives to the eyelet214) can be included on the SAM containment member 212. For example, in some embodiments one or more protrusions, ball ends, recesses, clips, breakable elements, deflectable elements, bends, and the like, and combinations thereof, can be included on the SAM containment member 212 as an attachment element 214.

Still referring to FIGS. 18-21, as described above the anchor feet 220a, 220b, 220c, and 220d are sized and shaped to engage the sub-annular gutter 19 of the mitral valve 17 (FIG. 12). In some embodiments, the anterior feet 220a and 220d are spaced apart from each other by a distance in a range of about 30 mm to about 45 mm, or about 20 mm to about 35 mm, or about 40 mm to about 55 mm. In some embodiments, the posterior feet 220b and 220c are spaced apart from each other by a distance in a range of about 20 mm to about 30 mm, or about 10 mm to about 25 mm, or about 25 mm to about 40 mm.

In some embodiments, the anchor feet 220a, 220b, 220c, and 220d have a height ranging from about 8 mm to about 12 mm, or more than about 12 mm. In some embodiments, the anchor feet 220a, 220b, 220c, and 220d have a gutter engaging surface area (when fabric covered) ranging from about 6 mm² to about 24 mm². In some embodiments, the anchor feet 220a, 220b, 220c, and 220d each have essentially the same gutter engaging surface area. In particular embodiments, one or more of the anchor feet 220a, 220b, 220c, and 220d has a different gutter engaging surface area than one or more of the other anchor feet 220a, 220b, 220c, and 220d. The anchor feet 220a, 220b, 220c, and 220d can have widths ranging within about 1.5 mm to about 4.0 mm or more, and lengths ranging within about 3 mm to about 6 mm or more. The anchor feet 220a, 220b, 220c, and 220d are sized and shaped so that the anchor assembly 200 does not significantly impair the natural function of mitral valve chordae tendineae, the native mitral valve leaflets, and papillary muscles even after the anchor assembly is anchored at the mitral valve site.

As described previously, the anchor assembly 200 is designed to avoid interference with the functioning of the native mitral valve 17 (FIG. 12). Therefore, the anchor assembly 200 can be implanted within the native mitral valve 17 some time prior to the deployment therein of a replacement valve assembly, without degradation of valve 17 function during the period of time between the anchor implantation and the valve implantation (whether that time is on the order of minutes, or even several days or months). To avoid such interference between the anchor assembly 200 and the native mitral valve 17, the inter-annular connections 270a, 270b, 270c, and 270d pass through the coaptation line 32 approximately. More particularly, the lateral anterior inter-annular connection 270a passes through the coaptation line 32 adjacent to the anterolateral commissure 30a. In like manner, the medial anterior inter-annular connection 270d passes through the coaptation line 32 adjacent to the posteromedial commissure 30b. In some implementations, the lateral posterior inter-annular connection 270b and medial posterior inter-annular connection 270c pass through the native mitral valve 17 in locations that are posteriorly biased from the natural coaptation line 32. In such a case, the posterior leaflet 22 will tend to compliantly wrap around the lateral posterior inter-annular connection 270b and medial posterior inter-annular connection 270c to facilitate sealing of the mitral valve 17 with the anchor assembly 200 coupled thereto.

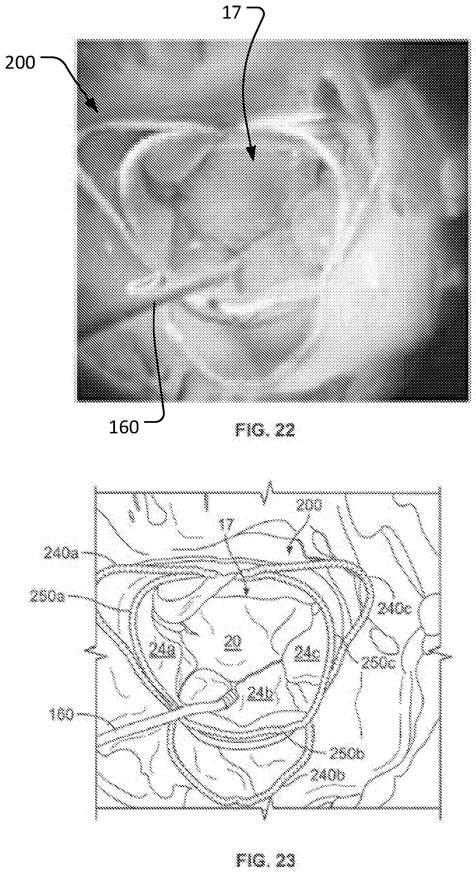

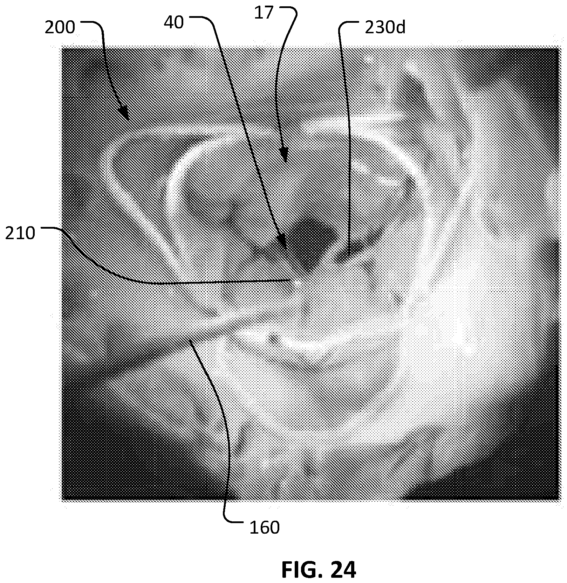

Referring to **FIGS. 22-24****,** the anchor assembly 200 is shown implanted within a native mitral valve 17. The inner catheter 160 is still coupled to the anchor assembly 200 in these figures. FIGS. 22 is a photographic image that corresponds to FIG. 23 which shows the mitral valve 17 in a closed state. FIG. 24 is a photographic image showing the anchor assembly 200 coupled with the native mitral valve 17 while the mitral valve 17 is in an open state. These illustrations are from the perspective of the left atrium looking inferior (downwardly) towards the mitral valve 17. For instance, in FIG. 24 some chordae tendineae 40 are visible through the open leaflets of the mitral valve 17.

These figures illustrate the supra-annular structures and sub-annular structures of the anchor assembly 200 in their relationships with the native mitral valve 17. For example, the closed state of the native mitral valve 17 in FIGS. 22 and 23 allows visibility of the supra-annular structures such as the lateral anterior atrial holding feature 240a, the posterior atrial holding feature 240b, and the medial anterior atrial holding feature 240c. In addition, the lateral anterior anchor arch 250a, the posterior anchor arch 250b, and the medial anterior anchor arch 250c are visible. However, the sub-annular structures are not visible in FIG. 13A because such structures are obstructed from view by the anterior leaflet 20 and the three-part posterior leaflet 24a, 24b, and 24c.

In contrast, in FIG. 24 certain sub-annular structures of the anchor assembly 200 are visible because the native mitral valve 17 is open. For example, the medial anterior sub-annular support arm 230d and hub 210 are in view through the open mitral valve 17. Other sub-annular portions of the anchor assembly 200, such as the anchor feet 220a, 220b, 220c, and 220d, remain out of view because of visual obstructions of the native mitral valve 17. In addition, no SAM containment member (which is a sub-annular structure) is visible in this view as it is in its pre-deployed configuration.

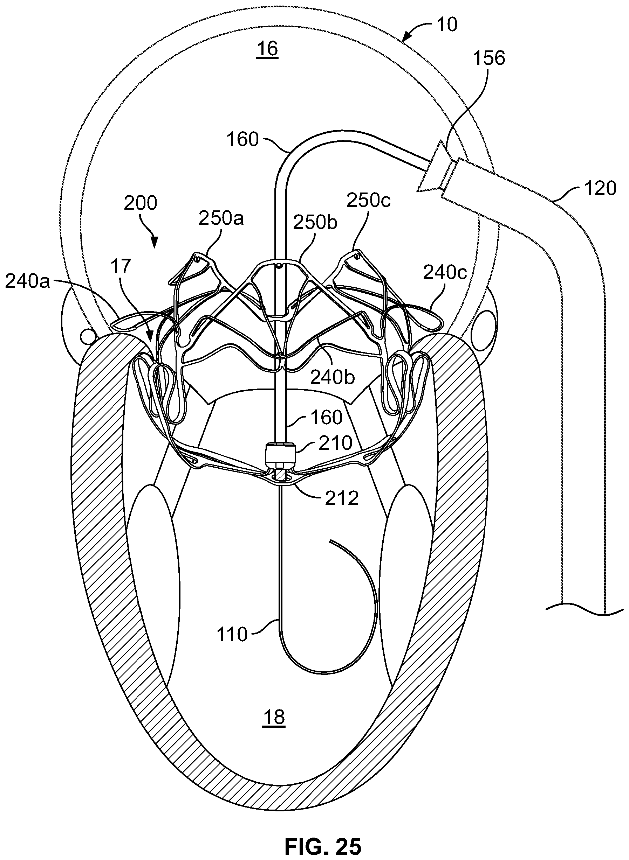

Referring to **FIG. 25****,** during or after an implantation procedure of the anchor assembly 200, in some cases the anchor assembly 200 may need to be removed, and therefore retrieved from the heart. For example, the anchor assembly 200 may be unable to be positioned correctly for various reasons such as, but not limited to, anatomical limitations, sub-optimal sizing of the anchor assembly 200, instability of the anchor assembly 200, and/or unsuccessful engagement of one of the four feet of the anchor assembly 200. In some embodiments, it may be desirable to retrieve the anchor assembly 200 in the event that a subsequently deployed valve assembly is not providing the desired hemodynamic results. In such a scenario, both the valve assembly and the anchor assembly may be removed, as will be described herein.

When it is decided to retrieve the anchor assembly 200 (either prior to deployment of a valve assembly, or after deployment and removal of a valve assembly), an anchor retrieval sheath 156 can be installed by passing it over the inner catheter 160 and through the guide catheter 120. As shown, in some cases a distal end portion of the anchor retrieval sheath 156 can be positioned distally of a distal end of the guide catheter 120 such that the anchor retrieval sheath 156 protrudes from the guide catheter 120 into the left atrium 16. Optionally, the anchor retrieval sheath 156 can include an expandable funnel or beveled shaped distal end portion.

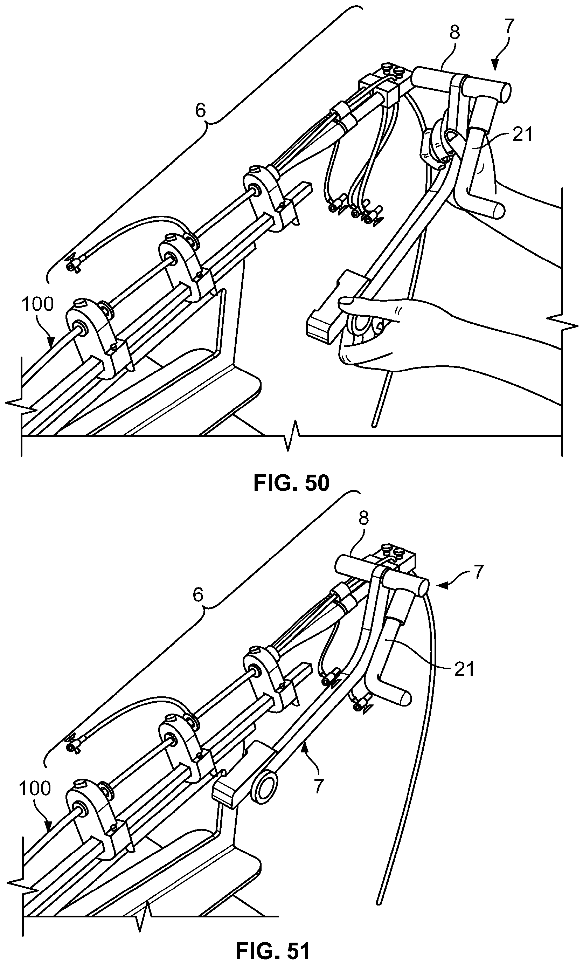

Then, to retrieve the anchor assembly 200, a proximally directed force can be applied to the inner catheter 160 to pull the anchor assembly 200 toward the anchor retrieval sheath 156. For example, in some cases a pulling force to retrieve the anchor assembly 200 can be applied to the inner catheter 160 using an anchor retrieval accessory (refer to FIGS. 50-52). In some scenarios, the anchor assembly 200 may tend to invert as the anchor assembly 200 is pulled into the atrium 16. In any event, the anchor assembly 200 will invert as the hub 210 is pulled into the anchor retrieval sheath 156 and as the other portions of the anchor assembly 200 enter into the anchor retrieval sheath 156 as a result of a continued proximal pulling on the inner catheter 160.

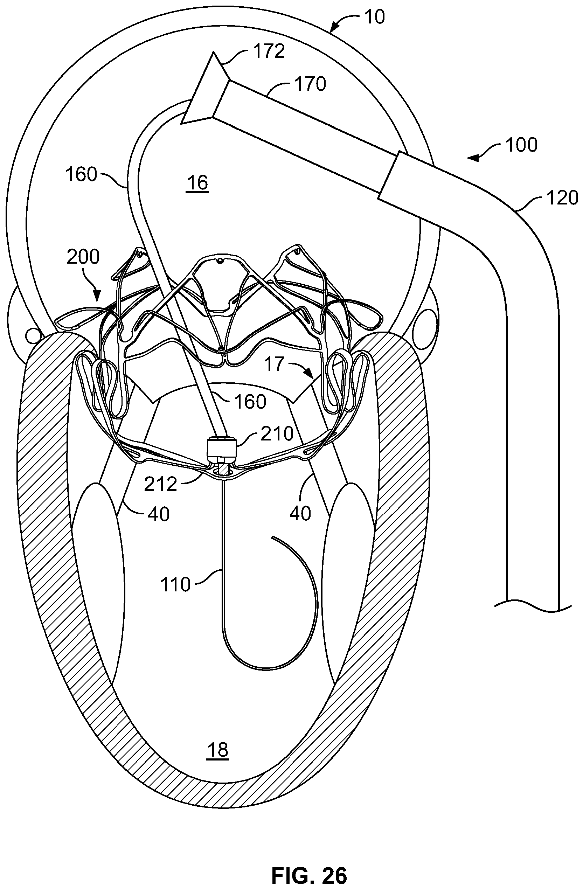

Referring to **FIG. 26****,** after implantation of the anchor assembly 200 within the native mitral valve 17 (as performed, for example, in accordance with FIGS. 1-5 and 7-11 described above), a valve delivery sheath 170 of the delivery system 100 can be used to deploy a valve assembly within the anchor assembly 200. As described above in reference to FIG. 11, with the inner catheter 160 coupled with the hub 210 of the anchor assembly 200, the inner catheter 160 can be used to guide the valve assembly into the interior of the anchor assembly 200.

In the depicted embodiment, the SAM containment member 212 is constrained in its pre-deployed configuration. However, in some other SAM containment member embodiments, the SAM containment member may be deployed prior to installation of a valve assembly within the anchor assembly 200. Generally speaking, depending on the SAM containment member embodiment's design, if the SAM containment member may potentially interfere with the function of the anterior leaflet, it may be preferable to wait until the valve is implanted to deploy the SAM containment member. But, if the SAM containment member does not or is unlikely to interfere with the leaflet function, the SAM containment member may be deployed prior to valve implant (which may be beneficial for situations where the anchor is implanted in a separate procedure from the valve implantation).

In some implementations, with the guide catheter 120 positioned with its distal end in the left atrium 16, the valve delivery sheath 170 is installed into a lumen of the guide catheter 120 (over the inner catheter 160) and advanced through the guide catheter 120. As described further below, in some embodiments the valve delivery sheath 170 is loaded at that time with a prosthetic valve assembly and other components of the delivery system 100. The guide catheter 120 may be the same catheter that was used to deliver the anchor assembly 200, or it may be a different catheter (but still referred to here as the guide catheter 120 for simplicity sake). Depending on the time interval between implantation of the anchor assembly 200 and the valve assembly 300, it may also be desirable to leave the same guide catheter 120 *in situ* during the time between the deliveries of each assembly.

In some embodiments, the valve delivery sheath 170 can be made from the materials described above in reference to the guide catheter 120. In some embodiments, the valve delivery sheath 170 has an outer diameter in the range of about 20 Fr to about 28 Fr (about 6.7 mm to about 9.3 mm). In some embodiments, the valve delivery sheath 170 has an outer diameter in the range of about 14 Fr to about 24 Fr (about 4.7 mm to about 8.0 mm).

In the depicted embodiment, the valve delivery sheath 170 includes a flared distal end portion 172. In some embodiments, no such flared distal end portion 172 is included. The flared distal end portion 172 can collapse to a lower profile when constrained within the guide catheter 120. When the flared distal end portion 172 is expressed from the guide catheter 120, the flared distal end portion 172 can self-expand to the flared shape. In some embodiments, the material of the flared distal end portion 172 includes pleats or folds, may be a continuous flared end or may be separated into sections such as flower pedals, and may include one or more resilient elements that bias the flared distal end portion 172 to assume the flared configuration in the absence of restraining forces (such as from containment within the guide catheter 120). The flared distal end portion 172 can be advantageous, for example, for recapturing the valve assembly (if desired) within the lumen of the valve delivery sheath 170 after the valve assembly has been expressed from the flared distal end portion 172.

In some embodiments, the maximum outer diameter of the flared distal end portion 172 is in a range of about 30 Fr to about 34 Fr (about 10.0 mm to about 11.3 mm). In some embodiments, the maximum outer diameter of the flared distal end portion 172 is in a range of about 32 Fr to about 44 Fr (about 10.7 mm to about 14.7 mm). In some embodiments, the maximum outer diameter of the flared distal end portion 172 is in a range of about 24 Fr to about 30 Fr (about 8.0 mm to about 10.0 mm). In some embodiments, the maximum outer diameter of the flared distal end portion 172 is less than about 24 Fr (about 8.0 mm) or greater than about 44 Fr (about 14.7 mm).

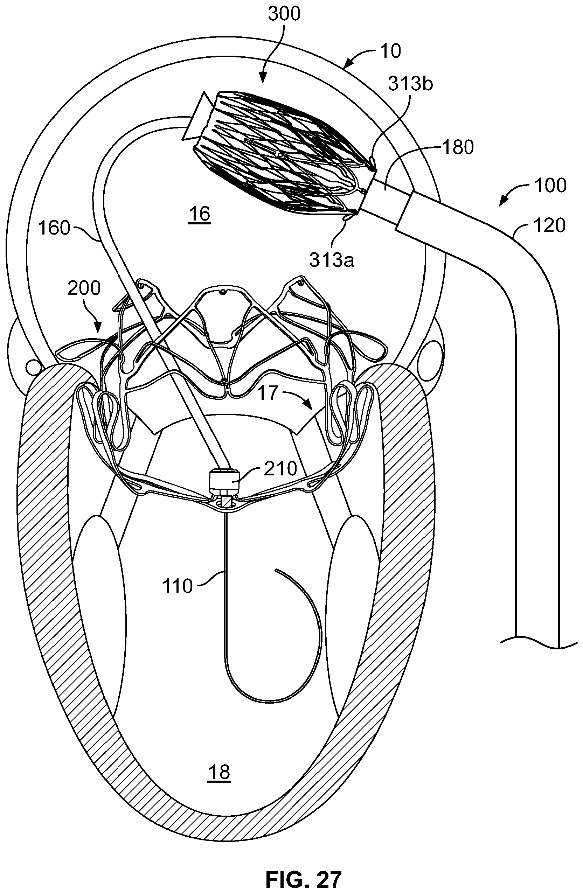

Referring also to **FIG. 27****,** in some implementations the valve delivery sheath 170 can be withdrawn into the guide catheter 120 while a valve delivery catheter 180 is held substantially stationary to thereby express a valve assembly 300 from a lumen of the valve delivery sheath 170. The valve delivery sheath 170 and the valve delivery catheter 180 are additional components in some embodiments of the example delivery system 100. It should be understood that movements of the components (e.g., the valve delivery sheath 170 and the valve delivery catheter 180) of the delivery system 100, whether the movements be those of individual components or two or more components in combination with each other, can in some embodiments be initiated and controlled using a deployment frame system (such as the example deployment frame system of FIG. 49 described below).

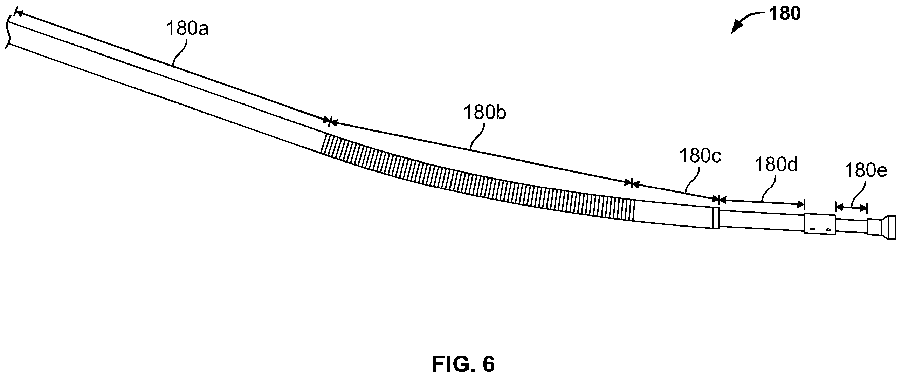

Referring also to **FIG. 6****,** in some embodiments the valve delivery catheter 180 can be advantageously configured with multiple zones that have differing mechanical properties such as flexibility, durometer, column strength, crush strength, elasticity, torqueability, trackability, and the like. For example, in the depicted embodiment the valve delivery catheter 180 includes a first zone 180a, a second zone 180b, a third zone 180c, a fourth zone 180d, and a fifth zone 180e. In one example, the first zone 180a has a durometer of about 72D, the second zone 180b has a durometer of about 35D, the third zone 180c has a durometer of about 25D, the fourth zone 180d has a durometer of about 55D, and the fifth zone 180e has a durometer of about 35D. The different zones may be constructed differently in relation to each other (e.g., using different polymers, coatings, coil reinforcements, braided reinforcements, hypotubes, etc.). Such variations in the mechanical properties (e.g., flexibility, etc.) of the valve delivery catheter 180 can be advantageous for the navigation of the valve delivery catheter 180 through the curvatures of a patient's vasculature. For example, in the depicted embodiment, the first zone 180a being 72D (for example) provides column strength for the section of the valve delivery catheter 180 that is expected to be in the inferior vena cava and/or right atrium. The zones 180b, 180c, 180d and 180e having example durometers of 35D, 25D, 55D and 35D respectively provide the flexibility for the valve delivery catheter 180 to navigate the curvature from right atrium to mitral annulus plane through fossa ovalis and left atrium. The zone 180d of 55D (for example) also provides the stiffness profile to align the axis of the valve delivery catheter 180 along the normal to the native mitral annulus plane. It should be understood that this is merely one example and other arrangements are also envisioned within the scope of this disclosure. Moreover, one or more other catheter devices of delivery system 100 can be configured with such multiple zones that have differing mechanical properties (as exemplified here in regard to valve delivery catheter 180).

Still referring to FIG. 27, the valve assembly 300 can be releasably coupled to the valve delivery catheter 180 and retained in a low-profile configuration. In some embodiments, both the distal and proximal ends of the valve assembly 300 are releasably coupled to the valve delivery catheter 180. In some embodiments, just one of the distal end or the proximal end of the valve assembly 300 is releasably coupled to the valve delivery catheter 180. In particular embodiments, one or more control wires (e.g., a proximal control wire 184a and distal control wire 184b, as described further below) may be included to releasably couple one or more portions of the valve assembly 300 to the valve delivery catheter 180. In some such embodiments, the one or more control wires may act as lassos to radially constrain the bias of the valve assembly 300 from radially self-expanding. Hence, a release of tension on the one or more control wires may allow at least a portion of the valve assembly 300 to radially self-expand.

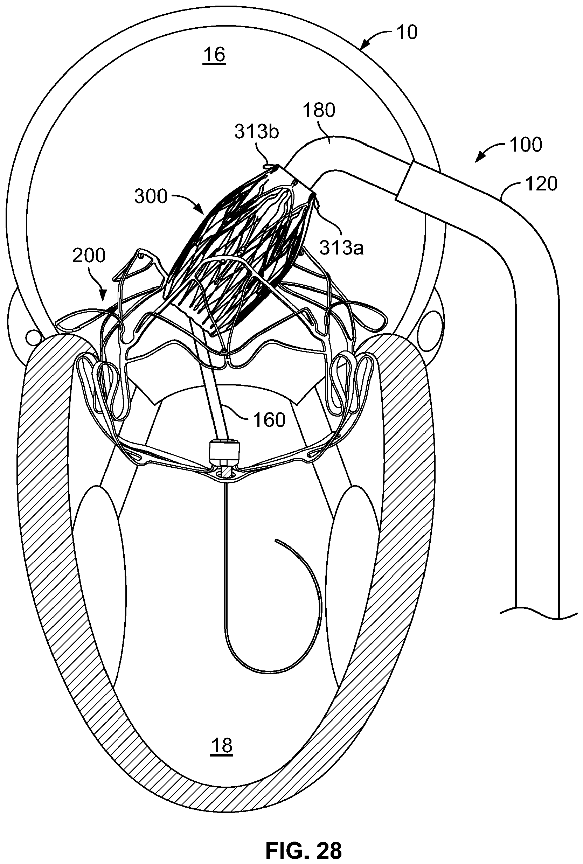

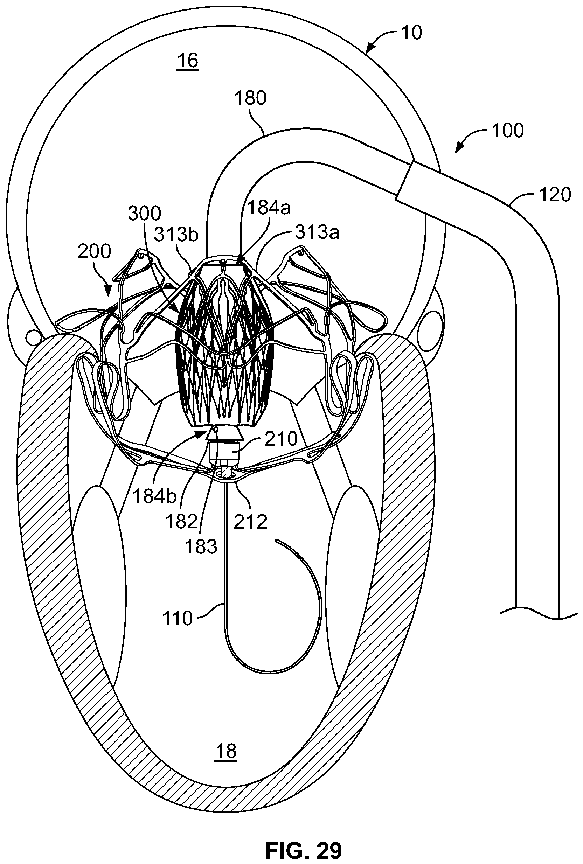

Referring to **FIGS. 28** **and** **29****,** the delivery system 100 can be manipulated by a clinician operator to perform a lateral pivot (panning, rotation, etc.) of the valve assembly 300 within the left atrium 16. The rotation of the valve assembly 300 changes the alignment of the valve assembly 300 from being generally axial with the distal end portion of the guide catheter 120 to being generally axial with the anchor assembly 200 (in preparation for installation of the valve assembly 300 into the interior of the anchor assembly 200).

In some implementations, the aforementioned rotation of the valve assembly 300 can be performed as follows. As shown in FIG. 27, because of the influence from the guide catheter 120 on the valve delivery catheter 180, the axis of the valve assembly 300 is initially in general alignment with the axis of the distal end portion of the guide catheter 120. From this arrangement, a generally simultaneous counter-movement of/between the inner catheter 160 and the valve delivery catheter 180 can be performed by the clinician to rotate the valve assembly 300. That is, as the inner catheter 160 is pulled proximally, the valve delivery catheter 180 is pushed distally. As a result of that counter movement, the valve assembly 300 rotates/pans in a relatively tight radius within the left atrium 16, as required by the confines of the left atrium 16. Thereafter, the valve delivery catheter 180 can be advanced further so that the valve assembly 300 is coaxially positioned within the interior of the anchor assembly 200 as shown in FIG. 29. As with other movements of the components of the delivery system 100 described herein (and other movements of the components of the delivery system 100 that are like those described herein), the generally simultaneous counter-movements of/between the inner catheter 160 and the valve delivery catheter 180 can be initiated and controlled using a deployment frame system (such as the example deployment frame system of FIG. 49 described below) in some implementations. At this stage of the valve deployment process, the valve assembly 300 is releasably coupled to the valve delivery catheter 180 by the proximal control wire 184a and the distal control wire 184b.

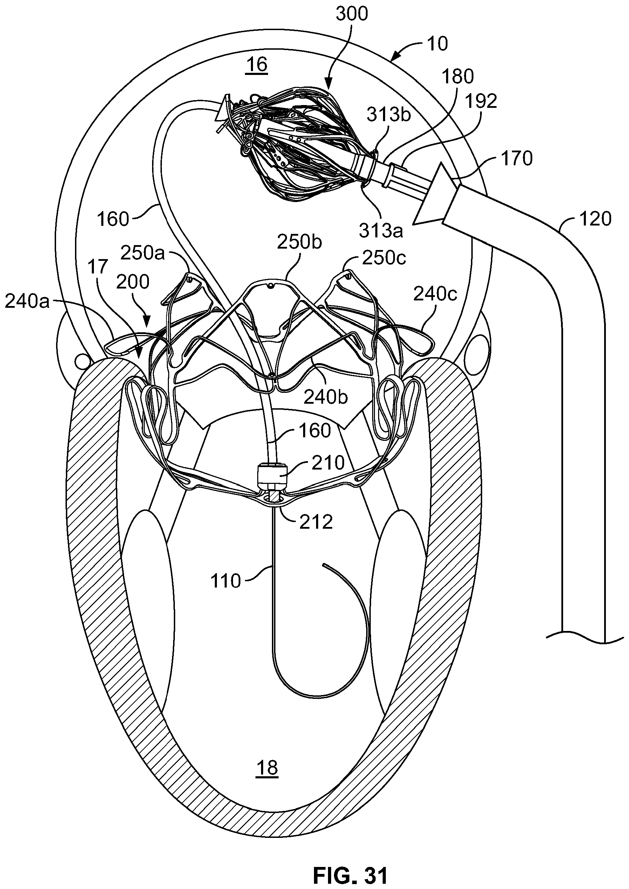

Referring generally to **FIGS. 31-34****,** in some cases during or after an implantation of the valve assembly 300 (and, for example, prior to uncoupling the control wires 184a-b from the valve assembly 300), removal of the valve assembly 300 from the heart 10 may be desired or necessary. That could be the case, for example, if the deployed valve assembly 300 is not providing the desired hemodynamic results. For instance, the valve assembly 300 may not be reducing valve regurgitation (e.g., mitral regurgitation) to the extent expected. Removal of the valve assembly 300 may be desired or necessary prior to release of the control wires 184a-b that couple the valve assembly 300 to the valve delivery catheter 180.

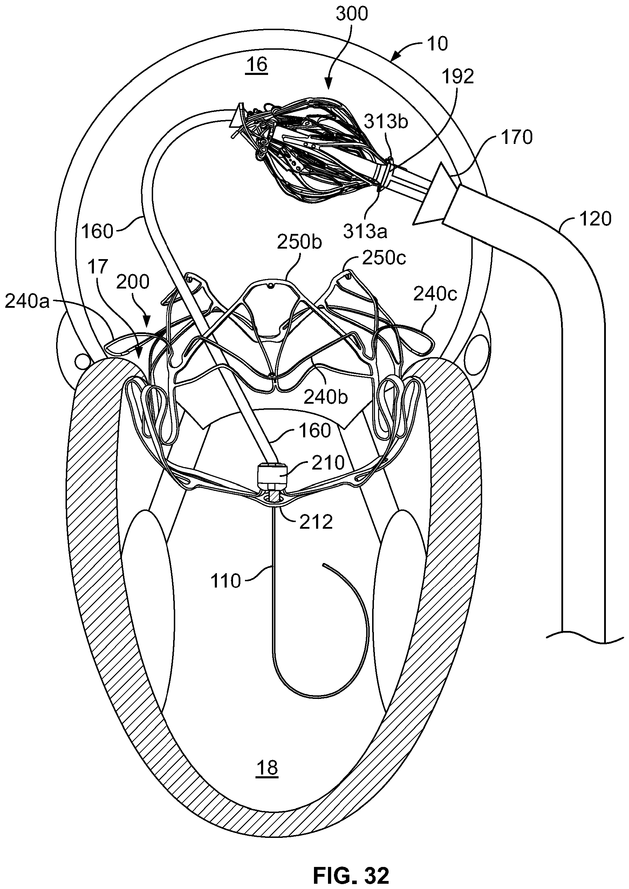

Referring to **FIGS. 31** **and** **32****,** some example techniques for valve assembly 300 retrieval include the use of a valve retrieval catheter 190 (not visible because of being positioned within the valve delivery sheath 170) that is positioned between the valve delivery catheter 180 and the valve delivery sheath 170. That is, in some embodiments the valve retrieval catheter 190 is passed over the valve delivery catheter 180 and within the valve delivery sheath 170. Optionally, the valve retrieval catheter 190 can increase the overall stiffness of the delivery system 100.

A valve engagement portion 192 at the distal end of the valve retrieval catheter 190 can be extended into the left atrium 16 beyond a distal end of the valve delivery sheath 170. In some embodiments, the valve engagement portion 192 is a framework structure that is thermally coupled to the shaft of the valve retrieval catheter 190. The valve engagement portion 192 can be constructed of one or more materials such as, but not limited to, stainless steel, nitinol, polymers, and the like, and combinations thereof.

In some embodiments, the valve delivery catheter 180 (while the valve delivery catheter 180 is still coupled to the valve assembly 300) can be retracted proximally (before, during or after the distal advancement of the valve retrieval catheter 190) to position the valve assembly 300 near to the valve engagement portion 192 of the valve retrieval catheter 190.

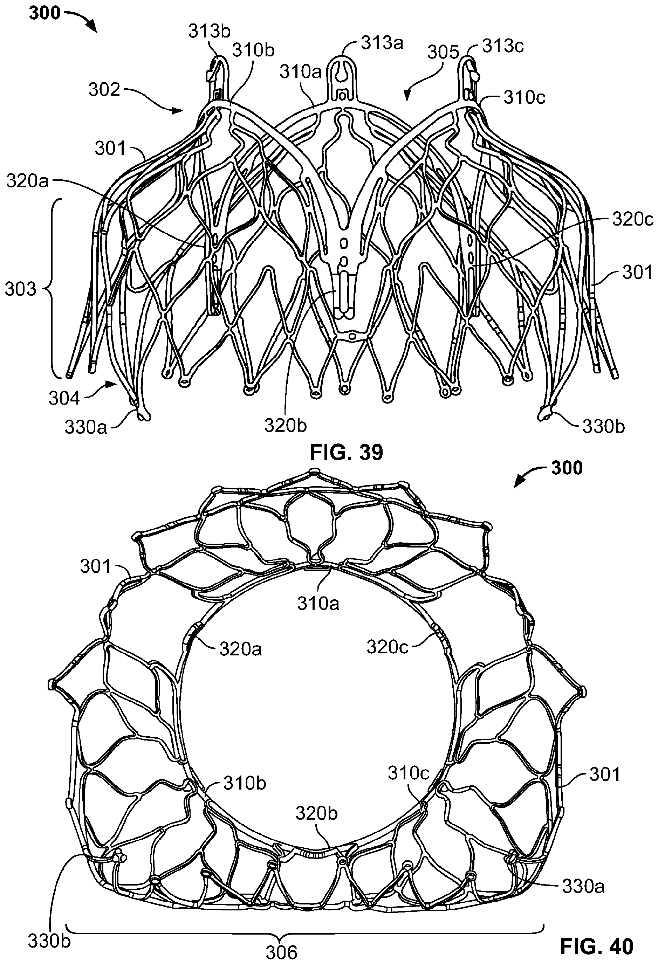

In some embodiments, the top of the arches 310a-c of the valve assembly 300 includes optional valve arch hooks 313a, 313b, and 313c (here the valve arch hook 313c is obscured from view-refer to FIG. 39 for a view of all three valve arch hooks 313a-c). The valve arch hooks 313a-c on the proximal end of the valve assembly 300 are configured to selectively mechanically engage with the valve engagement portion 192 to facilitate removal of the valve assembly 300. In some embodiments, the valve arch hooks 313a-c are biased to project radially outward away from the center of the valve assembly 300 at an acute angle like hook members. When the valve arch hooks 313a-c start to make contact with the valve engagement portion 192, the valve arch hooks 313a-c will be radially compressed to deflect inward. As the process of engagement between the valve assembly and the valve retrieval catheter 190 continues, the valve arch hooks 313a-c pass into interstitial spaces of the framework of the valve engagement portion 192 and then return to the original radially outward biased position when they pass into open space(s) defined by the valve engagement portion 192. In some cases, the valve arch hooks 313a-c effectively 'snap' into mechanical engagement with the valve engagement portion 192. In that fashion, the engagement between the valve arch hooks 313a-c and the valve engagement portion 192 results in the coupling together of the valve assembly 300 and the valve retrieval catheter 190.

Once the valve assembly 300 and the valve retrieval catheter 190 are coupled together, the valve assembly 300 can be pulled into the valve delivery sheath 170. Optionally, the valve arch hooks 313a-c, and/or the valve engagement portion 192 can include radio opaque markers so as to be visible with an imaging modality. Accordingly, the relative positions and/or coupling of the valve arch hooks 313a-c and the valve engagement portion 192 can be confirmed.

The coupling of the valve assembly 300 and the valve retrieval catheter 190 as described above can reduce stress (e.g., pulling, stretching, and/or breakage) on the valve assembly 300 as the valve assembly 300 is pulled into the valve delivery sheath 170. For example, since all three valve arch hooks 313a-c are engaged with the valve retrieval catheter 190, none of the arches will be inadvertently stuck outside the valve delivery sheath 170. Additionally, the coupling between the valve arch hooks 313a-c and the valve engagement portion 192 causes the valve assembly 300 be pulled into the valve delivery sheath 170 concentrically, thereby minimizing the force required.

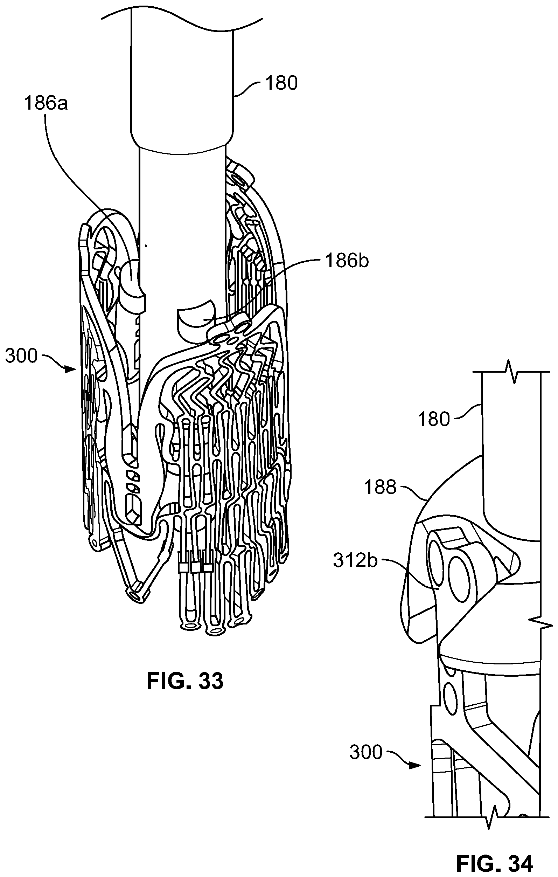

Referring to **FIG.** 33, in an example not forming part of the claimed invention that is designed to facilitate retrieval of the valve assembly 300, the valve delivery catheter 180 can include one or more radial protrusions 186a, 186b, and 186c (radial protrusion 186c is obscured from view in FIG. 33) located on a distal end portion of the valve delivery catheter 180. In some cases, the radial protrusions 186a-c can be located on an exterior of the valve delivery catheter 180 as shown. In some embodiments, the valve delivery sheath 170 can include radial protrusions on an interior of the valve delivery sheath 170 along a distal end portion of the valve delivery sheath 170. The radial protrusions 186a-c can be constructed of one or more materials such as, but not limited to, PEEK, Pebax, Nylon, other polymers, silicone, foam, metals, and the like, and combinations thereof.

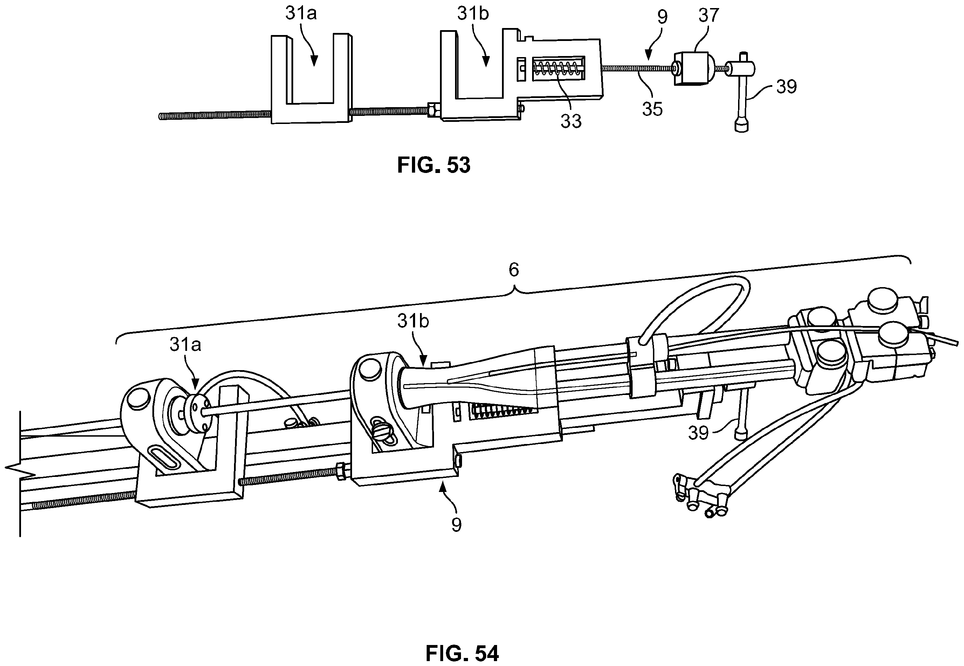

The radial protrusions 186a-c can aid in frictionally coupling the valve assembly 300 to the valve delivery catheter 180, or the valve delivery sheath 170, depending on the location of the radial protrusions 186a-c. The engagement and coupling between the valve assembly 300 and the valve delivery catheter 180, or the valve delivery sheath 170, depending on the location of the radial protrusions 186a-c, can in turn aid in retrieval of the valve assembly 300. Once the valve assembly 300 and the radial protrusions 186a-c are frictionally engaged, the valve assembly 300 can be pulled into the valve delivery sheath 170. In some cases, a valve retrieval accessory 9 (refer to FIGS. 53 and 54) can be used to assist in the process of puling the valve assembly 300 into the valve delivery sheath 170.

In some embodiments, the coupling of the valve assembly 300 and the radial protrusions 186a-c can reduce stress (e.g., pulling, stretching, and/or breakage) on the valve assembly 300 as the valve assembly 300 is pulled into the valve delivery sheath 170. For example, since the radial protrusions 186a-c can be engaged with the valve delivery catheter 180, the likelihood of arches getting stuck outside the valve delivery sheath 170 can be reduced. As another example, the engagement between the radial protrusions 186a-c and the valve assembly 300 can cause the valve assembly 300 to be pulled into the valve delivery sheath 170 more concentrically.

Referring to FIG. 34, in another example embodiment that is designed to facilitate retrieval of the valve assembly 300, the valve delivery catheter 180 can include a valve retainer 188 located on a distal end portion of the valve delivery catheter 180. In some cases, the valve retainer 188 can be thermally affixed to the valve delivery catheter 180.

In order to releasably couple with the valve retainer 188, the valve assembly 300 can include arch extensions 312a, 312b, and 312c (the arch extensions 312a and 312c are not visible here) that are configured to mechanically mate with the valve retainer 188. For example, the arch extensions 312a-c can be located at the apices of the arches 310a-c (refer to FIG. 37) of the valve assembly 300. The valve retainer 188 can include a plurality of recesses that can receive the arch extensions 312a-c (e.g., an equal number of recesses and arch extensions). For example, the recesses of the valve retainer 188 can have a narrow portion more distal that widens proximally, such that a shape of the recesses is complementary to a shape of the arch extensions 312a-c. The complementary configuration of the valve retainer 188 and the arch extensions 312a-c can facilitate the releasable coupling between the valve assembly 300 and the valve delivery catheter 180.

Accordingly, the arch extensions 312a-c, and therefore the valve assembly 300, can be coupled to the valve delivery catheter 180 via the valve retainer 188. Optionally, the valve assembly 300 (e.g., via the arch extensions 312a-c) can lock, and therefore unlock, onto the valve delivery catheter 180 (e.g., via the valve retainer 188). In some embodiments, the valve retainer 188 can aid in implantation of the valve assembly 300, retrieval of the valve assembly 300, or both.

During retrieval of the valve assembly 300, once the valve assembly 300 and the valve delivery catheter 180 are coupled together as depicted in FIG. 34, the valve assembly 300 can be pulled into the valve delivery sheath 170 for retrieval. Optionally, the arch extensions 312a-c, or the valve retainer 188 can be visible with an imaging modality, such that the position and/or coupling of the arch extensions 312a-c and/or the valve retainer 188 can be confirmed. In some embodiments, larger valve assemblies can require greater forces to pull the valve assembly 300 into the valve delivery sheath 170. In some cases, a valve retrieval accessory 9 (refer to FIGS. 53 and 54) can be used to assist in the process of puling the valve assembly 300 into the valve delivery sheath 170.

The coupling of the valve assembly 300 and the valve delivery catheter 180 can reduce stress (e.g., pulling, stretching, and/or breakage) on the valve assembly 300 as the valve assembly 300 is pulled into the valve delivery sheath 170. For example, since all three arch extensions 312a-c are engaged with the valve delivery catheter 180, the likelihood of portions of the valve assembly 300 being temporarily stuck outside the valve delivery sheath 170 can be reduced. As another example, the coupling between the arch extensions 312a-c and the valve retainer 188 can facilitate the valve assembly 300 to be pulled into the valve delivery sheath 170 concentrically.

In some embodiments, different configurations of the valve assembly 300 and/or the valve delivery system, and/or the valve retrieval catheter 190, can be used to aid in coupling the valve assembly 300 to various devices of the valve delivery system. Optionally, these configurations can be used alone, or in combination with other configurations described herein.

In some embodiments, the valve assembly 300 can be controlled using lassos or loops (e.g., control wires), similar to those described herein. For example, the control wires can be used to radially compress the valve assembly 300 to facilitate retrieval of the valve assembly 300. In such some embodiments, the control wires can be located on a proximal end portion of the valve assembly 300. Optionally, the control wires can aid in aligning the valve assembly 300 concentrically with the valve delivery sheath 170 during retrieval, and/or reduce the likelihood of a portion of the valve assembly 300 (e.g., valve arch hooks 313a-c), which can reduce a force needed to pull the valve assembly 300 into the valve delivery sheath 170. Compression of the valve assembly 300 can aid in retrieval of the valve assembly 300 because if the valve assembly 300 has a smaller radius, less force tends to be needed to pull the valve assembly 300 into the valve delivery sheath 170. Reduced force can reduce stress experienced by the valve assembly 300 during retrieval and subsequently reduce potential damage to the valve assembly 300.

In some embodiments, the valve delivery catheter 180 or the valve retrieval catheter 190 can include one or more hooks (e.g., two, three, four, or more hooks) located on a distal end portion of the catheter (e.g., valve delivery catheter 180 or the valve retrieval catheter 190). The hooks can extend outward and upward from the catheter (e.g., valve delivery catheter 180 or the valve retrieval catheter 190) to create a hook shape. In some embodiments, the hooks can extend inward and upward from an interior of the catheter to create a hook shape. The valve assembly 300 can include openings, such as loops, to engage with the hooks. For example, the loops can be located at the top of the arches of the valve assembly 300. In some embodiments, the valve assembly 300 can include a plurality of loops (e.g., three or more arch extensions). The number of hooks can correspond with the number of loops, such that each hook can engage with a loop, and vice-versa. Accordingly, the loops, and therefore the valve assembly 300, can be coupled to the catheter (e.g., valve delivery catheter 180 or the valve retrieval catheter 190), via the hooks. In some embodiments, the hooks and loops can aid in implantation of the valve assembly 300, retrieval of the valve assembly 300, or both. Optionally, the loops and/or the hooks can be visible with an imaging modality, such that the position and/or coupling of the attachment hooks and/or loops can be confirmed. In some embodiments, the loops are located on the catheter (e.g., valve delivery catheter 180 or the valve retrieval catheter 190) and the hooks are located on the valve assembly 300. When the hooks are located on the valve assembly, the hooks can face inward or outward.

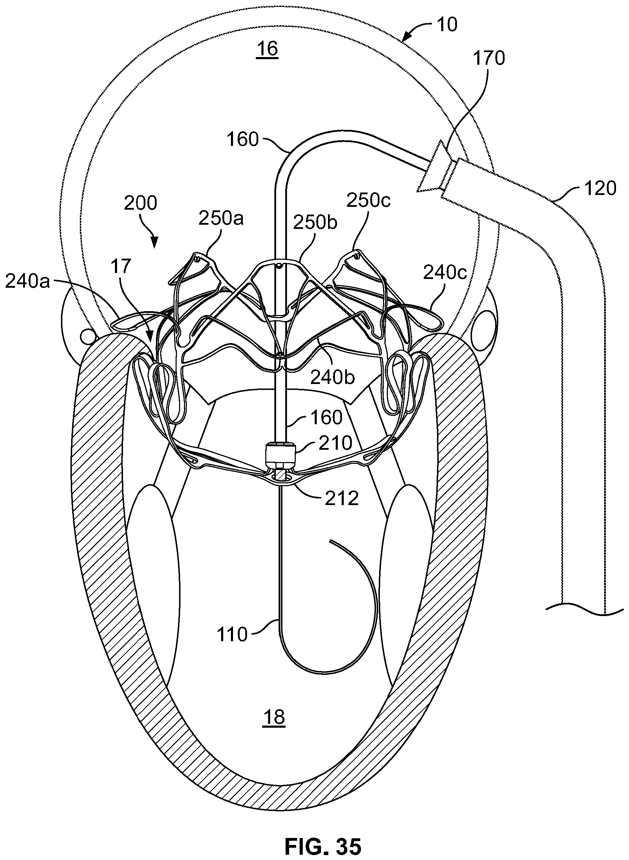

Referring to FIG. 35, after the valve assembly 300 has been retrieved from the heart 10, the valve assembly 300 can be disposed. In some embodiments, the valve delivery sheath 170 can be removed, while retaining the position of the inner catheter 160 and/or the guide catheter 120. In such a case, the implanted anchor assembly 200 can remain engaged with the native mitral valve 17. Accordingly, a new valve delivery system can be deployed for implantation of a new valve assembly with the anchor assembly 200 after retrieval of the initial valve assembly 300.

In some embodiments, the anchor assembly 200 can be retrieved in tandem with retrieval of the valve assembly 300. Using such a tandem retrieval technique, the anchor assembly 200 can be retrieved into the valve delivery sheath 170 closely behind the valve assembly 300.

In some embodiments, the valve delivery sheath 170 and/or the guide catheter 120 can have distal end portion with a flared, beveled, or funnel shape to aid in retrieval of the anchor assembly 200 and/or the valve assembly 300. Optionally, the distal end portion can self-expand to the pre-determined shape, such as during implantation, and/or the distal end portion can collapse to a lower profile, such as during removal. In some embodiments, the material of the distal end portion can include pleats and/or folds, can be a continuous flared end, and/or can be separated into sections, such as resembling flower petals. Optionally, the distal end portion can include one or more resilient elements that bias the distal end portion to assume the flared configuration in the absence of the restraining forces. Such a distal end portion can be advantageous, for example, for facilitating retrieval of the anchor assembly 200 and/or the valve assembly 300.

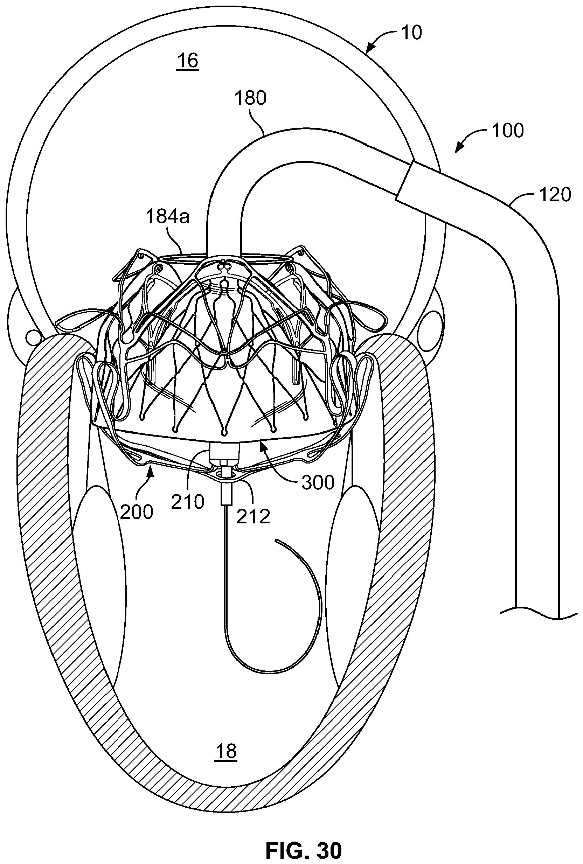

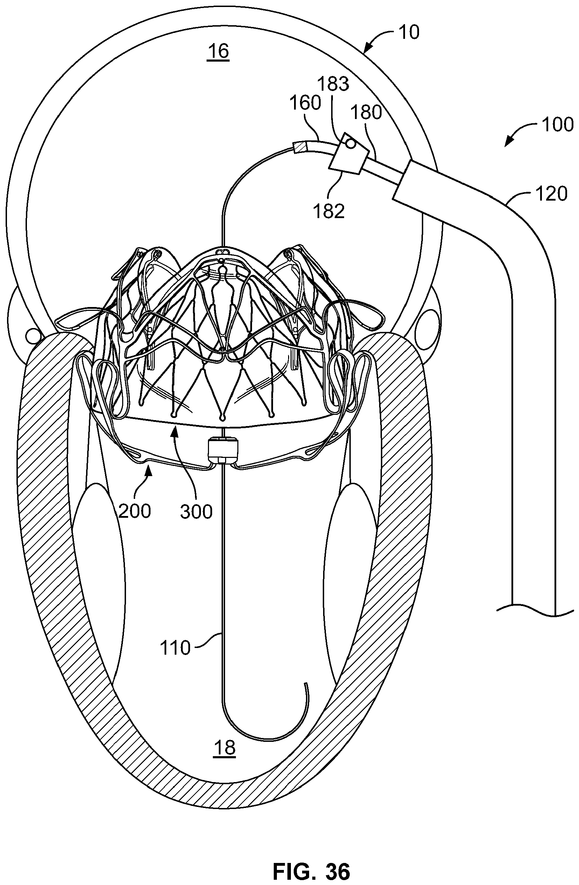

Referring now also to FIGS. 30 **and** 36, in some embodiments the valve assembly 300 and the anchor assembly 200 become aligned with each other coaxially, linearly (along their axes), and rotationally prior to or during the expansion of the valve assembly 300, resulting in engagement between the valve assembly 300 and the anchor assembly 200.

Coaxial alignment between the valve assembly 300 and the anchor assembly 200, as described above, is achieved by virtue of the valve delivery catheter 180 being slidably disposed over the inner catheter 160. Linear alignment between the valve assembly 300 and the anchor assembly 200 can be achieved by the interaction of a distal end feature 182 (FIG. 29) of the valve delivery catheter 180 and the hub 210 of the anchor assembly 200. For example, in some embodiments an abutting of the distal end feature 182 and the hub 210 can result in proper linear alignment between the valve assembly 300 and the anchor assembly 200. Such abutting of the distal end feature 182 and the hub 210 can be attained by translating the valve delivery catheter 180 distally until the distal end feature 182 abuts the hub 210.

Relative rotational alignment between the valve assembly 300 and the anchor assembly 200 (about their longitudinal axes) can be achieved in various manners. For example, in some embodiments the valve delivery catheter 180 is mechanically keyed to the inner catheter 160 to slidably fix a desired rotational alignment between the valve assembly 300 and the anchor assembly 200. In some embodiments, other types of mechanical features (e.g., pins/holes, protrusions/receptacles, etc.) can be included to facilitate a desired rotational/spin alignment between the valve assembly 300 and the anchor assembly 200. Alternatively, or additionally, one or more radiopaque markers can be included on the valve assembly 300 and/or on the anchor assembly 200 in locations and/or patterns that are indicative of the relative rotational orientation (about their axes) of the valve assembly 300 and the anchor assembly 200. Accordingly, fluoroscopy can be used to attain a desired relative orientation of the radiopaque markers and, consequently, of the valve assembly 300 and the anchor assembly 200. For example, in some embodiments one or more radiopaque markers 183 are disposed on the distal end feature 182. The one or more radiopaque markers 183 can be in locations and/or arranged in patterns to indicate the rotational orientation of the distal end feature 182 and, in turn, the rotational orientation of the valve assembly 300 that is releasably coupled in relation to the distal end feature 182. In some embodiments, the one or more radiopaque markers 183 can be arranged as one or more beads, one or more half-rings, and the like, and combinations thereof. One or more radiopaque markers can be included on the SAM containment member 212 in some embodiments.

In some embodiments (e.g., when the valve delivery catheter 180 is configured to be "torqueable"), the valve delivery catheter 180 can be rotated about its longitudinal axis until the radiopaque markers are in proper position relative to the anchor assembly 200, prior to final expansion of valve assembly 300. Such rotation of the valve delivery catheter 180 can, in some implementations, be initiated and controlled using a deployment frame. Fluoroscopy can be used to attain a desired relative orientation of the radiopaque markers, and of the valve assembly 300 and the anchor assembly 200 (including on the SAM containment member) correspondingly.

In the depicted implementation, the SAM containment member 212 is still in its pre-deployed configuration. Therefore, the depicted embodiment of the SAM containment member 212 is deployed after the valve assembly 300 is engaged within the anchor assembly 200. However, for some alternative embodiments of the SAM containment member (as described further below) the SAM containment member is deployed prior to the engagement of the valve assembly 300 within the anchor assembly 200.

After proper alignment between the valve assembly 300 and the anchor assembly 200 is achieved, the valve assembly 300 can be expanded within the interior of the anchor assembly 200 such that the valve assembly 300 and anchor assembly 200 become releasably coupled to each other. In some embodiments, force(s) are applied to the valve assembly 300 to cause it to expand. In some embodiments, the valve assembly 300 is biased to self-expand.

The expansion of a self-expanding valve assembly 300 can be initiated by releasing tension on the one or more control wires of the valve delivery catheter 180. For example, in some embodiments the valve delivery catheter 180 includes a proximal control wire 184a that restrains the proximal end portion of the valve assembly 300, and a distal control wire 184b that restrains the distal end portion of the valve assembly 300. As tension on the proximal control wire 184a is released, the proximal end portion of the valve assembly 300 is allowed to radially expand. Similarly, as tension on the distal control wire 184b is released, the distal end portion of the valve assembly 300 is allowed to radially expand. The expansions of the portions of the valve assembly 300 may be allowed to take place sequentially, concurrently, or partially concurrently. As described further below, such individual and/or simultaneous movements of components of the delivery system 100 (such as the one or more control wires of the valve delivery catheter 180) can be initiated and controlled using a deployment frame system in some implementations.

After the valve assembly 300 has been expanded into a coupled relationship with the anchor assembly 200, the clinician can verify that the anchor assembly 200 and the valve assembly 300 are in the desired positions. Additionally, the clinician may verify other aspects such as, but not limited to, the hemodynamic performance and sealing of the anchor assembly 200 and the valve assembly 300.

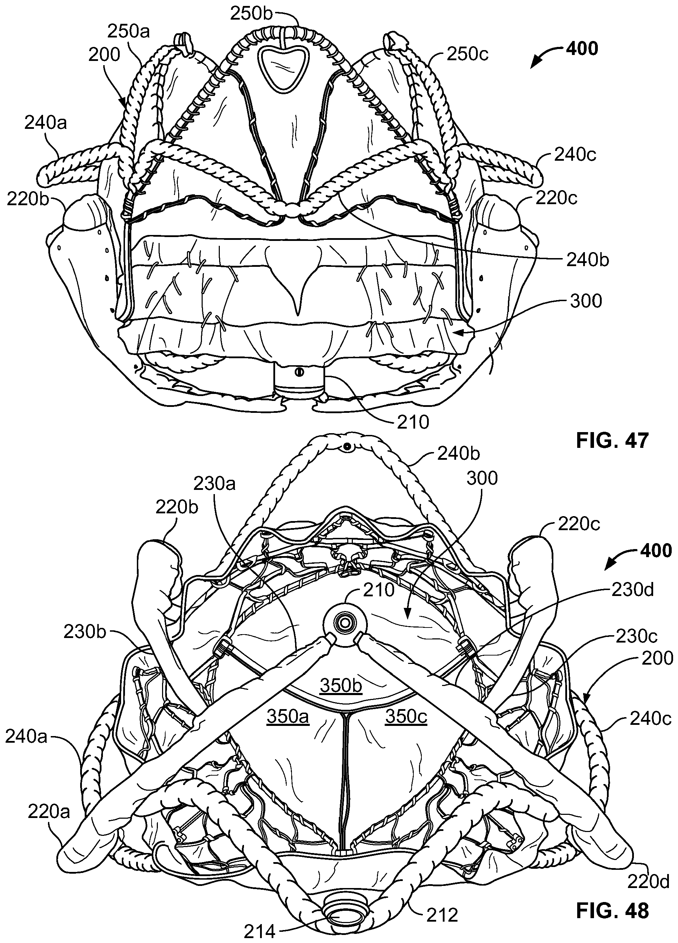

In some embodiments, the SAM containment member 212 is deployed after the valve assembly 300 has been expanded into a coupled relationship with the anchor assembly 200. To deploy the SAM containment member 212, in some embodiments the inner catheter 160 is rotated about its longitudinal axis so that the distal end of the inner catheter 160 is uncoupled from the hub 210 of the anchor assembly 200. For example, in some embodiments the distal end of the inner catheter 160 is uncoupled from the hub 210 by unthreading the distal end of the inner catheter 160 from the hub 210 by rotating the inner catheter 160 about its longitudinal axis. Then, in some embodiments the guidewire 110 is retracted to allow full deployment of the SAM containment member 212. The SAM containment member 212 may self-expand to its fully deployed configuration in some embodiments. The configuration of the fully deployed SAM containment member 212 is depicted in FIGS. 16-21 and 48, for example.

In its fully deployed configuration, the SAM containment member 212 is at least partially disposed behind the natural mitral valve anterior leaflet 20 (FIG. 12). The deployed SAM containment member 212 can reduce or prevent the potential for the natural mitral valve anterior leaflet 20 to "flop" outward and/or from being drawn by a Venturi effect into the left ventricular outflow tract (LVOT). Accordingly, the SAM containment member 212 can reduce the risk of full or partial blockages of the LVOT. In some patient scenarios, the potential for suffering future adverse health events, such as heart failure, is thereby reduced.

With the valve assembly 300 and the anchor assembly 200 fully deployed and functioning as desired, the remaining components of the delivery system 100 can be withdrawn. To do so, the valve delivery catheter 180 and the inner catheter 160 can be retracted into the guide catheter 120. Then the valve delivery catheter 180, the inner catheter 160, and the guide catheter 120 can be jointly or individually withdrawn from the patient.

Referring to **FIGS. 37 and 38****,** an example valve assembly 300 is shown without any covering or valve/occluder leaflets. Hence, a valve assembly frame 301 of the valve assembly 300 is shown. FIG. 36 shows an anterior side view of the valve assembly frame 301, and FIG. 37 shows a bottom view of the valve assembly frame 301. The valve assembly 300 can be constructed using any of the various materials and manufacturing techniques described above in reference to the anchor frame 200 (e.g., refer to FIG. 9). It should be understood that the depicted valve assembly 300 is merely one non-limiting example of the valve assemblies provided within the scope of this disclosure.

The valve assembly 300 includes a proximal end portion 302 and a distal end portion 304. The valve assembly includes a flared external skirt portion 303 and defines an interior orifice portion 305. When the valve assembly 300 is implanted in a native mitral valve, the proximal end portion 302 is located supra-annular (in the left atrium) and the distal end portion 304 is located sub-annular (in the left ventricle). The proximal end portion 302 defines the generally circular entrance orifice of the valve assembly 300, as described further below.

In the depicted embodiment, the valve assembly 300 generally flares outward along a distal direction. Said differently, the distal end portion 304 is flared outward in comparison to the proximal end portion 302. Accordingly, the proximal end portion 302 defines a smaller outer profile in comparison to the distal end portion 304. However, some regions of the distal end portion 304 bow inwardly. In particular, for example, a posteromedial commissural corner 330a and anterolateral commissural corner 330b of the valve assembly 300 may bow inwardly. Such inward bowing of the commissural corners 330a and 330b can serve to mitigate LVOT obstructions and enhance sealing in some cases. It should be understood that the outward flare of the distal end portion 304 in comparison to the proximal end portion 302 is merely one example configuration for a profile of the valve assembly 300. In some embodiments, for example, a shoulder (a portion of the valve assembly 300 having the largest outer periphery) is located proximal of the middle of the valve assembly 300.

The valve assembly 300 also includes an anterior side 306 between the posteromedial commissural corner 330a and anterolateral commissural corner 330b. When the valve assembly 300 is implanted in a native mitral valve, the anterior side 306 faces the anterior leaflet of the native mitral valve. The anterior side 306 of the distal end portion 304 defines a generally flat surface, whereas the other sides of the distal end portion 304 are rounded. Hence, the periphery of the distal end portion 304 is generally D-shaped. The D-shaped periphery of the distal end portion 304 provides the valve assembly 300 with an advantageous outer profile for interfacing and sealing with the native mitral valve. As described further below, sealing is attained by coaptation between the D-shaped periphery of the distal end portion 304 and the leaflets of the native mitral valve, and, in some embodiments, between the D-shaped periphery in the region of the skirt 303 with the native valve annulus.

In the depicted embodiment, the proximal end portion 302 of the valve assembly 300 includes three atrial leaflet arches 3 10a, 3 10b, and 310c that together define an undulating ring at the proximal end portion 302. Each of the leaflet arches 310a, 310b, and 310c includes an apex having one or more arch extensions 312a, 312b, and 312c respectively. In some embodiments, the arch extensions 312a, 312b, and 312c are used for coupling the proximal end of the valve assembly 300 to a delivery catheter (e.g., valve delivery catheter 180 of FIGS. 27-36 using proximal control wire 184a). In some embodiments, spaces defined by one or more of the arch extensions 312a, 312b, and 312c are used for containing radiopaque material.

The valve assembly 300 also includes three commissural posts 320a, 320b, and 320c that each extend distally from the intersections of the three leaflet arches 310a, 310b, and 310c. In some embodiments, the commissural posts 320a, 320b, and 320c are disposed at about 120° apart from each other. The commissural posts 320a, 320b, and 320c each have a series of holes that can be used for attachment of leaflets, such as by suturing. The three leaflet arches 310a, 310b, and 310c and the three commissural posts 320a, 320b, and 320c are areas on the valve assembly 300 to which three prosthetic valve leaflets become attached to comprise a tri-leaflet occluder (e.g., refer to FIG. 43).

As seen in FIG. 38, the three leaflet arches 310a, 3 10b, and 310c and the commissural posts 320a, 320b, and 320c define a generally cylindrical frame for the tri-leaflet occluder construct. As such, the valve assembly 300 provides a proven and advantageous frame configuration for the tri-leaflet occluder. The tri-leaflet occluder provides open flow during diastole and occlusion of flow during systole.

Referring to **FIGS. 39 and 40****,** another variation of the valve assembly 300 described above is shown without any covering or valve/occluder leaflets. Hence, a valve assembly frame 301 of the valve assembly 300 is shown. FIG. 39 shows an anterior side view of the valve assembly frame 301, and FIG. 40 shows a bottom view of the valve assembly frame 301.

In the depicted embodiment, the proximal end portion 302 of the valve assembly 300 includes three atrial leaflet arches 310a, 310b, and 310c that together define an undulating ring at the proximal end portion 302. Each of the leaflet arches 310a, 310b, and 310c includes an apex from which extends a valve arch hook 313a, 313b, and 313c respectively. The valve arch hooks 313a-c can be used for coupling with the valve retrieval catheter 190 (FIGS. 31 and 32) to facilitate retrieval of the valve assembly 300 as described above. As shown in the depicted embodiment, the valve arch hooks 313a, 313b, and 313c can be biased to extend radially away from the center of the valve assembly 300 at an acute angle. Accordingly, when the valve arch hooks 313a, 313b, and 313c abut the valve engagement portion 192 of the valve retrieval catheter 190, the valve arch hooks 313a, 313b, and 313c become radially compressed and then return to the original outwardly biased position when they pass through to an interstitial opening of a framework of the valve engagement portion 192. Accordingly, the valve arch hooks 313a, 313b, and 313c, and therefore the valve assembly 300, can be coupled to the valve retrieval catheter 190.

The valve arch hooks 313a, 313b, and 313c can include a hole or other type of attachment structure for one or more suture loops. Such suture loops can be used for coupling the valve arch hooks 313a-c the proximal end of the valve assembly 300 to a catheter (e.g., valve delivery catheter 180 of FIGS. 27-36 using proximal control wire 184a, or). In some embodiments, one or more portions of the valve arch hooks 313a, 313b, and 313c are used for containing radiopaque material.

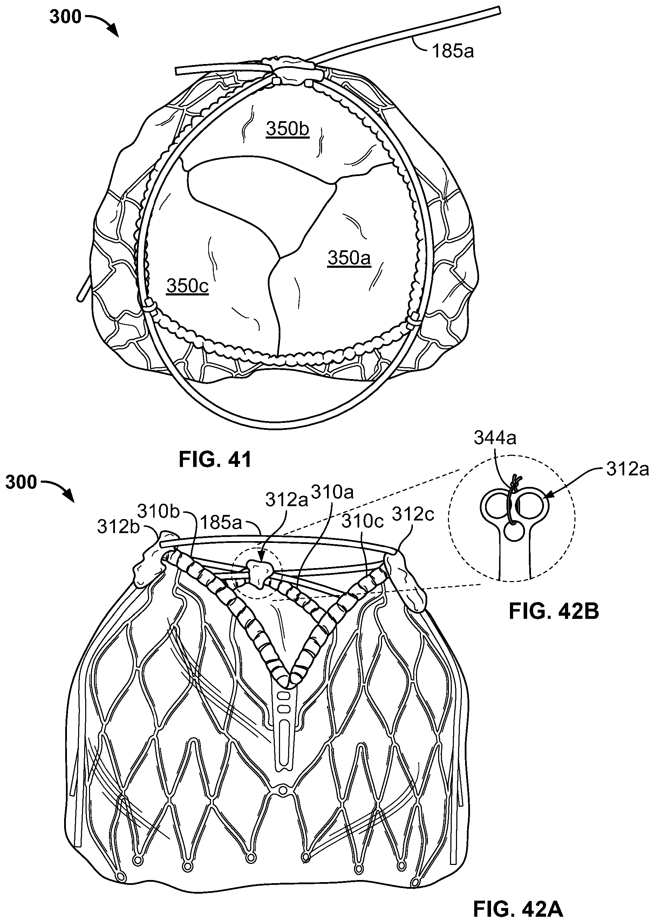

Referring to **FIGS. 41, 42A, 42B** **and** **43****,** in some embodiments the valve assembly 300 is configured to make the process of coupling one or more control wires (e.g., control wires 142a and 142b as described above in reference to FIGS. 3 and 4) to the valve assembly 300 more convenient. For example, in the depicted embodiment the valve assembly 300 is releasably coupled with a proximal end threading tube 185a and a distal end threading tube 185b. The threading tubes 185a and 185b can be used by a clinician as tools for threading the control wires 142a and 142b into engagement with the valve assembly 300. After using the threading tubes 185a and 185b to thread the control wires 142a and 142b into engagement with the valve assembly 300, the clinician can uncouple the threading tubes 185a and 185b from the valve assembly 300 and discard the threading tube 185a and 185b.

It should be understood that, in some embodiments, the valve assembly 300 is stored and transported to clinicians in sterile packaging containing a storage solution that keeps the valve assembly 300 moist. The storage solution is beneficial for preserving tissue of the valve assembly 300 during shipment and storage. The valve assembly 300 is not coupled to a delivery system during shipment and storage of the valve assembly 300. Therefore, an individual at the end-use site (e.g., a clinician in preparation for a procedure) will perform the task of coupling the valve assembly 300 to the delivery system (e.g., delivery system 100 as described above). In some embodiments, the task of coupling the valve assembly 300 to the delivery system includes coupling control wires (e.g., proximal control wire 184a and distal control wire 184b) to the valve assembly 300. Because the task of coupling control wires to the valve assembly 300 can be time-consuming, in some embodiments the valve assembly 300 is provided with one or more threading tubes, such as the proximal end threading tube 185a and the distal end threading tube 185b in the depicted embodiment.

The threading tubes 185a and 185b can be made of various materials such as, but not limited to, polyether ether ketone (PEEK), polyaryl ether ketone (PAEK), PTFE, FEP, HYTREL^{®}, nylon, PICOFLEX^{®}, PEBAX^{®}, TECOFLEX^{®}, nitinol, and the like, and combinations thereof.

In some embodiments, the proximal end threading tube 185a is releasably engaged with the valve assembly 300. For example, in the depicted embodiment the proximal end threading tube 185a passes through one or more attachment features (suture loops in this example) at the arch extensions 312a, 312b, and 312c that are located at the apices of the leaflet arches 310a, 310b, and 310c respectively. In the depicted example of FIG. 42B, a suture loop 344a is attached at the apex of leaflet arch 310a using the arch extension 312a. The same or a similar type of arrangement can be used at the arch extensions 312b and 312c located at the apices of leaflet arches 310b and 310c respectively. While in the depicted embodiment a single suture loop 344a is used, in some embodiments two or more suture loops are included at a single site. Such an arrangement can be used for redundancy, for example. The suture loops can be constructed of materials such as, but not limited to, ultra-high molecular weight polyethylene, nylon, polypropylene, polybutester, and the like. In some embodiments, other types of attachment elements (other than suture loops) such as, but not limited to, eyelets, grommets, rings, clips, pins, fabric portions, and/or the like, are used as to couple a threading tube (and control wire) to the valve assembly 300.

In some embodiments, the distal end threading tube 185b is releasably engaged with the valve assembly 300. For example, in the depicted embodiment the distal end threading tube 185b passes through one or more attachment features (suture loops in this example) that are located on or near the distal end of the framework of the valve assembly 300. In some embodiments, the distal end threading tube 185b can be used to couple the distal control wire 142b to the distal portion of the valve assembly 300.

In some implementations, a clinician can perform the following technique for using the threading tubes 185a and 185b to thread the control wires 142a and 142b into engagement with the valve assembly 300. For example, a clinician can insert a free end of the proximal control wire 142a into a lumen of the proximal end threading tube 185a at a first end of the proximal end threading tube 185a. The clinician can push the proximal control wire 142a in relation to the proximal end threading tube 185a, through the lumen of the proximal end threading tube 185a, until the free end emerges from a second end (opposite of the first end) of the proximal end threading tube 185a. Then, while holding the proximal control wire 142a essentially stationary in relation to the valve assembly 300, the clinician can slide the proximal end threading tube 185a out of engagement with the valve assembly 300, and off of the proximal control wire 142a. The proximal end threading tube 185a can then be discarded. The technique for using the distal end threading tube 185b to couple the distal control wire 142b to the distal portion of the valve assembly 300 can be the same technique as described in regard to the proximal end threading tube 185a. Thereafter, each of the free ends of the control wires 142a and 142b, having been passed through the suture loops, can be fed back into the distal portion of the valve delivery catheter 180 (FIGS. 27-36) and to a proximal securement and control system (not shown). The control wires 142a and 142b can then be tensioned which will reduce the diameter of the valve assembly 300, and allow for insertion into the distal end of the valve delivery sheath 170.

Still referring to **FIGS. 41, 42A, 42B** **and** **43****,** the valve assembly 300 can include an occluder portion, such as a tri-leaflet occluder or another type of occluder. For example, in the depicted embodiment the valve assembly 300 includes three leaflets 350a, 350b, and 350c that perform the occluding function of the prosthetic mitral valve 400. The cusps of the three leaflets 350a, 350b, and 350c are fixed to the three atrial leaflet arches 310a, 3 10b, and 310c, and to the three commissural posts 320a, 320b, and 320c (refer to FIGS. 20 and 21). The free edges of the three leaflets 350a, 350b, and 350c can seal by coaptation with each other during systole and open during diastole.

The three leaflets 350a, 350b, and 350c can be comprised of natural or synthetic materials. For example, the three leaflets 350a, 350b, and 350c can be comprised of any of the materials described above in reference to the covering 340, including the natural tissues such as, but not limited to, bovine, porcine, ovine, or equine pericardium. In some such embodiments, the tissues are chemically cross-linked using glutaraldehyde, formaldehyde, or triglycidyl amine solution, or other suitable crosslinking agents. In some embodiments, the leaflets 350a, 350b, and 350c have a thickness in a range of about 0.005" to about 0.020" (about 0.13 mm to about 0.51 mm), or about 0.008" to about 0.012" (about 0.20 mm to about 0.31 mm). In some embodiments, the leaflets 350a, 350b, and 350c have a thickness that is less than about 0.005" (about 0.13 mm) or greater than about 0.020" (about 0.51 mm).

Referring to **FIG. 44****,** an exploded depiction of an example prosthetic mitral valve 400 includes an anchor assembly 200 and a valve assembly 300. This figure provides a posterior side view of the anchor assembly 200 and the valve assembly 300.

The valve assembly 300 includes a covering 340. The covering 340 can be made of any of the materials and constructed using any of the techniques described above in reference to covering 270. Additionally, in some embodiments the covering 340 can comprise natural tissues such as, but not limited to, bovine, porcine, ovine, or equine pericardium. In some such embodiments, the tissues are chemically cross-linked using glutaraldehyde, formaldehyde, or triglycidyl amine solution, or other suitable crosslinking agents.

When the valve assembly 300 and the anchor assembly 200 are coupled together, the valve assembly 300 is geometrically interlocked within the interior defined by the anchor assembly 200 (e.g., in some embodiments by virtue of the tapered shape of the proximal end 302 valve assembly 300 within the supra-annular ring 250 and interior space defined by the anchor assembly 200). In particular, in some embodiments the valve assembly 300 is contained within the interior space between the supra-annular ring 250 and the sub-annular support arms 230a, 230b, 230c, and 230d. As described above, the interlocked arrangement between the valve assembly 300 and the anchor assembly 200 is accomplished by positioning a valve assembly 300 in a low-profile configuration within the interior of the anchor assembly 200 and then allowing expansion of the valve assembly 300 within the interior of the anchor assembly 200 (e.g., refer to FIGS. 29, 30, and 36).

In some embodiments, such as the depicted embodiment, a fabric portion 314a is attached (e.g., sewn) to the outer surface of coving 340 near the apex of the leaflet arch 310a. The other leaflet arches 310b and 310c can also have such a fabric portion. The fabric portion 314a aligns up with the covering-material cut out 252b of the anchor assembly 200 when the valve assembly 300 is coupled with the anchor assembly 200. By positioning the fabric portion 314a within the covering-material cut out 252b, the valve assembly 300 becomes coupled with the anchor assembly 200 with an additional resiliency. This additional securement resiliency may be advantageous, for example, to resist migration of the valve assembly 300 into the ventricle during diastole.

While in the depicted embodiment a triangular shape is used for the fabric portion 314a and the covering-material cut out 252b, in some embodiments other shapes such as, but not limited to, polygons, circles, ovals, and the like can be used. In some embodiments, the fabric portion 314a (and the other fabric portions on leaflet arches 310b and 310c) is made of a material such as, but not limited to, felt, polyester, a silicone, a urethane, ELAST-EON^{™} (a silicone and urethane polymer), another biocompatible polymer, DACRON^{®}, polyethylene terephthalate (PET), copolymers, or combinations and subcombinations thereof.

In some embodiments, one or more supplementary covering portions are attached (e.g., sewn) to the outer surface of the covering 340 of the valve assembly 300. In some cases, such supplementary covering portions can provide an enhanced sealing capability between the skirt 303 and surrounding native tissues when the prosthetic mitral valve 400 is deployed in a native mitral valve. Moreover, such supplementary covering portions can facilitate tissue healing and/or ingrowth, which can in turn provide enhanced sealing. For example, in the depicted embodiment, the valve assembly 300 includes a first supplementary covering portion 316a and a second supplementary covering portion 316b. In some embodiments, the first supplementary covering portion 316a and the second supplementary covering portion 316b are made of a material such as, but not limited to, DACRON^{®}, felt, polyester, a silicone, a urethane, ELAST-EON^{™} (a silicone and urethane polymer), another biocompatible polymer, polyethylene terephthalate (PET), copolymers, or combinations and subcombinations thereof.

While the valve assembly 300 is shown with the arch extensions 312a, 312b, and 312c, other embodiments of the valve assembly 300, as described with respect to FIG. 44 and elsewhere herein, can include valve arch hooks 313a, 313b, and 313c, as described with respect to FIGS. 39 and 40.

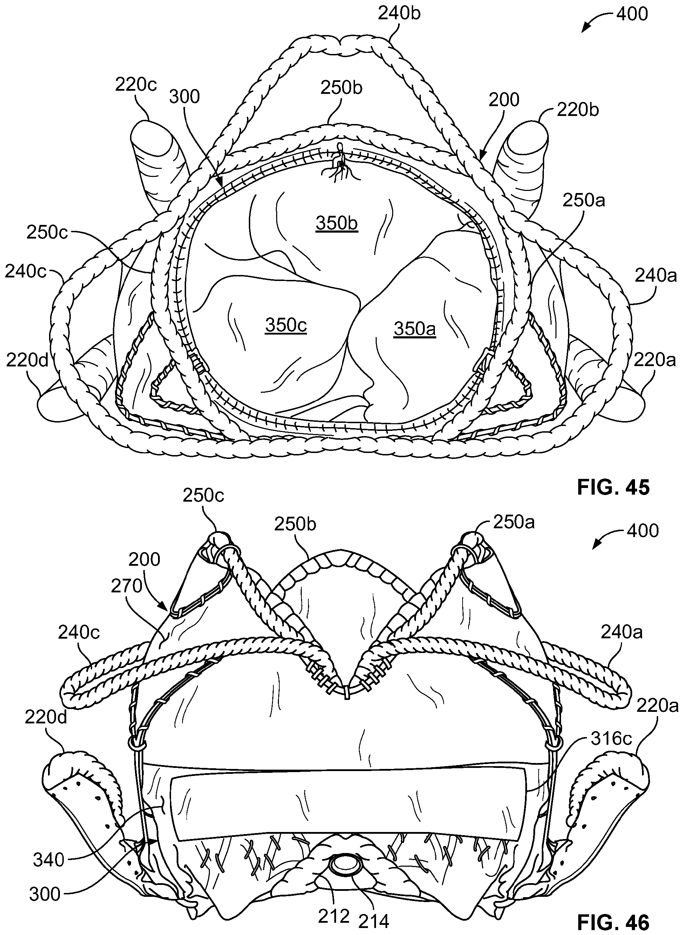

Referring to **FIGS. 45-48****,** the prosthetic mitral valve 400 (comprised of the valve assembly 300 coupled within the anchor assembly 200) is shown in top (atrial), anterior, posterior, and bottom (ventricle) views, respectively. In some embodiments, the occluding function of the prosthetic mitral valve 400 can be performed using configurations other than the depicted tri-leaflet occluder. For example, bi-leaflet, quad-leaflet, or mechanical valve constructs can be used in some embodiments.

As shown in FIG. 46, a supplemental covering portion 316c can positioned on an anterior surface of the valve assembly 300. The supplemental covering portion 316c can provide an enhanced sealing capability between the skirt 303 and surrounding native tissues (e.g., an anterior leaflet) when the prosthetic mitral valve 400 is deployed in a native mitral valve. The supplemental covering portion 316c can be made of a material such as, but not limited to, DACRON^{®}, felt, polyester, a silicone, a urethane, ELAST-EON^{™} (a silicone and urethane polymer), another biocompatible polymer, polyethylene terephthalate (PET), copolymers, or combinations and subcombinations thereof.

Referring to **FIG. 49****,** in some implementations the prosthetic mitral valve 400 of FIGS. 44-48 is deployed in a patient 1 using the transcatheter delivery system 100 as described above. In some implementations, the prosthetic mitral valve 400 is percutaneously deployed via a femoral or iliac vein through a groin opening/incision 2 in the patient 1. In particular implementations, a deployment frame system 6 is used to initiate and/or control the movements of various components of the transcatheter delivery system 100.

While the deployment frame system 6 is described in the context of the deployment of the prosthetic mitral valve 400 using the transcatheter delivery system 100, it should be understood that the practical applications of the inventive concepts associated with the deployment frame system 6 is not limited to such a context. That is, the inventive concepts associated with the deployment frame system 6 can be applied to contexts such as, but not limited to, other types of delivery systems for prosthetic heart valves of any type, deployment systems for other types of medical devices/implants, and so on.

In the depicted embodiment, the deployment frame system 6 is attached or releasably attached to an operating table 4 on which the patient 1 is laying. In some embodiments, the deployment frame system 6 is separated or substantially separated from the operating table 4.

As described above in reference to FIGS. 1-11 and 26-36, the deployment of the prosthetic mitral valve 400 is, in summary, a two-step process. The first step is the deployment of the anchor assembly 200, and the second step is the deployment of the valve assembly 300. Some components of the deployment frame system 6 may be used for both steps, while other components of the deployment frame system 6 may be used for one or the other of the two steps.

In general, the configuration of the deployment frame system 6 is different for the two deployment steps (i.e., the first step being the deployment of the anchor assembly 200, and the second step being the deployment of the valve assembly 300). That is, the configuration of the deployment frame system 6 for delivering the anchor assembly 200 is different than the configuration of the deployment frame system 6 for delivering the valve assembly 300.

The transcatheter delivery system 100 can be releasably coupled with deployment frame system 6, as described further below. The deployment frame system 6 can be used by one or more clinicians to initiate and control movements of the components of the delivery system 100. Some such movements of the components of the delivery system 100 are described above in reference to FIGS. 1-11 and 26-36.

As described above, the example transcatheter delivery system 100 includes the guidewire 110, the guide catheter 120, the anchor delivery sheath 130, the anchor delivery catheter 140, the secondary steerable catheter 150, and the inner catheter 160. In general, in the depicted embodiment those components of delivery system 100 are disposed in a telescopic fashion in relation to each other. That is, the guidewire 110 is slidably disposed within the inner catheter 160; the inner catheter 160 is slidably disposed within the secondary steerable catheter 150; the secondary steerable catheter 150 is slidably disposed within the anchor delivery catheter 140; the anchor delivery catheter 140 is slidably disposed within the anchor delivery sheath 130; and the anchor delivery sheath 130 is slidably disposed within the guide catheter 120.

A proximal end portion of those components (e.g., the guide catheter 120, the anchor delivery sheath 130, the anchor delivery catheter 140, the secondary steerable catheter 150, and the inner catheter 160) can be terminated at a respective location along the deployment frame system 6. As described further below, by manipulating the respective components' proximal end portions (individually or in unison) using the deployment frame system 6, clinicians can initiate and control movements of the delivery system 100. In some embodiments, the example deployment frame system 6 includes a main frame and a secondary frame.

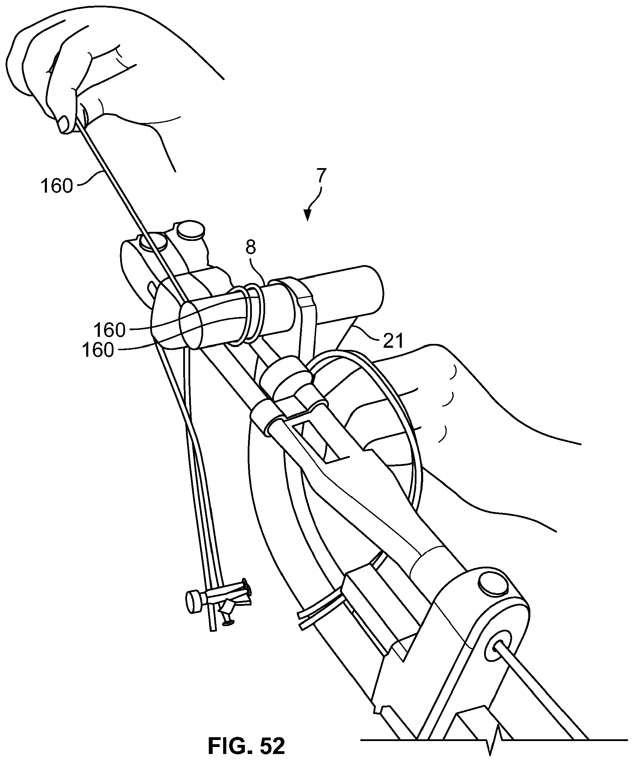

Referring to **FIGS. 50-52****,** in some implementations the deployment frame system 6 can be used to initiate and/or control the movements of various components of the transcatheter delivery system 100 as described above. In some such implementations, an anchor retrieval accessory 7 can be selectively attached to the deployment frame system 6, such as when it is desired to retrieve an anchor assembly 200. For example, FIG. 50 shows the anchor retrieval accessory 7 prior to being attached to the deployment frame system 6, and FIG. 51 shows the anchor retrieval accessory 7 after being attached to the deployment frame system 6. FIG. 52 shows the anchor retrieval accessory 7 being used while it is attached to the deployment frame system 6.

The anchor retrieval accessory 7 can be used when needed to help facilitate retrieval of the anchor assembly 200. In the depicted embodiment, the anchor retrieval accessory 7 includes a capstan 8 that can be manually rotated using a handle 21. Such an arrangement can provide a mechanical advantage that can be used to reduce the amount of manual force required to retrieve the anchor assembly 200. In some embodiments, the anchor retrieval accessory 7 can provide a substantially constant force to retrieve the anchor assembly 200. For example, a slip clutch mechanism can be included in some embodiments.

As described above in reference to FIGS. 1-11 and 26-36, the deployment of the two-part prosthetic mitral valve 400 is, in summary, a two-step process. The first step is the deployment of the anchor assembly 200, and the second step is the deployment of the valve assembly 300. In some embodiments, the process can include retrieval of the anchor assembly 200.

As described above, the example transcatheter delivery system 100 can include, but is not limited to, the guidewire 110, the guide catheter 120, the anchor delivery sheath 130, the anchor delivery catheter 140, the secondary steerable catheter 150, and the inner catheter 160. After deploying the anchor assembly 200, the anchor delivery sheath 130, the anchor delivery catheter 140, and the secondary steerable catheter 150 can be removed.

The deployment frame system 6 can be used by one or more clinicians to initiate and control movements of the components of the delivery system 100. Accordingly, the transcatheter delivery system 100 can be releasably coupled with deployment frame system 6, as described further herein. Some such movements of the components of the delivery system 100 are described above in reference to FIGS. 1-11 and 26-36. In some embodiments, the anchor retrieval accessory 7 can be used to initiate and control some such movements during the retrieval of the anchor assembly 200, such as movements of the inner catheter 160.

To facilitate retrieval of the anchor assembly 200, the anchor retrieval sheath 156 (FIG. 25) can be installed by passing it over the inner catheter 160 and inside the guide catheter 120. In general, in the depicted embodiment those components of delivery system 100 are disposed in a telescopic fashion in relation to each other. That is, for example, the guidewire 110 is slidably disposed within the inner catheter 160; the inner catheter 160 is slidably disposed within the anchor retrieval catheter 156 (when installed into the delivery system 100); and the anchor retrieval catheter 156 is slidably disposed within the guide catheter 120.

Proximal end portions of those components (e.g., the guide catheter 120, the anchor delivery sheath 130, the anchor delivery catheter 140, the secondary steerable catheter 150, the anchor retrieval catheter 156, and the inner catheter 160) can be terminated at respective locations along the deployment frame system 6 (including the anchor retrieval accessory 7). As described further herein, by manipulating the respective components' proximal end portions (individually or in unison) using the deployment frame system 6, clinicians can initiate and control movements of the delivery system 100, and of the anchor and valve assemblies coupled thereto.

For example, using the anchor retrieval accessory 7, the inner catheter 160 can be pulled proximally to retrieve the anchor assembly 200 from the heart 10. In some embodiments, the anchor assembly 200 can be pulled using the inner catheter 160 so that the hub 210, and then the other portions of the anchor assembly 200, enter the guide catheter 120 in an inverted fashion. Optionally, the guide catheter 120 can be rotated to aid in pulling the hub 210 and the other portions of the anchor assembly 200 into the guide catheter 120.

A proximal portion of the inner catheter 160 can be wrapped around the capstan 8 of the anchor retrieval accessory 7. For example, in some cases the inner catheter 160 can be wrapped around the capstan 8 away from the user, without any overlap. The lever handle 21 on the anchor retrieval accessory 7 can be manually rotated to pull the inner catheter 160 proximally to thereby retrieve the anchor assembly 200 into the guide catheter 120.

Referring to **FIGS. 53 and 54****,** in some implementations a valve retrieval accessory 9 can be selectively attached to the deployment frame system 6 when retrieval of the valve assembly 300 is so desired. The valve retrieval accessory 9 can be used to reduce the amount of manual force required to retrieve the valve assembly 300. In some embodiments, the valve retrieval accessory 9 can provide a controlled, substantially constant force to retrieve the valve assembly 300.

As described above in reference to FIGS. 1-11 and 26-36, the deployment of the two-part prosthetic mitral valve 400 is, in summary, a two-step process. The first step is the deployment of the anchor assembly 200, and the second step is the deployment of the valve assembly 300. In some cases, the retrieval of the valve assembly 300 may also be performed as a part of the process.

The two-part prosthetic mitral valve 400 is deployable using the transcatheter delivery system 100 that can be controlled by the deployment frame system 6, as described further herein. That is, the deployment frame system 6 can be used by one or more clinicians to initiate and control movements of the components of the delivery system 100. Some such movements of the components of the delivery system 100 are described above in reference to FIGS. 1-11 and 26-36. In some cases, the valve retrieval accessory 9 can be used to initiate and control some such movements during retrieval of the valve assembly 300.

As described above, the example transcatheter delivery system 100 includes, but is not limited to, the guidewire 110, the guide catheter 120, the inner catheter, the valve delivery catheter 180, the valve delivery sheath 170, and/or the valve retrieval catheter 190. When retrieving the valve assembly 300, the valve retrieval catheter 190 can be passed over the valve delivery catheter 180 and inside the valve delivery sheath 170. In general, in the depicted embodiment those components of delivery system 100 are disposed in a telescopic fashion in relation to each other. That is, the guidewire 110 is slidably disposed within the inner catheter 160, the inner catheter 160 is slidably disposed within the valve delivery catheter 180; the valve delivery catheter 180 is slidably disposed within the valve retrieval catheter 190; the valve retrieval catheter 190 is slidably disposed within the valve delivery sheath 170; and the valve delivery sheath 170 is slidably disposed within the guide catheter 120. A proximal end portion of those components (e.g., the guide catheter 120, the valve delivery sheath 170, the valve retrieval catheter 190, the valve delivery catheter 180, and the inner catheter 160) can be terminated at a respective location along the deployment frame system 6. In some embodiments, the proximal end portion of these components (e.g., the guide catheter 120, the valve delivery sheath 170, the valve retrieval catheter 190, the valve delivery catheter 180, and the inner catheter 160) can include a carrier on the deployment system 6 that can couple the component to the deployment system 6. As described further herein, by manipulating the respective components' proximal end portions (individually or in unison) using the deployment frame system 6, clinicians can initiate and control movements of the delivery system 100.

In the depicted embodiment, the valve retrieval accessory 9 defines two carrier engagement compartments 31a and 31b (e.g., open areas defined by U-shaped frame portions of the valve retrieval accessory 9) that can be positioned around the carriers of various components (e.g., the valve delivery catheter 180 and the valve retrieval catheter 190) on the deployment system 6 as depicted in FIG. 54. In some cases, the positions of the carrier engagement compartments 31a-b relative to each other can be adjusted or otherwise modified to accommodate different positons of the various component carriers with which the carrier engagement compartments 31a-b are to be engaged. By engaging the carrier engagement compartments 31a-b and the carriers of the deployment system 6, the engaged carriers can be longitudinally translated distally and proximally in unison with each other using the valve retrieval accessory 9.

The valve retrieval accessory 9 can be secured to the deployment frame system 6 via an anchor 37. In some embodiments, the valve retrieval accessory 9 includes a load indicator spring 33 and a lead screw 35. The valve retrieval accessory 9 can also include a handle 39 (e.g., a slide bar handle) that can be used to actuate movements of the carrier engagement compartments 31a-b in a distal or proximal longitudinal translation along the lead screw and in relation to the frame of the deployment system 6. By virtue of the lead screw 35 and handle 39, the valve retrieval accessory 9 can reduce the amount of manual force from the clinician-user that is required to retrieve the valve assembly 300. The valve retrieval accessory 9 can increase consistency of forces (e.g., 30 lbs) used to retrieve the valve assembly 300. In some cases, the valve retrieval accessory 9 can be used during tandem retrieval of the valve assembly 300 and the anchor assembly 200.

As described above in reference to FIGS. 1-11 and 26-36, various movements of the components of the delivery system 100 may be desired during the process of deploying (or retrieving) a medical device, such as the anchor assembly 200 and valve assembly 300 of prosthetic mitral valve 400 (refer to FIG. 44). For example, the types of desired movements of the components of the delivery system 100 may include, but are not limited to: (i) a distal longitudinal translation, (ii) a proximal longitudinal translation, (iii) rotations about the longitudinal axis in either direction, (iv) a deflection of one or more portions of a component (e.g., steering or bending), and (v) a tensioning or untensioning of a control wire.

In some implementations, it may be desirable to initiate some of such movements (e.g., example movements (i)-(v) above) in synchronization (e.g., generally simultaneously) with one or more other such movements. One example of desirable simultaneous movement of two or more components of the delivery system 100 was described above in reference to FIG. 7. In that example, the inner catheter 160 and the anchor delivery catheter 140 were translated distally in conjunction with each other, while maintaining the positions of the other components of the delivery system 100 (e.g., the secondary steerable catheter 150) generally stationary. In some embodiments, accessories that include clips and/or clamps can be used to releasably lock two or more various components (e.g., the guide catheter 120, the anchor delivery sheath 130, the anchor delivery catheter 140, the secondary steerable catheter 150, the anchor retrieval catheter 156, the inner catheter 160, the valve delivery sheath 170, the valve retrieval catheter 190, the valve delivery catheter 180, and/or the guidewire 110) to one another for synchronized movement. Optionally, the components can be locked to one another for axial movement, rotational movement, or both. The secondary frame of the deployment frame system 6 can be advantageously utilized to facilitate such synchronization of movements of two or more components of the delivery system 100. For example, the anchor retrieval sheath 156 and the inner catheter 160 can be locked together, once the anchor assembly 200 has been pulled inside the anchor retrieval sheath 156. As another example, the valve delivery catheter 180 and the valve retrieval catheter 190 can be locked together using the valve retrieval accessory 9 during retrieval of the valve assembly 300.

A number of embodiments of the invention have been described. Nevertheless, it will be understood that various modifications may be made without departing from the scope of the invention as defined by the claims. Accordingly, other embodiments are within the scope of the following claims.

## Claims

1. A two-part prosthetic heart valve, being comprised of an anchor assembly (200) and a valve assembly (300), and deployment system, comprising:
a guide catheter (120) defining a lumen, the guide catheter sized for insertion within a vasculature of a human patient;
an inner catheter (160) releasably coupleable with the anchor assembly (200) of the prosthetic heart valve, wherein the inner catheter (160) and the anchor assembly (200) are disposable within the lumen of the guide catheter (120) while the anchor assembly (200) is in a low-profile delivery configuration;
a valve delivery catheter (180) releasably coupleable with the valve assembly (300) of the prosthetic heart valve, wherein the valve delivery catheter (180) and the valve assembly (300) are disposable within the lumen of the guide catheter (120) while the valve assembly (300) is in a low-profile delivery configuration; and
a valve retrieval catheter (190) defining a lumen, wherein the valve delivery catheter (180) is disposable within the lumen of the valve retrieval catheter (190), and wherein a distal end portion of the valve retrieval catheter (190) includes a valve engagement portion (192) that is configured to selectively mechanically engage with the valve assembly (300), wherein the valve engagement portion (192) comprises a framework structure defining one or more interstitial open spaces that are sized and positioned to selectively mechanically engage with one or more hooks on a proximal end of the valve assembly, and wherein the framework structure is thermally coupled to a shaft of the valve retrieval catheter (190); and
a valve delivery sheath (170) defining a lumen, wherein the valve retrieval catheter (190) is disposable within the lumen of the valve delivery sheath (170).

2. The two-part prosthetic heart valve and deployment system of claim 1, wherein the one or more hooks on the proximal end of the valve assembly are each biased to project radially outward away from a center of the valve assembly at an acute angle.

3. The two-part prosthetic heart valve and deployment system of any of claims 1 and 2, wherein the one or more hooks on the proximal end of the valve assembly are configured to snap into mechanical engagement with the valve engagement portion.

4. The two-part prosthetic heart valve and deployment system of any of claims 1-3, wherein the valve assembly (300) includes arches (310a-c), and a top of the arches (310a-c) includes the one or more hooks (313a-c) on the proximal end of the valve assembly (300).

5. The two-part prosthetic heart valve and deployment system of any of claims 1-4, wherein the one or more hooks (313a-c) on the proximal end of the valve assembly (300) are configured such that when they start to make contact with the valve engagement portion (192), they will be radially compressed to deflect inward.

6. The two-part prosthetic heart valve and deployment system of claim 5, wherein the one or more hooks (313a-c) on the proximal end of the valve assembly (300) are configured to pass into the one or more interstitial open spaces of the framework structure and then return to a radially outward biased position when the one or more hooks (313a-c) on the proximal end of the valve assembly (300) pass into the one or more interstitial open spaces.

7. The two-part prosthetic heart valve and deployment system of any of claims 1-6, wherein one of the valve delivery catheter (180) and the valve retrieval catheter (190) includes one or more catheter hooks on a distal end portion.

8. The two-part prosthetic heart valve and deployment system of claim 7, wherein the one or more catheter hooks on one of the valve delivery catheter and the valve retrieval catheter extend outward and upward from the catheter to create a hook shape.

9. The two-part prosthetic heart valve and deployment system of claim 7, wherein the one or more catheter hooks on one of the valve delivery catheter and the valve retrieval catheter extend inward and upward from an interior of the catheter to create a hook shape.

10. The two-part prosthetic heart valve and deployment system of any of claims 1-9, further comprising an anchor retrieval sheath defining a lumen, wherein the inner catheter and the anchor assembly are disposable within the lumen of the anchor retrieval sheath, and wherein the anchor retrieval sheath is disposable within the lumen of the guide catheter.

11. The two-part prosthetic heart valve and deployment system of claim 10, wherein a distal end portion of the anchor retrieval sheath is positioned distally of a distal end of the guide catheter such that the anchor retrieval sheath protrudes from the guide catheter and into a left atrium.

12. The two-part prosthetic heart valve and deployment system of any of claims 10 and 11, wherein the anchor retrieval sheath includes an expandable funnel or beveled shaped distal end portion.

13. The two-part prosthetic heart valve and deployment system of any of claims 10-12, wherein the anchor retrieval sheath and the inner catheter (160) are configured to be locked together, once the anchor assembly has been pulled inside the anchor retrieval sheath.

## Patentansprüche

1. Zweiteilige Herzklappenprothese, die von einer Verankerungsanordnung (200) und einer Klappenanordnung (300) umfasst wird, und ein Entfaltungssystem, umfassend:
einen Führungskatheter (120), der ein Lumen definiert, wobei der Führungskatheter für das Einführen in ein Gefäßsystem eines menschlichen Patienten bemessen ist;
einen inneren Katheter (160), der lösbar mit der Verankerungsanordnung (200) der Herzklappenprothese koppelbar ist, wobei der innere Katheter (160) und die Verankerungsanordnung (200) innerhalb des Lumens des Führungskatheters (120) angeordnet werden können, während sich die Verankerungsanordnung (200) in einer niedrigprofiligen Einführungskonfiguration befindet;
einen Klappeneinführungskatheter (180), der lösbar mit der Klappenanordnung (300) der Herzklappenprothese koppelbar ist, wobei der Klappeneinführungskatheter (180) und die Klappenanordnung (300) innerhalb des Lumens des Führungskatheters (120) angeordnet werden können, während sich die Klappenanordnung (300) in einer niedrigprofiligen Einführungskonfiguration befindet; und
einen Klappenrückholkatheter (190), der ein Lumen definiert, wobei der Klappeneinführungskatheter (180) innerhalb des Lumens des Klappenrückholkatheters (190) angeordnet werden kann, und wobei ein distaler Endabschnitt des Klappenrückholkatheters (190) einen Klappeneingriffsabschnitt (192) umfasst, der konfiguriert ist, selektiv mechanisch mit der Klappenanordnung (300) in Eingriff zu kommen, wobei der Klappeneingriffsabschnitt (192) eine Rahmenstruktur umfasst, die einen oder mehrere offene Zwischenräume definiert, die so bemessen und positioniert sind, dass sie selektiv mechanisch mit einem oder mehreren Haken an einem proximalen Ende der Klappenanordnung in Eingriff kommen, und wobei die Rahmenstruktur thermisch mit einem Schaft des Klappenrückholkatheters (190) gekoppelt ist; und
eine Klappeneinführungshülse (170), die ein Lumen definiert, wobei der Klappenrückholkatheter (190) innerhalb des Lumens der Klappeneinführungshülse (170) angeordnet werden kann.

2. Zweiteilige Herzklappenprothese und Entfaltungssystem nach Anspruch 1, wobei der eine oder die mehreren Haken am proximalen Ende der Klappenanordnung jeweils so vorgespannt sind, dass sie von einer Mitte der Klappenanordnung in einem spitzen Winkel radial nach außen abstehen.

3. Zweiteilige Herzklappenprothese und Entfaltungssystem nach einem der Ansprüche 1 und 2, wobei der eine oder die mehreren Haken am proximalen Ende der Klappenanordnung dazu konfiguriert sind, in mechanischen Eingriff mit dem Klappeneingriffsabschnitt einzuschnappen.

4. Zweiteilige Herzklappenprothese und Entfaltungssystem nach einem der Ansprüche 1 bis 3, wobei die Klappenanordnung (300) Bögen (310a-c) aufweist und ein oberer Teil der Bögen (310a-c) den einen oder die mehreren Haken (313a-c) am proximalen Ende der Klappenanordnung (300) aufweist.

5. Zweiteilige Herzklappenprothese und Entfaltungssystem nach einem der Ansprüche 1 bis 4, wobei der eine oder die mehreren Haken (313a-c) am proximalen Ende der Klappenanordnung (300) so konfiguriert sind, dass sie, wenn sie beginnen, mit dem Klappeneingriffsabschnitt (192) in Kontakt zu kommen, radial zusammengedrückt werden, um sich nach innen zu biegen.

6. Zweiteilige Herzklappenprothese und Entfaltungssystem nach Anspruch 5, wobei der eine oder die mehreren Haken (313a-c) am proximalen Ende der Klappenanordnung (300) dazu konfiguriert sind, in den einen oder die mehreren offenen Zwischenräume der Rahmenstruktur einzudringen und dann in eine radial nach außen vorgespannte Position zurückzukehren, wenn der eine oder die mehreren Haken (313a-c) am proximalen Ende der Klappenanordnung (300) in den einen oder die mehreren offenen Zwischenräume eindringen.

7. Zweiteilige Herzklappenprothese und Entfaltungssystem nach einem der Ansprüche 1 bis 6, wobei entweder der Klappeneinführungskatheter (180) oder der Klappenrückholkatheter (190) einen oder mehrere Katheterhaken an einem distalen Endabschnitt aufweist.

8. Zweiteilige Herzklappenprothese und Entfaltungssystem nach Anspruch 7, wobei der eine oder die mehreren Katheterhaken an einem der Katheter, nämlich dem Klappeneinführungskatheter und dem Klappenrückholkatheter, sich von dem Katheter nach außen und nach oben erstrecken, um eine Hakenform zu bilden.

9. Zweiteilige Herzklappenprothese und Entfaltungssystem nach Anspruch 7, wobei der eine oder die mehreren Katheterhaken an einem der Katheter, nämlich dem Klappeneinführungskatheter und dem Klappenrückholkatheter, sich von einem Innenraum des Katheters nach innen und nach oben erstrecken, um eine Hakenform zu bilden.

10. Zweiteilige Herzklappenprothese und Entfaltungssystem nach einem der Ansprüche 1 bis 9, ferner umfassend eine ein Lumen definierende Ankerrückholhülse, wobei der innere Katheter und die Verankerungsanordnung innerhalb des Lumens der Ankerrückholhülse angeordnet werden können, und wobei die Ankerrückholhülse innerhalb des Lumens des Führungskatheters angeordnet werden kann.

11. Zweiteilige Herzklappenprothese und Entfaltungssystem nach Anspruch 10, wobei ein distaler Endabschnitt der Ankerrückholhülse distal von einem distalen Ende des Führungskatheters positioniert ist, so dass die Ankerrückholhülse aus dem Führungskatheter und in ein linkes Atrium ragt.

12. Zweiteilige Herzklappenprothese und Entfaltungssystem nach einem der Ansprüche 10 und 11, wobei die Ankerrückholhülse einen ausdehnbaren trichterförmigen oder abgeschrägten distalen Endabschnitt aufweist.

13. Zweiteilige Herzklappenprothese und Entfaltungssystem nach einem der Ansprüche 10 bis 12, wobei die Ankerrückholhülse und der Innenkatheter (160) dazu konfiguriert sind, miteinander verriegelt zu werden, sobald die Verankerungsanordnung in die Ankerrückholhülse eingezogen worden ist.

## Revendications

1. Valve cardiaque prothétique en deux parties, constituée d'un ensemble ancre (200) et d'un ensemble valve (300), et d'un système de déploiement, comprenant :
un cathéter de guidage (120) définissant une lumière, le cathéter de guidage calibré pour l'insertion à l'intérieur d'une vasculature d'un patient humain ;
un cathéter interne (160) pouvant être accouplé, de manière à pouvoir être libéré, avec l'ensemble ancre (200) de la valve cardiaque prothétique, dans laquelle le cathéter interne (160) et l'ensemble ancre (200) peuvent être mis en place à l'intérieur de la lumière du cathéter de guidage (120) tandis que l'ensemble ancre (200) présente une configuration de pose de faible profil ;
un cathéter de pose de valve (180) pouvant être accouplé de manière à pouvoir être libéré avec l'ensemble valve (300) de la valve cardiaque prothétique, dans laquelle le cathéter de pose de valve (180) et l'ensemble valve (300) peuvent être mis en place à l'intérieur de la lumière du cathéter de guidage (120) tandis que l'ensemble valve (300) présente une configuration de pose de faible profil ; et
un cathéter de récupération de valve (190) définissant une lumière, dans laquelle le cathéter de pose de valve (180) peut être mis en place à l'intérieur de la lumière du cathéter de récupération de valve (190), et dans laquelle une portion d'extrémité distale du cathéter de récupération de valve (190) inclut une portion d'engagement de valve (192) qui est configurée pour s'engager mécaniquement et sélectivement avec l'ensemble valve (300), dans laquelle la portion d'engagement de valve (192) comprend une structure charpente définissant un ou plusieurs espaces interstitiels ouverts qui sont calibrés et positionnés pour s'engager mécaniquement et sélectivement avec un ou plusieurs crochets sur une extrémité proximale de l'ensemble valve, et dans laquelle la structure charpente est thermiquement couplée à une tige du cathéter de récupération de valve (190) ; et
une gaine de pose de valve (170) définissant une lumière, dans laquelle le cathéter de récupération de valve (190) peut être mis en place à l'intérieur de la lumière de la gaine de pose de valve (170).

2. Valve cardiaque prothétique en deux parties et système de déploiement selon la revendication 1, dans lesquels le un ou plusieurs crochets sur l'extrémité proximale de l'ensemble valve sont chacun inclinés pour se projeter radialement vers l'extérieur à l'opposé d'un centre de l'ensemble valve sous un angle aigu.

3. Valve cardiaque prothétique en deux parties et système de déploiement selon l'une quelconque des revendications 1 et 2, dans lesquels le un ou plusieurs crochets sur l'extrémité proximale de l'ensemble valve sont configurés pour s'ajuster en engagement mécanique avec la portion d'engagement de valve.

4. Valve cardiaque prothétique en deux parties et système de déploiement selon l'une quelconque des revendications 1 à 3, dans lesquels l'ensemble valve (300) inclut des arches (310a-c), et un sommet des arches (310a-c) inclut le un ou plusieurs crochets (313a-c) sur l'extrémité proximale de l'ensemble valve (300).

5. Valve cardiaque prothétique en deux parties et système de déploiement selon l'une quelconque des revendications 1 à 4, dans lesquels le un ou plusieurs crochets (313a-c) sur l'extrémité proximale de l'ensemble valve (300) sont configurés de sorte que lorsqu'ils commencent à entrer en contact avec la portion d'engagement de valve (192), ils seront radialement comprimés pour fléchir vers l'intérieur.

6. Valve cardiaque prothétique en deux parties et système de déploiement selon la revendication 5, dans lesquels le un ou plusieurs crochets (313a-c) sur l'extrémité proximale de l'ensemble valve (300) sont configurés pour passer dans le un ou plusieurs espaces interstitiels ouverts de la structure charpente et ensuite reviennent à une position radialement inclinée vers l'extérieur lorsque le un ou plusieurs crochets (313a-c) sur l'extrémité proximale de l'ensemble valve (300) passent dans le un ou plusieurs espaces interstitiels ouverts.

7. Valve cardiaque prothétique en deux parties et système de déploiement selon l'une quelconque des revendications 1 à 6, dans lesquels l'un du cathéter de pose de valve (180) et du cathéter de récupération de valve (190) inclut un ou plusieurs crochets de cathéter sur une portion d'extrémité distale.

8. Valve cardiaque prothétique en deux parties et système de déploiement selon la revendication 7, dans lesquels le un ou plusieurs crochets de cathéter sur l'un du cathéter de pose de valve et du cathéter de récupération de valve s'étendent vers l'extérieur et vers le haut depuis le cathéter pour créer une forme de crochet.

9. Valve cardiaque prothétique en deux parties et système de déploiement selon la revendication 7, dans lesquels le un ou plusieurs crochets de cathéter sur l'un du cathéter de pose de valve et du cathéter de récupération de valve s'étendent vers l'intérieur et vers le haut depuis un intérieur du cathéter pour créer une forme de crochet.

10. Valve cardiaque prothétique en deux parties et système de déploiement selon l'une quelconque des revendications 1 à 9, comprenant en outre une gaine de récupération d'ancre définissant une lumière, dans lesquels le cathéter interne et l'ensemble ancre peuvent être mis en place à l'intérieur de la lumière de la gaine de récupération d'ancre, et dans lesquels la gaine de récupération d'ancre peut être mise en place à l'intérieur de la lumière du cathéter de guidage.

11. Valve cardiaque prothétique en deux parties et système de déploiement selon la revendication 10, dans lesquels une portion d'extrémité distale de la gaine de récupération d'ancre est positionnée au plan distal d'une extrémité distale du cathéter de guidage de sorte que la gaine de récupération d'ancre fait saillie depuis le cathéter de guidage et dans une oreillette gauche.

12. Valve cardiaque prothétique en deux parties et système de déploiement selon l'une quelconque des revendications 10 et 11, dans lesquels la gaine de récupération d'ancre inclut un entonnoir expansible ou une portion d'extrémité distale en forme de biseau.

13. Valve cardiaque prothétique en deux parties et système de déploiement selon l'une quelconque des revendications 10 à 12, dans lesquels la gaine de récupération d'ancre et le cathéter interne (160) sont configurés pour être verrouillés ensemble, une fois que l'ensemble ancre a été tiré à l'intérieur de la gaine de récupération d'ancre.
